(19) Europäisches Patentamt
European Patent Office
Office européen des brevets

(11) EP 1 055 729 A1

(12) **EUROPEAN PATENT APPLICATION**

(43) Date of publication:
**29.11.2000 Bulletin 2000/48**

(21) Application number: **99109751.0**

(22) Date of filing: **18.05.1999**

(51) Int Cl.[7]: **C12N 15/29**, C12N 15/11, C12N 15/62, C12N 15/82, C12N 5/10, C12N 1/21, C07K 14/415, C07K 16/16, A01H 5/00, A01H 5/10

(84) Designated Contracting States:
**AT BE CH CY DE DK ES FI FR GB GR IE IT LI LU MC NL PT SE**
Designated Extension States:
**AL LT LV MK RO SI**

(71) Applicant: **Max-Planck-Gesellschaft zur Förderung der Wissenschaften e.V.**
**80539 München (DE)**

(72) Inventors:
- **Hartmann, Ulrike**
  **50859 Köln (DE)**
- **Huijser, Peter**
  **6417 HC Heerlen (NL)**
- **Höhmann, Susanne**
  **50672 Köln (DE)**
- **Saedler, Heinz**
  **50829 Köln (DE)**
- **Wisman, Ellen**
  **Simpelveld (NL)**

(74) Representative:
**Wibbelmann, Jobst, Dr., Dipl.-Chem.**
**Wuesthoff & Wuesthoff,**
**Patent- und Rechtsanwälte,**
**Schweigerstrasse 2**
**81541 München (DE)**

Remarks:
The applicant has subsequently filed a sequence listing and declared, that it includes no new matter.

(54) **TRANSGENIC PLANTS EXHIBITING AN ALTERED FLOWERING TIME**

(57) The invention is directed to transgenic plants exhibiting an altered flowering time due to an altered expression of the MADS box gene *SVP* (Short Vegetative Phase), a gene which appears to be involved in the timing of the flowering of plants. Furthermore, the invention is directed to novel cDNA sequences of said *SVP* gene, DNA constructs, in particular vectors, carrying said novel cDNAs, microorganisms transformed with a construct comprising said novel cDNAs as well as the gene products of the *SVP* gene and antibodies recognizing said gene products.

EP 1 055 729 A1

## Description

The invention is directed to transgenic plants exhibiting an altered flowering time due to an altered expression of the gene *SVP* (Short Vegetative Phase), a gene which appears to be involved in the timing of the flowering of plants. Furthermore, the invention is directed to novel cDNA sequences of the SVP gene, DNA constructs, in particular vectors, carrying said novel cDNAs, microorganisms transformed with a construct comprising said novel cDNAs as well as the gene products of the *SVP* gene and antibodies recognizing said gene products.

Plant development, including the process of flowering is genetically controlled but is also strongly subjected to the environment. Together, the endogenous genetic programs and the environmental factors largely determine where plants of agricultural, horticultural, floricultural, silvicultural or fruit growing interest can be grown successfully.

Controlling the environment is well possible in greenhouses but is much more difficult to achieve for plants grown under field conditions. In the latter case the plant should be adapted to its environment which is best achieved through its genetic make-up. Varieties with genotypes better matching the local environmental conditions will increase yield per hectare and require less effort in growing (also of importance for cultivation under glass). As a consequence production costs will be lowered and competitiveness increased.

Flowering time is generally defined as the time between germination and opening of the first flower. In higher plants, the start of flower formation is the result of a developmental phase switch known as the floral transition.

Because of its strong correlation to maturation time, flowering time is an important trait in plant growth management and plant breeding programs. Moreover, it is either the vegetative parts of the plant formed before or the fruits and seeds produced following the formation of the flowers that are important commodities of numerous crop plants. The flowers themselves are often the end products of ornamental plants.

Controlled modification of flowering of economically important plants would allow an extension of their cultivation areas and/or, in certain cases, allow to grow more generations per year. In addition, earlier flowering lines of ornamental plants could be brought earlier to the market, thus, when followed by the marketing of the respective conventional ornamental plants, extending the time of commercial exploitation of a particular ornamental plant per year. Besides regular and strong flowering, early-flowering by itself is often a desirable trait in fruit and ornamental trees. Also breeding programs, especially in the case of trees, could benefit from early flowering varieties because of shortening of the generation time.

Early flowering is for instance desired in oilseed rape, turnip rape, cauliflower, cotton, sunflower, diverse ornamentals, fruit and ornamental trees and shrubs.

Late flowering varieties of economically important plants would allow an extension of their vegetative growth phase. This is of special interest in those crops which are grown for their vegetative parts, e.g. leaves, roots, tubers and contents thereof, e.g. sugar, starch. Late flowering is also desired in cold sensitive crops and ornamentals.

Examples of important crop species of interest with respect to a modification towards late flowering time comprise lettuce, potato, carrot, italian ryegrass, fodder beet, sugar beet, celery, endive, headed cabbage and onion. Furthermore late flowering would be favoured for diverse tree species for, for instance, wood production.

Furthermore, modification towards controlable flowering is of interest in ornamentals, for breeding purposes as well as to prevent the out-crossing of transgenic material to wild-type species.

The control of the flowering time of plants has been established to be associated with various types of genes. For example, some members of the family of MADS box genes have been found to have a corresponding function in the control of flowering time of plants. MADS box genes are a particular group of genes encoding proteins having a particular common DNA binding domain (Schwarz-Sommer et al., 1990).

The small crucifer weed *Arabidopsis thaliana* is regarded as a major model system for the molecular genetic analysis of the floral transition. Genetic studies in this plant led to the identificaton of more than 50 loci involved in the floral transition. Several of these genes could be cloned in recent years (Levy and Dean, 1998; http://www.salk.edu/LABS/pbio-w/flower web.html).

Modification of flowering time in transgenic plants has been achieved with the help of non-MADS-box genes derived from *Arabidopsis thaliana* (Weigel and Nilsson, 1995; Putterill et al., 1995; Simon et al., 1996; Macknight et al., 1997; Cardon et al., 1997) as well as with MADS-box genes derived from *Arabidopsis thaliana* as well as from different plant species, namely rice or *Sinapis alba.* However, in the case of the latter, gain of function (e.g. constitutive expression under the control of the 35S CaMV promoter) resulted in early flowering (Chung et al., 1994; Mandel and Yanofsky, 1995; Menzel et al., 1996; An et al. 1997; Kang et al., 1997) and loss-of-function lead to late flowering phenotypes (unpublished results).

Most recently, a particular MADS-box gene, FLF, has been described to act as a repressor of flowering in *Arabidopsis thaliana* (Sheldon et al., 1999).

Among the genes reviewed in Levy and Dean, 1998, are also such that cause early flowering upon loss-of-function mutation. However as presented in the overview of Levy and Dean, 1998, the early flowering mutant phenotype of the above mentioned genes is often accompanied with more or less severe morphological alterations. For instance, besides early flowering, loss-of-function of the genes *ELF3* and *PHYB* results in elongated hypocotyls. In addition, *elf3* mutants display earliness only under short day conditions and *phyB* mutants are pale green (Hicks et al., 1996). Early flowering mutants like *clf* and *wlc* have abnormally shaped leaves and the mutants *esd4* and *tfl1* form terminal flowers. In addition, *esd4* mutants have abnormal shaped siliques. The *spy* mutants show elongated inter-nodes and distorted growth.

Evidently, these morphological anomalies accompanying the above mentioned mutants are generally undesired in case of their application to modify the flowering time of crop species. Furthermore, due to the economic importance of this feature, there remains a consistent need to provide new alternative genetic information which enables the production of transgenic plants exhibiting an altered flowering time which show essentially no morphological alterations.

Accordingly, the problem underlying the present invention is to provide new genetic information enabling the production of transgenic plants exhibiting these features.

SUMMARY OF THE INVENTION

In the course of the research on the molecular genetic mechanisms controlling flowering time, the inventors have identified a heretofore unknown function of a particular *Arabidopsis thaliana* MADS-box gene located on an IGF-BAC clone, clone IGF-BAC 14M13. In the course of the partial sequencing of said clone, said MADS-box gene was identified and its sequence within the complete sequence of the IGF-BAC clone has subsequently been published in the INTERNET but no function of said gene has become known until now.

This MADS-box gene which has been named *SVP* (Short Vegetative Phase) by the inventors has been found to cause upon mutation early flowering in plants in the absence of obvious morphological alterations. Moreover, early flowering of the *svp* mutants has been observed to occur under different photoperiodic regimens, i.e. long as well as short days.

Plants heterozygous for mutations resulting in a loss of *SVP* gene function were found to flower earlier compared to wild type plants but to flower later when compared to plants homozygous for such mutations. On the contrary, later flowering of plants compared to wild type plants may be achieved by constitutive or over-expression of the *SVP* gene. The present findings show that flowering time in plants can be controlled through modification of the expression level of *SVP*.

The invention to control flowering time in plants is thus based on the manipulation of expression levels of an *SVP* gene which can be achieved with the help of modern biotechnological methods. According to the invention, transgenic plants can be generated that express one or more copies of a *SVP* cDNA in either sense or antisense orientation and under the control of different, i.e. constitutive, developmentally regulated or inducible promoters. Reduced levels of *SVP* will accelerate flowering whereas enhanced levels of *SVP* will delay flowering in such transgenic plants.

The evolutionary well conserved genetic functions of members of the MADS-box gene family in combination with conserved mechanisms in the control of flowering will allow modification of flowering behaviour in different plant species (Laurie, 1997; Colasanti and Sundaresan, 1996) with the help of *SVP* and/or its homologs.

As already mentioned above, modification of flowering time in transgenic plants has been achieved with the help of non-MADS-box genes derived from *Arabidopsis thaliana,* (Weigel and Nilsson, 1995; Putterill et al., 1995; Simon et al., 1996; Macknight et al., 1997; Cardon et al., 1997) as well as with MADS-box genes derived from *Arabidopsis thaliana* as well as from different plant species, namely rice or *Sinapis alba.* However, in the case of the latter, gain of function (e.g. constitutive expression under the control of the 35S CaMV promoter) resulted in early flowering (Chung et al., 1994; Mandel and Yanofsky, 1995; Menzel et al., 1996; An et al. 1997; Kang et al., 1997) and loss-of-function lead to late flowering phenotypes (unpublished results), i.e. effects opposite to those claimed for *SVP.*

Accordingly, under a first aspect the invention is directed to a plant comprising a genetic modification resulting in an altered flowering time of said plant when compared to a corresponding wild-type plant or a corresponding non-wild-type plant without said genetic modification wherein said genetic modification results in an altered expression of a *SVP* gene in the plant when compared to the wild-type plant or the non-wild-type plant without said genetic modification.

Furthermore, the invention comprises a method for altering the flowering time in a plant comprising the step of introducing at least one genetic modification into said plant said genetic modification resulting in an altered expression of a *SVP* gene in said plant.

The invention also pertains to a kit for use in the method according to the present invention.

Under a second aspect, the invention is directed to a DNA molecule essentially consisting of or comprising at least one of the two cDNAs of the *SVP* gene derived from *Arabidopsis thaliana* or a nucleotide sequence derived therefrom.

Although the genomic sequence of the *SVP* gene of *Arabidopsis thaliana* has become most recently known, the commonly employed sequence evaluation programs such as the GENESCAN program of Perkin Elmer failed to predict the cDNA sequence of the *SVP* gene correctly. It was accordingly surprising to the inventors to find that the *SVP* gene gives rise to at least two cDNAs due to alternative splicing and to determine the actual nucleotide sequences thereof.

Due to the unpredictability of the cDNA/mRNA sequence of the *SVP* gene, it has therefore only been possible to derive the exon-intron structure of said gene after the identification and determination of the cDNA sequences thereof by comparison of the cDNA and genomic sequences (see section "Examples" below).

The invention is furthermore directed to "antisense" nucleotide molecules and fragments of the above nucleotide sequences as well as constructs, in particular vectors carrying these nucleotide molecules and fragments. Furthermore, the invention encompasses microorganisms transformed with any of the nucleotide molecules, fragments, constructs or vectors of the present invention.

Under a further aspect, the invention pertains also to the gene products of the *SVP* gene. Due to the unpredictability of the cDNA/mRNA sequence of the *SVP* gene, it has also been impossible to determine the actual amino acid sequence of any gene product of said SVP gene previous to the identification and sequencing of the cDNA thereof. As is known with respect to the function of the gene products of other MADS box genes, the gene products of the SVP gene are supposed to act as transcription factors.

In this respect, further claims are also directed to polypeptide and peptide fragments of said gene products as well as proteins and polypeptides comprising said gene product(s) of the SVP gene, in particular fusion proteins.

Finally, the invention also provides monoclonal and polyclonal antibodies specifically recognizing and binding a gene product of a *SVP* gene of a fragment thereof.

## DETAILED DESCRIPTION OF THE INVENTION

Under a first aspect, the invention provides a plant comprising a genetic modification resulting in an altered flowering time of said plant when compared to a corresponding wild-type plant or a corresponding non-wild-type plant without said genetic modification wherein said genetic modification results in an altered expression of a *SVP* gene in the plant when compared to the wild-type plant or the non-wild-type plant without said genetic modification.

The term "*SVP* gene" as used throughout this specification and the appended claims shall preferably include

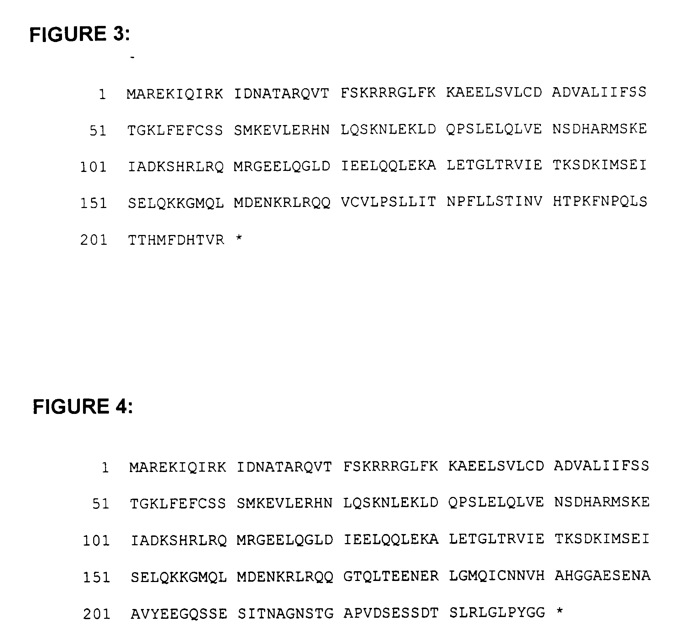

- a *SVP* gene comprising or essentially consisting of a nucleotide sequence as set forth in any one of SEQ ID NOS. 5 or 6; or
- a *SVP* gene comprising or essentially consisting of a nucleotide sequence exhibiting, within the coding regions thereof, at least 75% homology, particularly at least 80%, preferably at least 85%, more preferably at least 90%, even more preferably at least 95% and most preferably at least 98% homology to the coding regions of the *SVP* gene having the nucleotide sequence as set forth in any one of SEQ ID NOS. 5 or 6; and in particular
- a *SVP* gene giving rise to a cDNA comprising or essentially consisting of a nucleotide sequence as set forth in any one of SEQ ID NOS. 1 and 3 or a nucleotide sequence comprising or essentially consisting of having a homology of at least 75%, particularly at least 80%, preferably at least 85%, more preferably at least 90%, even more preferably at least 95% and most preferably at least 98% to any one of said sequences; or
- a DNA sequence encoding any one of the *SVP* gene products comprising or essentially consisting of an amino acid sequence as set forth in any one of SEQ ID. NOS. 21 and 22 (Figs. 3 and 4); or
- a DNA sequence encoding a SVP protein comprising or essentially consisting of an amino acid sequence exhibiting at least 85% homology, preferably at least 90%, more preferably at least 95% and most preferably at least 98% homology to any one of the *SVP* gene products having an amino acid sequence as set forth in any one of SEQ ID. NOS. 21 and 22 (Figs. 3 and 4); or
- an effective portion of any of the *SVP* genes or DNA sequences defined above.

Accordingly, for the purpose of the present invention, it is not necessary that the *SVP* gene includes intron sequences.

In the present context, the term "effective" characterizes a feature of a nucleotide or amino acid sequence, for example, a portion of the *SVP* genes or DNA sequences defined above, that one or the other, when the expression thereof being altered in a plant, is effective in modifying the flowering time of said plant. The inclusion of an "effective portion" of the *SVP* genes or DNA sequences defined above accounts for the fact that despite substantial homology a *SVP* gene exhibiting this particular effect on flowering time may also be considerably shorter than any one of the sequences referred to above.

Furthermore, throughout the present specification and claims, the term "altered expression" comprises any altered expression of a specified nucleotide sequence in a specified target organism including an enhanced, reduced or eliminated expression of said specified nucleotide sequence, an expression of said specified nucleotide sequence which is newly established in said target organism as well as the expression of an altered transcription product resulting in the translation of no or an altered translation product exhibiting an altered level of activity within the plant when compared to the corresponding unaltered translation product.

Within the present specification and claims, the terms "activity" or "active" are to be understood to refer to the "activity" of a nucleotide sequence or protein/polypeptide with respect to the control of the flowering time of a plant. As the gene products of MADS box genes are known to act as transcription factors it is assumed that the "activity" of the *SVP* genes and gene products with respect to the control of the flowering time of a plant will correlate with the transcription factor activity thereof. In the present invention, a reduced "activity" of the SVP genes and gene products within a plant will lead to early flowering whereas an enhanced "activity" will result in late flowering.

In a first embodiment the genetically modified plant according to the invention exhibits an early flowering time wherein said genetic modification results in a reduced, lacking or qualitatively altered expression of a *SVP* gene as defined above wherein the qualitatively altered expression of said *SVP* gene results in a reduced or lacking activity of the gene product of said *SVP* gene in said plant.

For this, said genetic modification may consist in

(1) a mutation of any one of said *SVP* genes, DNA sequences or effective portions thereof in said plant, wherein said mutation results in loss of function of said *SVP* gene, DNA sequence or effective portion thereof;

(2) a mutation of any one of said *SVP* genes, DNA sequences or effective portions thereof in said plant, wherein said mutation results in a reduced activity of the *SVP* gene, DNA sequence or effective portion thereof as defined above;

(3) an expression of an "antisense" nucleotide molecule with respect to any one of the *SVP* genes, DNA sequences or effective portions thereof as defined above; and/or

(4) an expression of a "sense" nucleotide molecule with respect to any one of the *SVP* genes, DNA sequences or effective portions thereof as defined above, wherein said expression of said "sense" nucleotide molecule results in cosuppression of said *SVP* gene, DNA sequence or effective portion thereof.

In the present context, the term "loss of function" of a *SVP* gene, DNA sequence or effective portion thereof comprises all possibilities of loss of the function of any of the latter including the functionality of any gene product thereof, respectively. Accordingly, for example, the mutation may affect a *SVP* gene in a way resulting in one of the following:

- lack of transcription of said gene;
- an altered transcription product of said gene, wherein said-altered transcription product being not amenable to translation; or
- an altered transcription product of said gene, wherein said altered transcription product being translated into a completely unfunctional or inactive gene product.

In the course of research on MADS box genes it has been found that the gene products of the MADS box genes exhibit their function as transcription factors in dimeric form which may include homodimeric as well as heterodimeric forms. In addition, for their biological activity further protein or polypeptide interaction with one or more different protein(s) or polypeptide(s) is required. Accordingly, the complete lack of function of any one of the gene products mentioned above may, for example, result from an inability to form the homodimer or heterodimer, an inability of interaction with the further different protein(s) or polypeptide(s) essential for the biological activity of the former or an inability of binding to or functional interaction with the DNA target structure.

The term "reduced activity" of a *SVP* gene, DNA sequence or effective portion thereof comprises all aspects of reduction of the activity of any one of the latter including the activity of any gene product thereof with respect to the control of the flowering time in a plant. Accordingly, for example, the mutation may affect the *SVP* gene in a way resulting in one of the following:

- an altered, i.e. reduced level of transcription of said gene;
- an altered, i.e. reduced level of translation of the transcription product of said gene; or
- an altered transcription product of said gene, wherein said altered transcription product leading to an altered translation product having a reduced activity in a plant when compared to the unaltered translation product.

In view of the biological prerequisites required for the activity of MADS box gene products outlined above including dimerisation and interaction with at least one further different protein or polypeptide essential for the biological activity of the former, the reduced activity of any one of the gene products mentioned above may, for example, result from a reduced ability to form the homodimer or heterodimer, a reduced ability of interaction with the further different protein(s) or polypeptide(s) essential for the biological activity of the former, a reduced ability of binding to or functional interaction with the DNA target structure, or an accelerated metabolic degradation of the gene product or the active homodimer or heterodimer.

In this context, it goes without saying that the invention encompasses also the possibility of multiple mutations leading to more than one of the above-mentioned effects.

Within the present specification and claims, the term "mutation" comprises any alteration of a nucleotide sequence resulting i.a. in a deletion, insertion, substitution, modification, or addition of one or more nucleotides. For example, a frequently observed type of mutation are the frame shift mutations which, upon expression, give rise to a different gene product which usually has a different length as well as a different amino acid sequence when compared to the gene product resulting from the unmutated gene. Depending on the intended effect on the gene function or activity, the term "mutation" also comprises the insertion or deletion of larger DNA portions.

A reduction, elimination or alteration of the expression of a *SVP* gene may be realized by mutation of said gene resulting in loss of function thereof (loss-of-function mutation; knock-out mutation) or resulting in a modified function thereof (reduced level of transcription and/or translation; altered transcription product; altered transcription and translation products).

Relevant mutations may affect coding and intron regions of a *SVP* gene as well as regulatory sequences in the 5' or 3' non coding regions as well as within intron sequences.

Classical techniques for a non sequence specific mutation comprise the use of X-rays or mutagenizing chemicals. Furthermore, the mutation may be carried out in a more sequence-specific way affecting only or substantially only the gene of interest. For this, specific transposons such as the *En-1* transposon or a technique called "gene targetting" utilizing homologous recombination in a target plant by use of a suitable DNA fragment derived from the *SVP* gene of interest similar to the procedure already established for yeast and mammal systems (Puchta and Hohn, 1996) may be used.

Alternatively, the reduction or elimination of the expression of a *SVP* gene may be realized by expression of particular "antisense" or "sense" nucleotide constructs in the plant. These strategies are generally summarized under the term "homologydependent gene silencing" (see e.g. Faske et al., 1997; Taylor, 1997). By the expression of "antisense" nucleotide constructs it is intended to obtain hybridization with *SVP* mRNA thus preventing a translation of the latter. With the expression of "sense" constructs hybridization phenomena on DNA level and/or posttranscriptional effects shall be achieved which result in a cosuppression of the *SVP* gene. Both strategies lead to a strongly reduced or even lacking expression of a target gene.

For this, an "antisense" or "sense" molecule comprises at least a part of a target *SVP* gene in "antisense" or "sense" orientation, respectively. The "antisense" or "sense" portion of the molecule or construct comprises usually at least between 50 and 200 nucleotides and may even comprise the complete *SVP* gene sequence in "antisense" or "sense" orientation, respectively.

The genetic information coding for the "antisense" or "sense" nucleotide molecule may be integrated into the genome of the plant or may be maintained, propagated and transcribed extrachromosomally, e.g., by use of suitable viruses comprising said genetic information.

The expression of the "antisense" or "sense" nucleotide molecule in the genetically modified plant according to the present invention may be realized under the control of a constitutive, developmentally regulated or inducible promoter. The promoter may further be tissue-specific. Exemplary promoters which may be used are the 35S promoter or the endogenous promoter which is associated with the *SVP* gene of *Arabidopsis thaliana in vivo.*

Strategies, techniques, methods and reagents for performing the above-described genetic modifications, e.g. mutations or transformations, in plants are well known to skilled persons. For the transformation of plant cells, for example, exemplary methods are a direct DNA transfer (e.g. by electroporation into protoplasts, by addition of a high molecular weight osmotic agent or by biolistic methods wherein DNA-coated particles are shot into plant tissue) as well as the use of natural host/vector systems (e.g. of *agrobacteria* or plant viruses). For extrachromosomal maintenance of recombinant DNA various specific viruses like tobacco mosaic virus (TMV) or potato virus X in the genome of which said recombinant DNA sequence then being inserted, may be utilized.

In a further embodiment, the genetically modified plant according to the present invention exhibits a late flowering time wherein said genetic modification results in a novel, enhanced or qualitatively altered expression of a *SVP* gene as defined above wherein the qualitatively altered expression of the *SVP* gene results in a *SVP* gene product of higher activity in said plant.

For this, the genetic modification may consist in introducing at least one transgene into the plant, said transgene comprising the nucleotide sequence of any one of the *SVP* genes, DNA sequences or effective portions thereof as defined above.

In a particular embodiment the transgene mentioned above may include besides the specifically defined nucleotide sequences further genetic information which in one embodiment upon expression may give rise to an expression product having an amino acid sequence comprising the *SVP* gene product or the SVP protein C and/or N-terminally fused to an additional amino acid sequence portion. In a very particular embodiment the expression product may be a fusion protein comprising the *SVP* gene product or *SVP* protein fused to another protein or polypeptide having a specific function or characteristic such as an enzymatic activity normally not present within the plant thus enabling an easy determination whether the *SVP* gene product or SVP protein is expressed in the genetically modified plant. Alternatively, the additional amino acid sequence portion may also represent a signal peptide specifically directing the *SVP* gene product or SVP protein to a specific compartment within the plant such as the nucleus.

Like the expression of the "antisense" nucleotide molecule and "sense" nucleotide molecule, the expression of the transgene in the genetically modified plant according to the present invention may be realized under the control of a constitutive, developmentally regulated or inducible promoter. The promoter may further be tissue-specific. Exemplary promoters which may be used are the 35S promoter or the endogenous promoter which is associated with the *SVP* gene of *Arabidopsis thaliana in vivo*. In a particular embodiment the endogenous promoter may additionally comprise specific intron sequences, particularly from the first intron immediately following the exon encoding the MADS domain of the *SVP* gene of *Arabidopsis thaliana.* These additional intron sequences are preferably provided integrated into the transcribed region in an intron-like manner.

With respect to experimental techniques utilizable for the transformation of the target plant, reference is made to the explanations given above with respect to the "antisense" and "sense" constructs.

For defining the term "plants" when, e.g. used for defining the starting material for producing the genetically modified, and in particular transgenic plants according to the present invention, this term shall include natural plants, hybrid plants and transgenic plants. Furthermore, this terms comprises mature as well as immature whole plants and developing plantlets.

The term "natural plants" shall include naturally occurring wild plants (wild-type plants) and cultivated plants obtained by a conventional plant breeding process.

The term "hybrid plants" shall mean plants which are obtained by hybridization, i.e. a genetic recombination process between genomes of plants which are derived from different populations and which cannot be stably propagated without losing some traits.

The term "transgenic plant" shall mean plants which contain at least one foreign DNA sequence, a so-called "transgene", that is normally not observed in said plants or, alternatively, which is normally observed in a different genetic environment within the plant. The genetically modified plants according to the present invention may also comprise additional genetic modifications, e.g. transgenes conferring resistance against pests or plant protection agents or leading to improved yield.

The transgenic plants according to the present invention comprise genetic information which leads to an altered, i.e. novel or modified expression of the *SVP* gene within the plant; this genetic information is normally not observed in said plants or is present in a genetic environment different from that which is normally observed in said plant.

Plants of interest as said transgenic plants according to the present invention are all plants of agricultural, horticultural, floricultural, silvicultural or fruit growing interest, monocotyledonous as well as dicotyledonous plants, e. g. crops, ornamentals, and trees including ornamental trees.

Non-limiting examples for plants with respect to a modification towards early flowering are plants such as oilseed rape (*Brassica napus,* e.g., L. var. napus), turnip rape *(Brassica rapa,* e.g., L. var. silvestris), cauliflower *(Brassica oleraceae,* e.g., L. var. botrytis), cotton (*Gossypium*), sunflower (*Helianthus annuus* L.), diverse ornamentals, fruit and ornamental trees and shrubs like rhododendron that do not flower the first few years. Also breeding programs, especially in the case of trees, could benefit from early flowering varieties because of shortening of the generation time.

Examples of important crop species of interest with respect to a modification towards late flowering time comprise onion (*Al-lium cepa* L.), celery *(Apium graveolens,* e.g., L. var. rapaceum), fodder beet *(Beta vulgaris,* e.g., L. var. alba), sugar beet (*Beta vulgaris,* e.g., L. var. altissima), headed cabbage (*Brassica oleracea,* e.g., L. var. capitata), endive (*Cichorium endivia* L.), carrot (*Daucus carota* L.), lettuce (*Lactuca sativa* L.), italian ryegrass (*Lolium multiflorum*) and potato (*Solanum tuberosum* L.). Furthermore late flowering would be favoured for diverse tree species for, for instance, wood production.

Furthermore, the invention pertains to any genetically modified plant part, plant cell or seed obtainable from a genetically modified plant or by the method for altering the flowering time in a plant according to the present invention.

The term "parts of plants" shall include highly differentiated plant parts such as plant organs, e.g. roots, leaves, etc.; propagating material, e.g. seeds, seedlings, etc., as well as poorly differentiated plant parts such as plant tissue, callus, plant cells or plant protoplasts.

In a further embodiment, the late flowering phenotype may be due to an expression of a *SVP* gene product of higher activity with respect to the control of flowering time. Said *SVP* gene product of higher activity may be due to a mutation of any one of the *SVP genes,* DNA sequences or effective portions thereof as defined above in said plant. The mutated gene or nucleotide sequence will give rise to an altered transcription product of said gene, wherein said altered transcription product will lead to an altered translation product having an enhanced activity in a plant when compared to the unaltered translation product.

In view of the biological prerequisites required for the activity of MADS box gene products outlined above including dimerisation and interaction with at least one further different protein or polypeptide essential for the biological activity of the former, the enhanced activity of any one of the gene products mentioned above may, for example, result from an enhanced ability to form the homodimer or heterodimer (i.e. an increased affinity for the binding partner), an enhanced ability of interaction with the further different protein(s) or polypeptide(s) essential for the biological activity of the former, an enhanced ability of binding to or functional interaction with the DNA target structure, or a retarded metabolic degradation of the gene product or the active homo or heterodimer.

Also in this case, it goes without saying that the invention encompasses also the possibility of multiple mutations leading to more than one of the above-mentioned effects.

The invention is also directed to a method for altering the flowering time in a plant comprising the step of introducing at least one genetic modification into said plant said genetic modification resulting in an altered expression of a *SVP* gene within said plant. As outlined before said *SVP* gene is preferably characterized by

(1) comprising a nucleotide sequence as set forth in any one of SEQ ID NOS. 5 or 6; or

(2) comprising a nucleotide sequence exhibiting, within the coding regions thereof, at least 75% homology, particularly at least 80%, preferably at least 85%, more preferably at least 90%, even more preferably at least 95% and most preferably at least 98% homology to the coding regions of the *SVP* gene having the nucleotide sequence as set forth in any one of SEQ ID NOS. 5 or 6; or

(3) being transcribable into mRNA giving rise to a cDNA comprising a nucleotide sequence as set forth in any one of SEQ ID NOS. 1 and 3 or a cDNA comprising a nucleotide sequence exhibiting at least 75% homology, particularly at least 80%, preferably at least 85%, more preferably at least 90%, even more preferably at least 95% and most preferably at least 98% homology to any one of said sequences of SEQ ID NOS. 1 and 3; or

(4) giving rise upon expression to a SVP protein comprising an amino acid sequence as set forth in any one of SEQ ID NOS. 21 and 22 (Figs. 3 and 4); or

(5) giving rise upon expression to a *SVP* gene product comprising an amino acid sequence exhibiting at least 85%, preferably at least 90%, more preferably at least 95% and most preferably at least 98% homology to any one of the SVP gene products having an amino acid sequence as set forth in any one of SEQ ID NOS. 21 and 22 (Figs. 3 and 4); or

(6) being an effective portion of any one of the SVP genes characterized in any one of (1) to (5).

As already outlined above, a reduced or even eliminated expression of a *SVP* gene or the expression of a *SVP* gene of lower activity will lead to an early flowering time of the plant whereas a novel or increased expression of a *SVP* gene or the expression of a *SVP* gene of higher activity will result in a late flowering time.

Accordingly, the plant which shall be modified with respect to flowering time may be a plant which already exhibits an expression of a *SVP* gene or may be a plant which, before the genetic modification, does not yet express an *SVP* gene. Evidently, the latter plant may be only modified to exhibit a later flowering time.

In order to determine the options present in a target plant which shall be modified with respect to flowering time, it is of interest to establish whether this plant shows an expression of a *SVP* gene or not.

For this, in principle, every conventional method of assaying the expression of genes, such as a Northern blot or Western blot analysis, may be used.

In a particular embodiment, the analysis of the expression of a *SVP* gene will be carried out using a Northern blot type analysis. As a hybridization probe for detecting any *SVP* gene present in the plant, for example, one of the DNA molecules (cDNAs) of the present invention (SEQ ID NOS. 1 and 3 and homologous sequences) or a fragment thereof which will be detestably labelled, may be used. The detection probe may be radioactively or non-radioactively, e.g. digoxigenin, labelled.

Alternatively, the analysis of the expression of a *SVP* gene may be carried out by using RT-PCR employing sequence specific PCR primers. The nucleotide sequence of the PCR primers used may be derived from the nucleotide sequence information given in the present application with respect to the *SVP* gene, cDNAs as well as the gene products thereof in a way known to the skilled person.

The technique as well as various protocols for performing Northern blot type analyses as well as RT-PCR are well known to the skilled scientists.

In a further alternative embodiment, the analysis of the expression of a *SVP* gene may be carried out using a monoclonal or polyclonal antibody detecting a gene product of the *SVP* gene in the plant (Western blot type analysis), said antibody being also provided with the present invention. Protocols for performing a Western blot type analysis are also well within the skills of a specialist in this field.

Depending on the result of this previous analysis, it may be determined to which type of modification the target plant is amenable.

Under a further aspect, the invention provides a method of obtaining a nucleic acid the expression of which within a plant being able to modify the flowering time thereof, said process comprising the steps of:

- hybridizing an oligonucleotide or nucleic acid molecule consisting of a *SVP* gene as defined above or being a fragment thereof to target nucleic acid derived from said plant;
- detecting and identifying successful hybridisation; and
- isolation of the relevant gene comprising the hybridizing portion from the target nucleic acid.

The hybridization step may involve performing the hybridization and identifying positive hybridisation under appropriately stringent conditions as well as performing a nucleic acid amplification, e.g. by means of PCR, and detecting any amplification product which is also usually carried out by probing these with suitable oligonucleotide probes.
Methods for performing the hybridization, detection and isolation steps of the method described above are well known in the art.
The invention further pertains to a kit for use in the method for altering the flowering time in a plant according to the present invention.
This kit may comprise

(a) a construct, preferably a vector, comprising a nucleotide sequence being transcribable into an "antisense" nucleotide molecule with respect to an endogenous *SVP* gene comprising a nucleotide sequence as set forth in any one of SEQ ID NOS. 5 or 6 or an endogenous *SVP* gene comprising a nucleotide sequence exhibiting, within the coding regions thereof, at least 75% homology, particularly at least 80%, preferably at least 85%, more preferably at least 90%, even more preferably at least 95% and most preferably at least 98% homology to the coding regions of the *SVP* gene having the nucleotide sequence as set forth in any one of SEQ ID NOS. 5 or 6 or an effective portion thereof; and/or
(b) a construct, preferably a vector, comprising a nucleotide sequence being transcribable into an "sense" nucleotide molecule with respect to an endogenous *SVP* gene comprising a nucleotide sequence as set forth in any one of SEQ ID NOS. 5 or 6 or an endogenous *SVP* gene comprising a nucleotide sequence exhibiting, within the coding regions thereof, at least 75% homology, particularly at least 80%, preferably at least 85%, more preferably at least 90%, even more preferably at least 95% and most preferably at least 98% homology to the coding regions of the *SVP* gene having the nucleotide sequence as set forth in any one of SEQ ID NOS. 5 or 6 or an effective portion thereof said "sense" nucleotide molecule being suitable to induce a cosuppression of the *SVP* gene; and/or
(c) a construct, preferably a vector, comprising, preferably operably linked to a plant-functional promoter, a transgene, said transgene being

(i) a *SVP* gene comprising a nucleotide sequence as set forth in any one of SEQ ID NOS. 5 or 6 or a *SVP* gene comprising a nucleotide sequence exhibiting, within the coding regions thereof, at least 75% homology, particularly at least 80%, preferably at least 85%, more preferably at least 90%, even more preferably at least 95% and most preferably at least 98% homology to the coding regions of the *SVP* gene having the nucleotide sequence as set forth in any one of SEQ ID NOS. 5 or 6; and/or
(ii) a DNA molecule essentially consisting of or comprising a nucleotide sequence as set forth in any one of SEQ ID NO. 1 or SEQ ID NO. 3 or a nucleotide sequence exhibiting a homology of at least 75%, particularly at least 80%, preferably at least 85%, more preferably at least 90%, even more preferably at least 95% and most preferably at least 98% to any one of said sequences, or a DNA molecule derived from said DNA molecule by nucleotide substitution, addition, insertion, deletion and/or modification, which hybrididizes with the latter; and/or
(iii) a DNA molecule encoding any one of the *SVP* gene products comprising an amino acid sequence as set forth in any one of SEQ ID. NO. 21 and 22 (Figs. No. 3 and 4); and/or
(iv) a DNA molecule encoding a SVP protein comprising an amino acid sequence exhibiting at least 85%, preferably at least 90%, more preferably at least 95% and most preferably at least 98% homology to any one of the *SVP* gene products having an amino acid sequence as set forth in SEQ ID. NOS. 21 and 22 (Figs. No. 3 and 4); and/or
(v) an effective portion of any one of the *SVP* genes and DNA molecules defined in any one of (i) to (iv).

In the context of the present invention, "constructs" are any synthetic nucleic acid molecules comprising a specific nucleic acid sequence linked to or situated within a nucleic acid sequence or genetic environment in combination with which it is not naturally found. A particular type of constructs are vectors, nucleic acid molecules designed as carrier constructs for manipulation and transfer/introduction of a specific nucleic acid sequence into a particular organism. Non-limiting examples of vectors are plasmids, cosmids, phagemids, viral or phage genomes, YACs (yeast artificial chromosomes) and BACs (bacterial artificial chromosomes).
In the constructs included in the kits according to the present invention, the indicated nucleotide sequences may be under the control of, i.e. operably linked to an active constitutive, developmentally regulated or inducible promoter which may optionally additionally provide for a tissue specific expression.

Furthermore, the kit may comprise reagents such as buffers and suitable enzymes such as restriction enzymes for maintaining and manipulating the nucleotide molecules of the kit. Additionally, the kit may comprise further means for transforming the target plant such as *agrobacteria* or plant viruses (potato virus X, TMV) when using natural host/vector systems, or specific particles when biolistic methods are to be used for transformation of the target plant. The kit may then further comprise additional reagents required for the transformation.

Under a further aspect, the invention pertains to the newly identified cDNA sequences of the *SVP* gene and to sequences homologous thereto.

Accordingly, the invention is directed to a DNA molecule essentially consisting of or comprising

- a nucleotide sequence as set forth in any one of SEQ ID NO. 1 or SEQ ID NO. 3; or
- a nucleotide sequence having a homology of at least 75%, particularly 80%, preferably 85%, more preferably 90%, even more preferably 95% and most preferably of at least 98% to any one of said sequences; or
- a sequence complementary thereto.

Furthermore the invention encompasses a DNA molecule derived from the above-defined DNA molecule by nucleotide substitution, addition, insertion, deletion and/or modification, which hybrididizes with the latter. Such hybridziation occurs at low stringency conditions, in a further embodiment between low and high stringency conditions and in a very particular embodiment even at high stringency conditions. As a rule, low stringency conditions can be defined as 3 x SSC at ambient temperature to 65°C and high stringency conditions as 0.1 x SSC at 68°C, SSC being the abbreviation of a 0.15 M sodium chloride, 0.015 M trisodium-citrate buffer, pH 7.

Additionally, the invention is directed to a DNA molecule encoding any one of the *SVP* gene products having an amino acid sequence as set forth in any one of SEQ ID. NO. 21 and 22. This takes into account the degeneration of the genetic code.

The invention also pertains to antisense DNA molecules having a nucleotide sequence in "antisense orientation" with respect to the DNA molecules according to the invention. These antisense DNA molecules may comprise as little as 15 nucleotides but will usually comprise between 50 and 200 and even more nucleotides. Encompassed are also antisense DNA molecules comprising the complete nucleotide sequence of a DNA molecule according to the present invention in antisense orientation.

Furthermore, the invention also includes fragments of the DNA molecules of the present inventions. These nucleotide fragments will typically comprise at least 8 nucleotides, preferably at least 15 nucleotides and more preferably more than 25 nucleotides up to several hundred nucleotides. In a very particular embodiment said DNA fragment is still effective in modifying the flowering time in a plant when introduced into the genotype thereof.

The invention is further directed to the use of one or more of these fragments as hybridization probes, particularly screening probes, or primers, in particular sequencing or PCR primers. Especially for use as nucleotide probes, DNA molecules comprising or consisting of one or more of the fragments according to the present invention are employed in a labeled form, e.g. radioactively labeled or labeled by attachment of non-radioactive reporter or indicator molecules, such as digoxigenin molecule(s). Particularly for use as PCR primers, DNA molecules comprising or essentially consisting of one or more of the fragments according to the present invention are employed in a form which comprises modified nucleotide sequences within the sequence(s) of the inventive fragment(s) which provide for appropriate restriction sites within said primers and any amplified products after the PCR which facilitate cloning of said amplified products. Despite these nucleotide sequence modifications the PCR primers, under the PCR conditions, will still hybridize specifically with the target DNA molecule according to the present invention.

DNA fragments according to the present invention may be prepared, for example, on the basis of the nucleotide sequence given in the attached Sequence Protocol, possibly by use of appropriate restriction nucleases, or by chemical synthesis. Various suitable techniques for preparing DNA fragments and DNA molecules comprising one or more of these fragments according to the present invention are known to the skilled scientist.

A further embodiment of the present invention are DNA constructs comprising at least one DNA molecule or fragment according to the present invention. A particular type of DNA constructs are vectors such as plasmids, cosmids, phagemids, viral or phage genomes, YACs or BACs, comprising at least one DNA molecule, fragment or construct according to the present invention.

Within a construct according to the present invention, the inventive DNA molecule of fragment may be under the control of, i.e. operably linked to a promoter enabling the transcription of encoded information contained in the DNA molecule of fragment of the present invention. This promoter may be a constitutive, developmentally regulated or inducible promoter. It may further be a tissue-specific promoter for specific expression of encoded information in a specific plant or animal tissue.

The invention also pertains to DNA molecules comprising a complementary sequence to the DNA molecules, fragments and constructs as outlined above in more detail as well as any RNA transcription products of these DNA molecules, fragments and constructs.

For the isolation, construction, synthesis and modification of the DNA molecules, fragments and constructs, vectors, plasmids, cosmids, viral or phage genomes according to the present invention various methods and techniques are known to the skilled scientist and are, e.g. described in Sambrook et al. (1989).

The nucleic acid molecules and constructs according to the present invention may be provided isolated and/or purified from their natural environment, in particular genetic environment, in substantially pure or homogenous form, or free or substantially free of other nucleic acid or genes of the producing species.

Furthermore, the invention is directed to microorganisms transformed with a DNA molecule or construct, in particular a vector of the present invention.

In the context of the present invention, the term "microorganism" includes bacteria and yeasts, fungi, algae, protozoa as well as human, animal and plant cells, bacteriophages, viruses and plasmids, i.e. all generally unicellular organisms with dimensions usually beneath the limits of vision.

These transformed microorganisms may i.a. be used as an expression system for producing the gene product (s) of the present invention. Typical microorganisms which are used for this purpose are bacteria, like *E. coli,* or yeasts, such as *Saccharomyces cerevisiae.* Other types of transformed microorganisms of the present invention such as *agrobacteria,* like *Agrobacterium tumefaciens,* may be used for the transformation of plants according to the method of the present invention.

Methods and reagents for transforming or transfecting a specific target microorganism with a DNA molecule, construct or vector of the present invention are well known to the skilled specialist. In this respect, for example, reference may be made to the manual of Sambrook et al. (1989).

Furthermore, the invention is directed to a protein or polypeptide encoded by any one of the DNA molecules of the present invention as well as peptide or polypeptide fragments thereof. The invention particularly encompasses peptide and polypeptide fragments of the inventive proteins or polypeptides which upon translation within a plant are still effective in modifying the flowering time in said plant.

In the present context, the term "polypeptide" includes molecules which typically comprise at least 18 amino acids, more particularly at least 25 amino acids and particularly preferred more than 40 amino acids. A polypeptide or polypeptide fragment according to the present invention may also comprise far more than 100 or 150 amino acids.

Further included are peptide or polypeptide fragments comprising at least 9 amino acids which may be specifically recognized and bound by an antibody specifically recognizing and binding a protein or a polypeptide fragment of the present invention.

In a specific embodiment, the invention is directed to a protein or polypeptide comprising the amino acid sequence as set forth in any one of SEQ ID. NOS. 21 or 22 or an amino acid sequence exhibiting at least 85% homology, preferably at least 90%, more preferably at least 95% and most preferably at least 98% homology thereto as well as polypeptide fragments thereof.

The invention also pertains to a protein or polypeptide, in particular a fusion protein, comprising a protein or a polypeptide fragment according to the present invention.

Under a further aspect, the invention is directed to a monoclonal or polyclonal antibody which specifically recognizes and binds a protein or a polypeptide fragment according to the present invention. Corresponding antibodies may be prepared by use of the proteins and polypeptides of the present invention for immunization of a host according to techniques well known to the skilled scientist.

The monoclonal or polyclonal antibody of the present invention may be used for screening plants of interest for the presence of *SVP* genes, more precisely for the presence of gene products of *SVP* genes, which would enable the manipulation of the plants with respect to an earlier flowering time by reducing or eliminating the expression of said genes.

## Figure legends

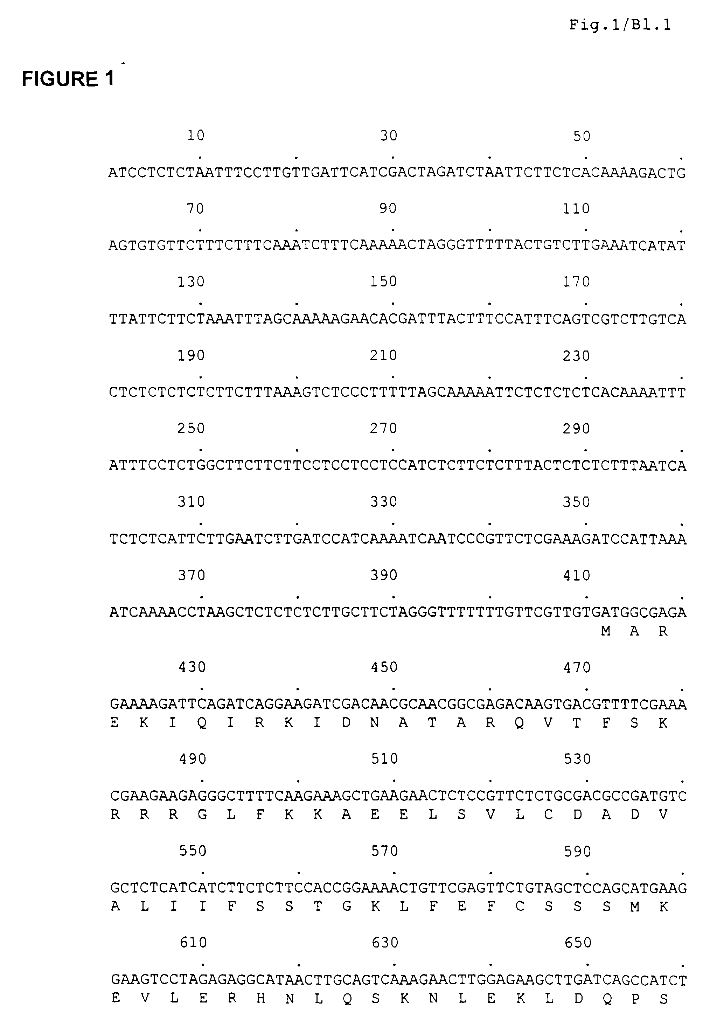

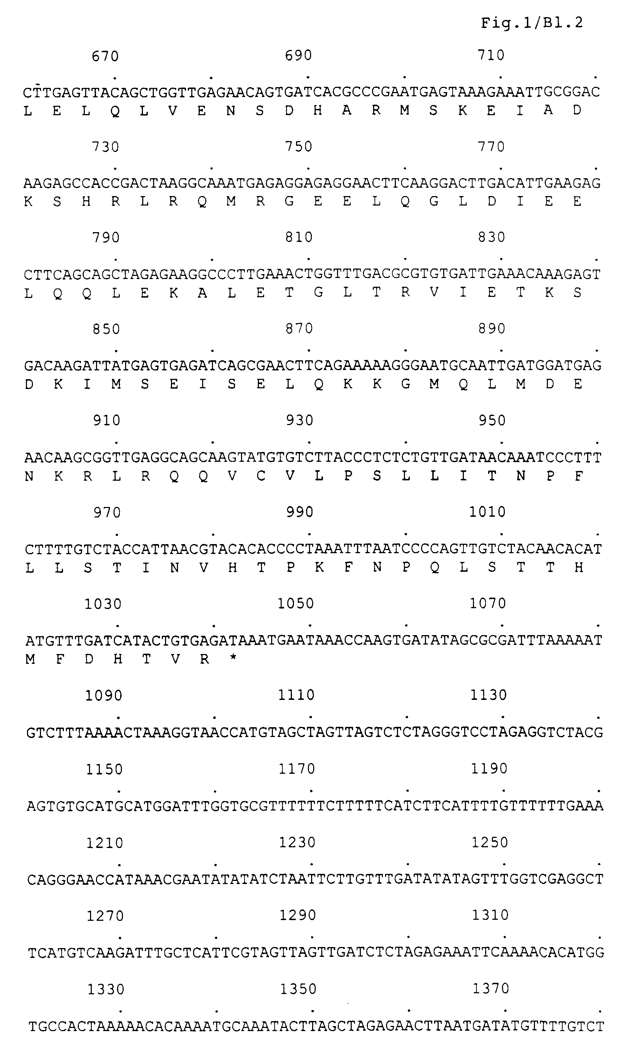

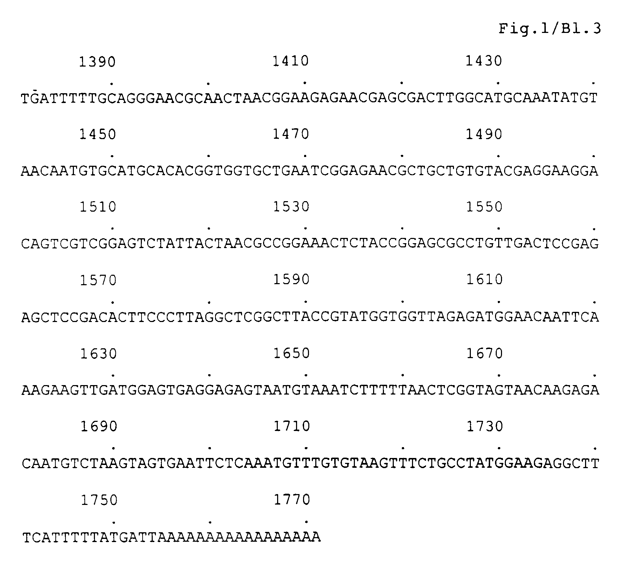

Figure 1 depicts a first *SVP* cDNA nucleotide sequence (SEQ ID NO. 1) including the encoded amino acid sequence of 210 amino acid residues.

Figure 2 depicts a second *SVP* cDNA nucleotide sequence resulting from a different splicing of the SVP transcript (SEQ ID NO. 3) including the encoded amino acid sequence of 240 amino acid residues.

Figure 3 depicts only the SVP amino acid sequence encoded by the *SVP* cDNA nucleotide sequence of Figure 1; (length: 210 amino acid residues) (SEQ ID NO. 21).

Figure 4 depicts only the SVP amino acid sequence encoded by the *SVP* cDNA nucleotide sequence of Figure 2; (length: 240 amino acid residues) (SEQ ID NO. 22).

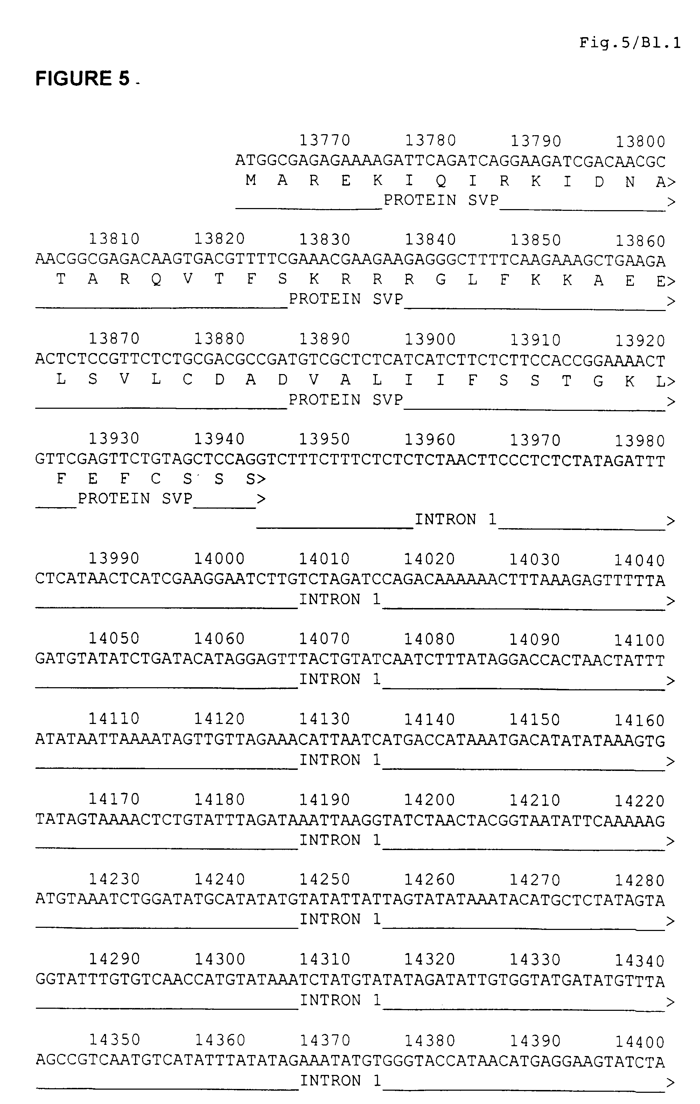

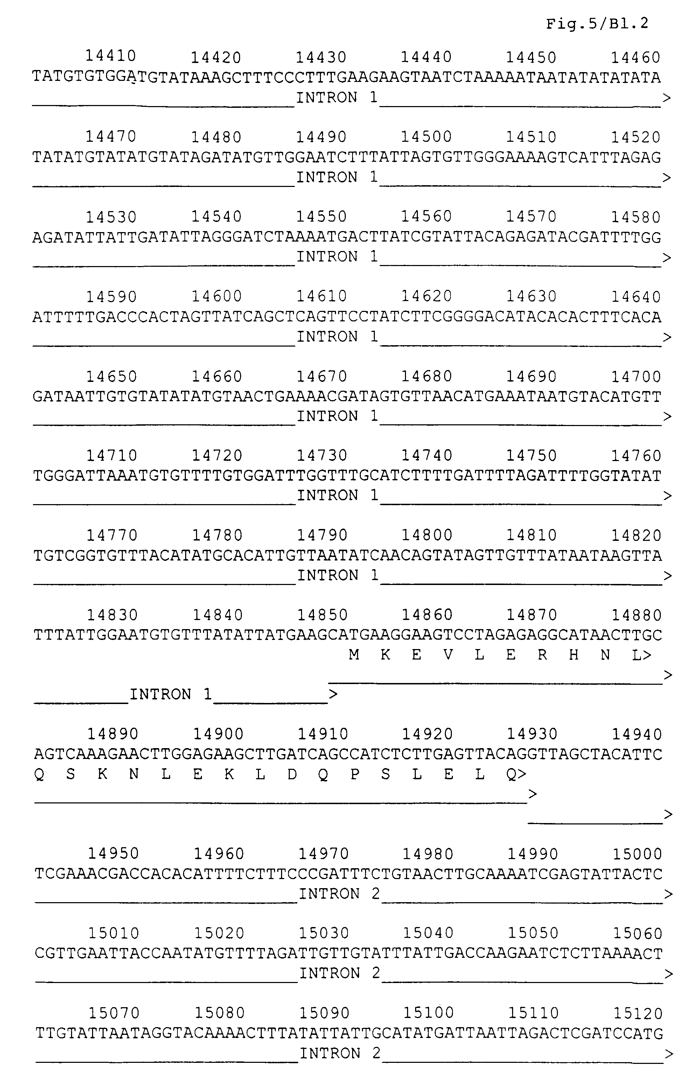

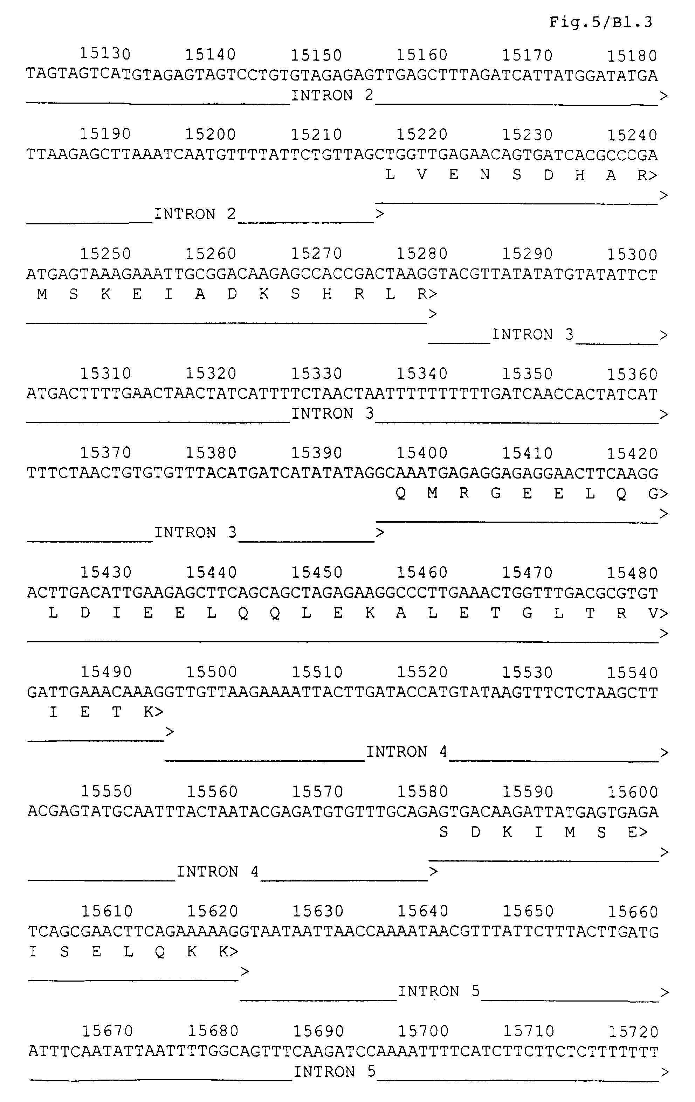

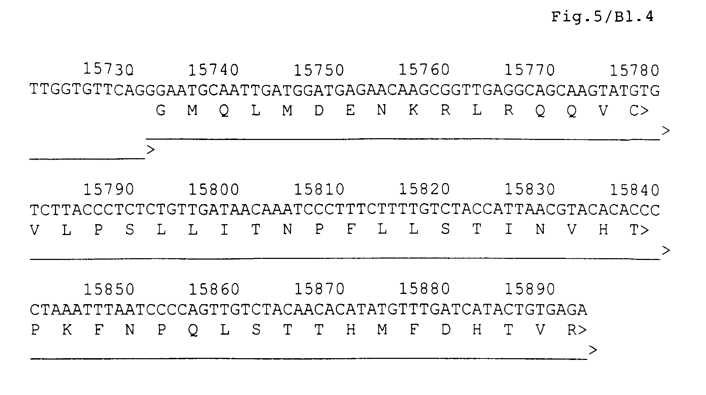

Figure 5 depicts a portion from the sequence of the *SVP* genomic locus covering the *SVP* exon/intron structure leading to the expression of the shorter SVP protein (SEQ ID NO. 21) starting from the ATG at the beginning of the first exon and ending with last coding nucleotide triplet (SEQ ID NO. 5).

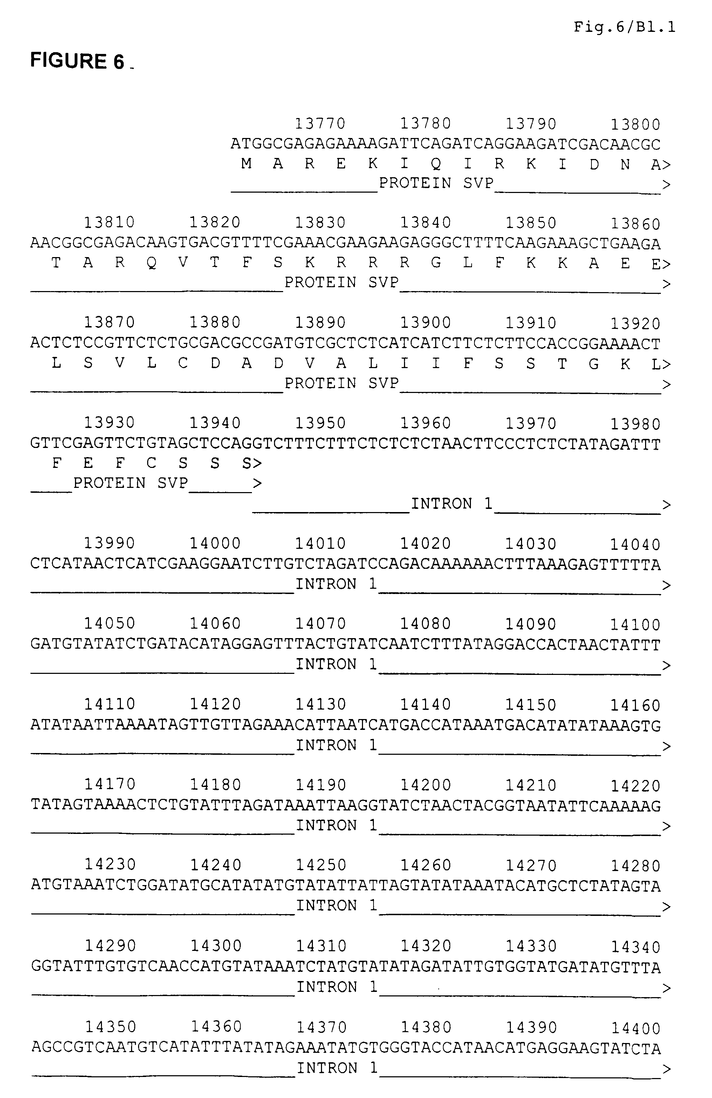

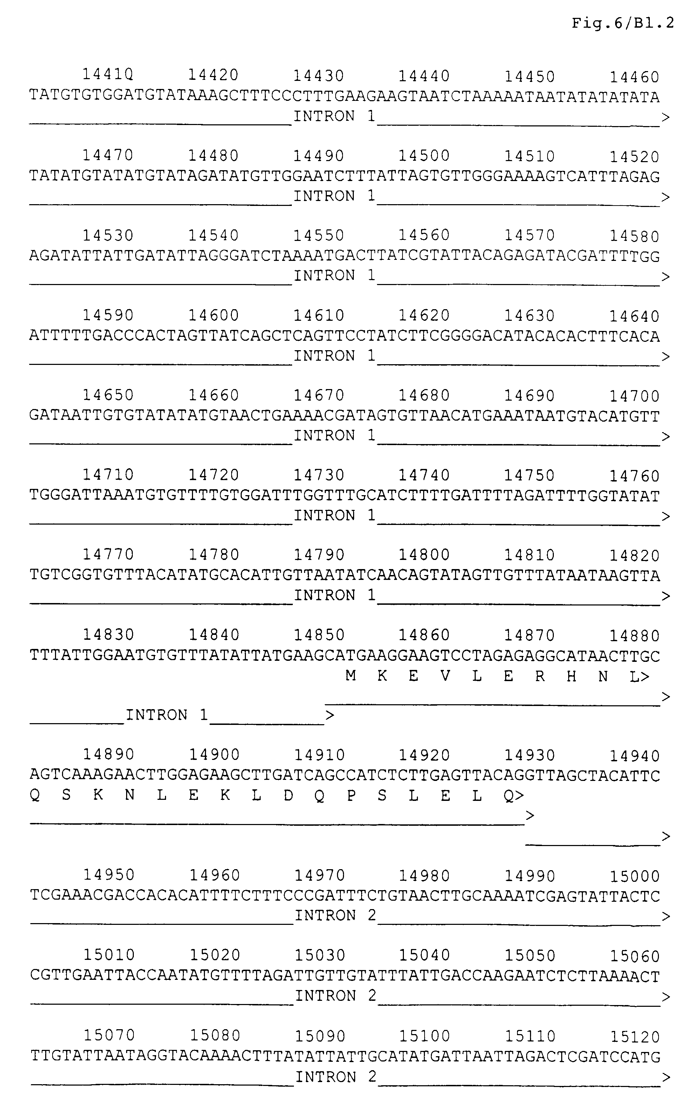

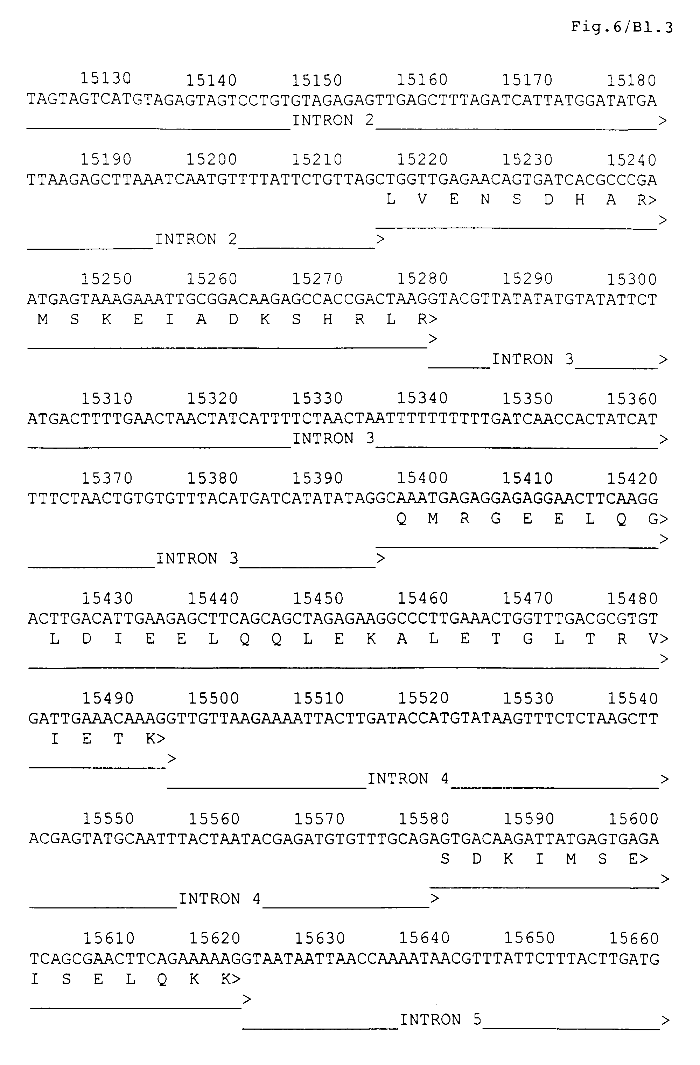

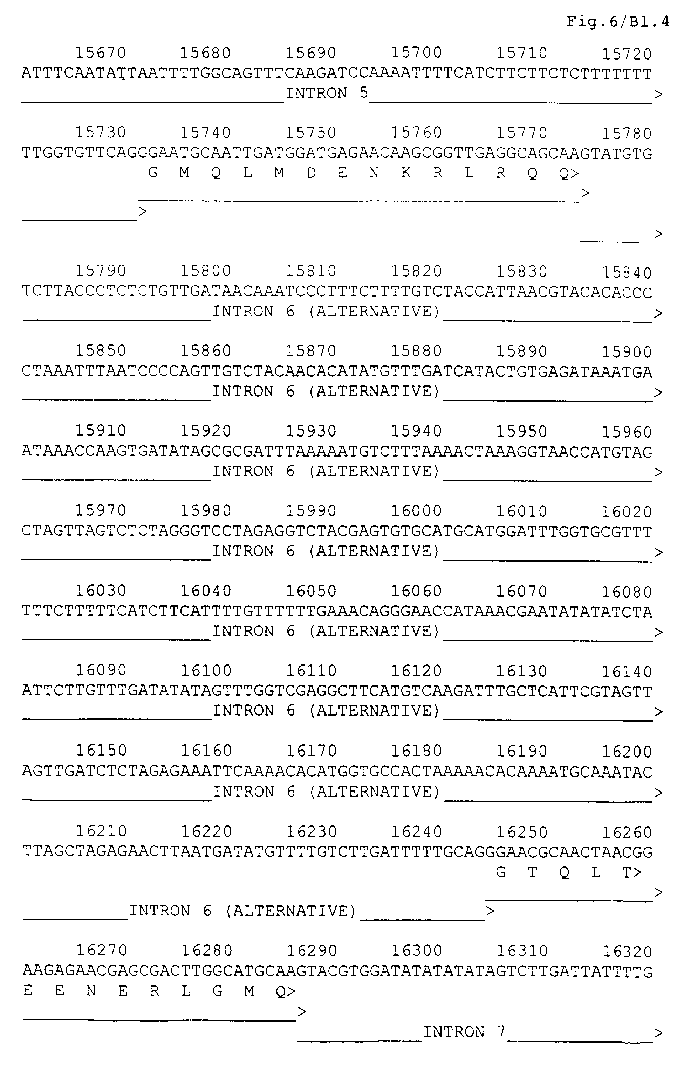

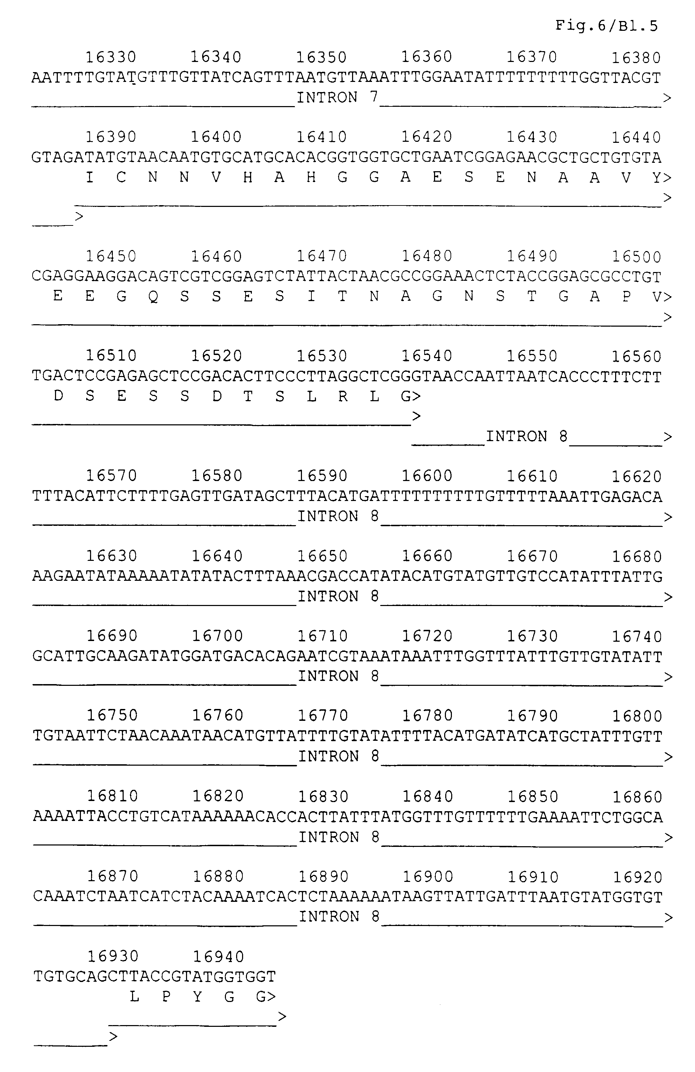

Figure 6 depicts a portion from the sequence of the SVP genomic locus covering the *SVP* exon/intron structure leading to the expression of the larger SVP protein (SEQ ID NO. 22) starting from the ATG at the beginning of the first exon and ending with last coding nucleotide triplet (SEQ ID NO. 6).

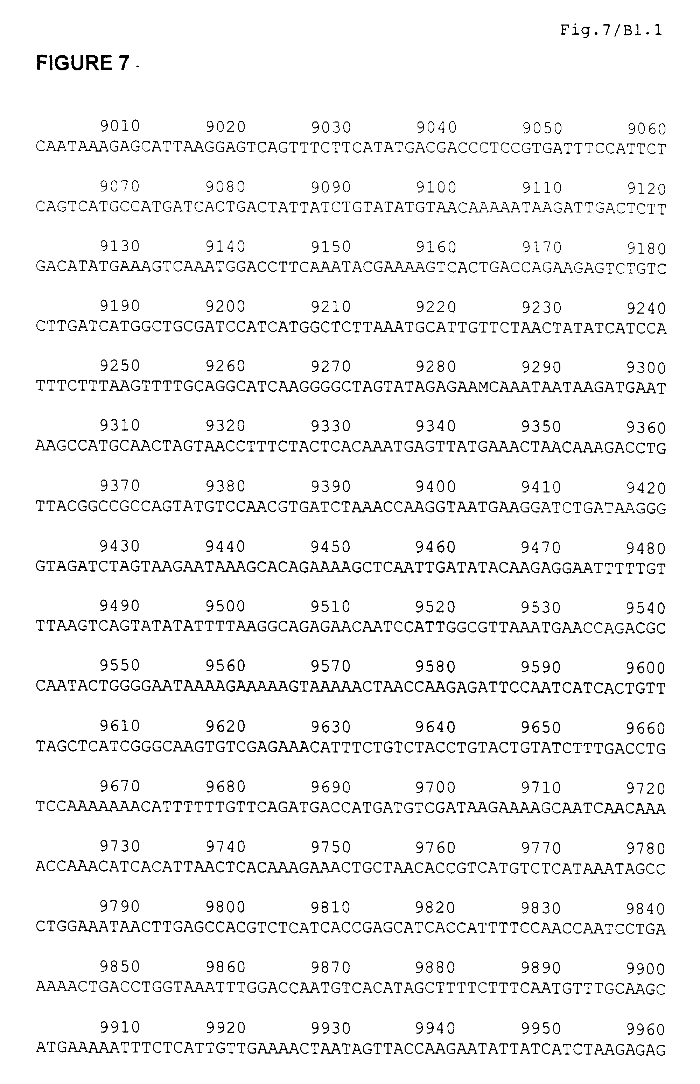

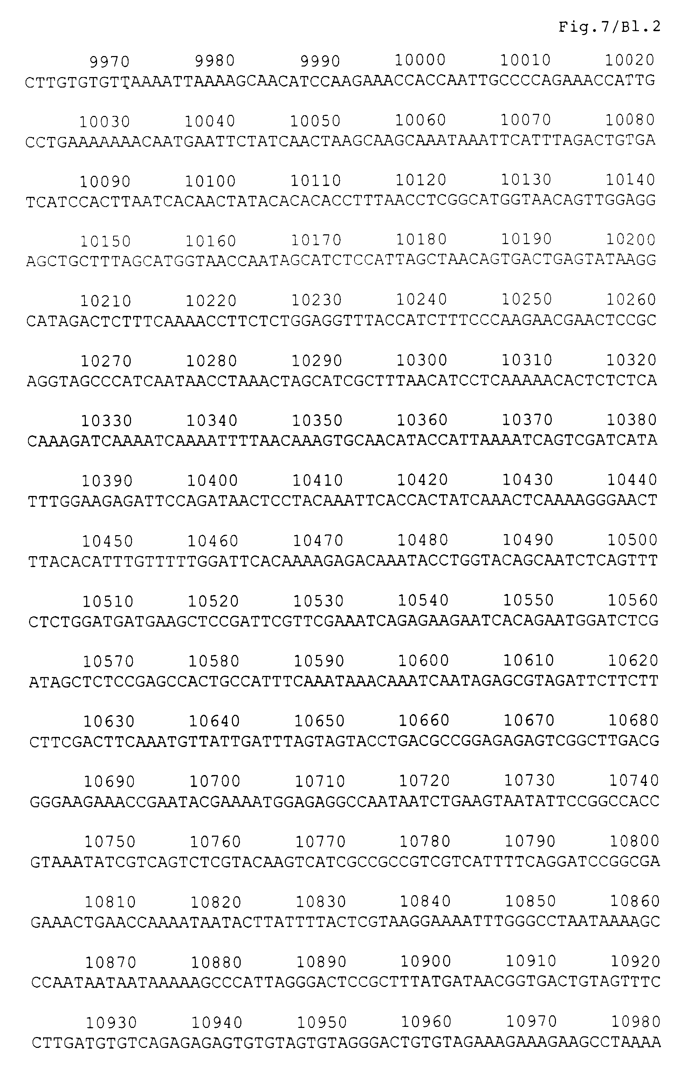

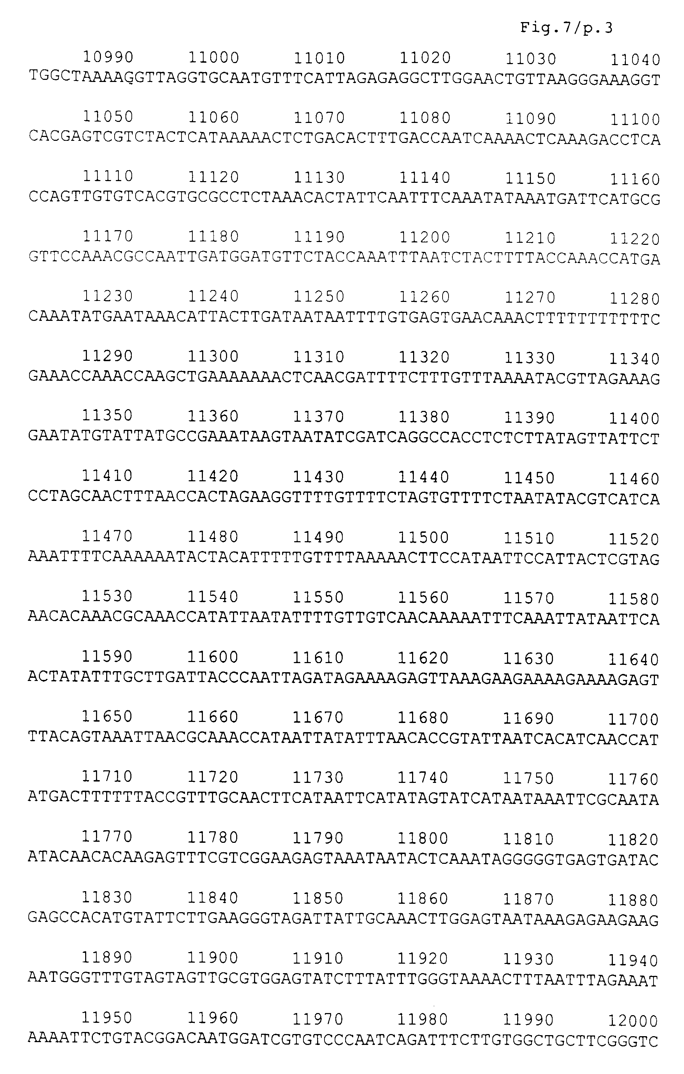

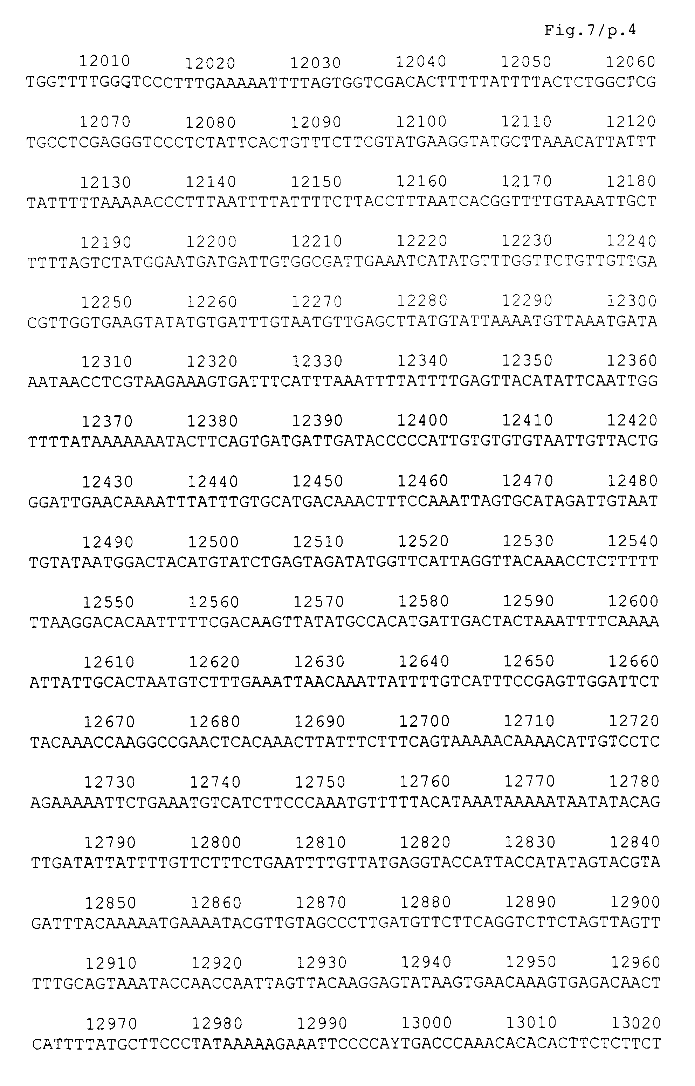

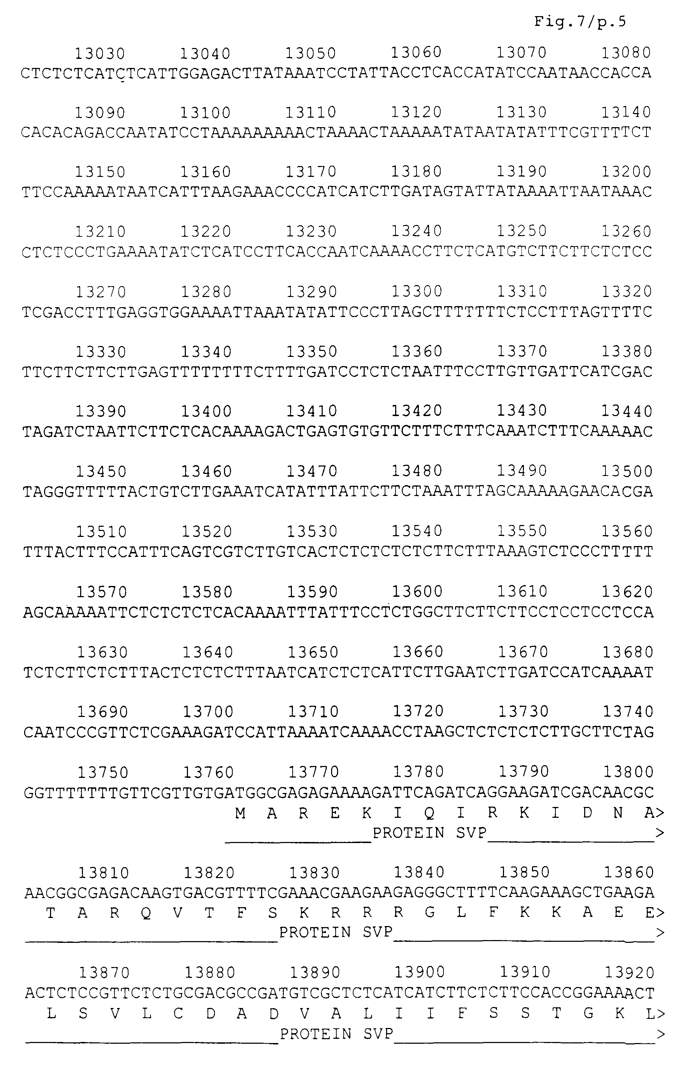

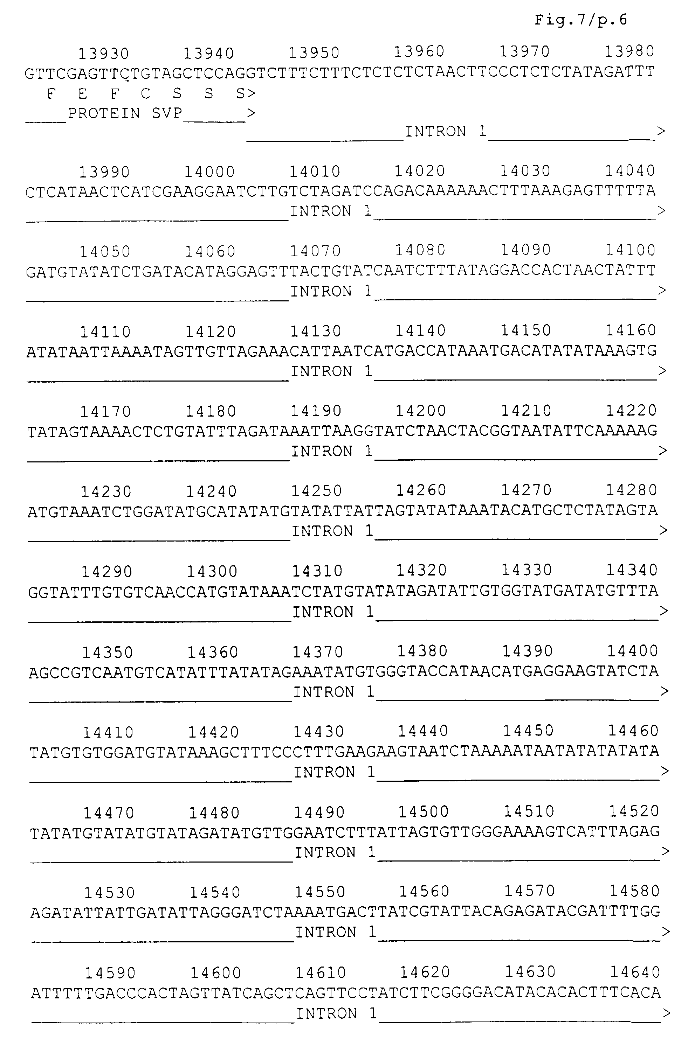

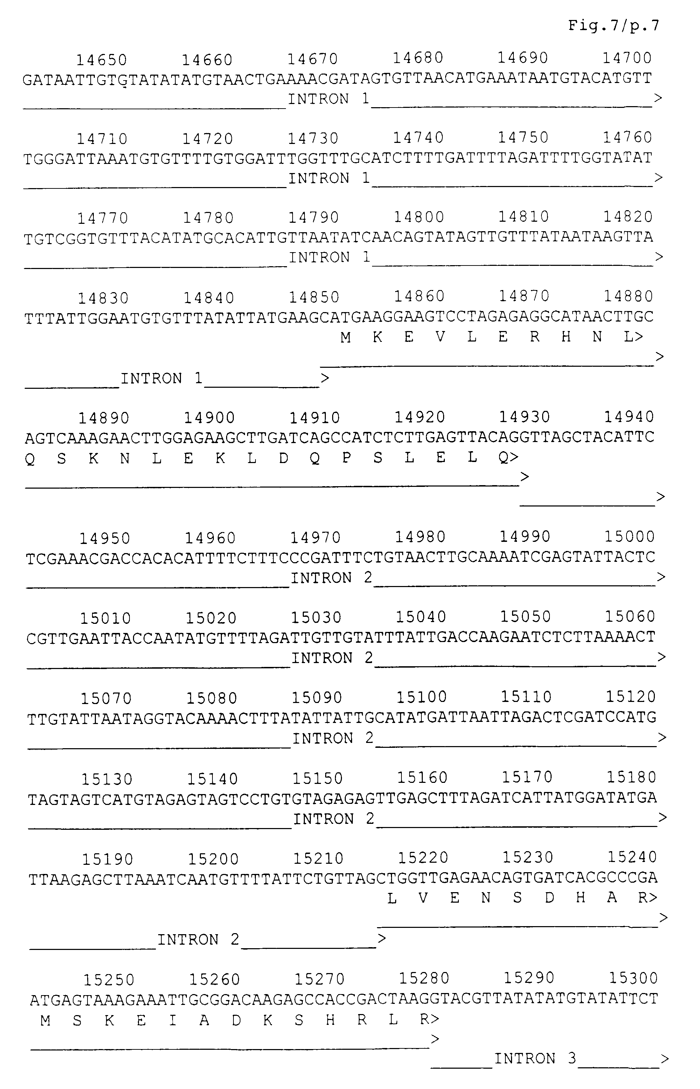

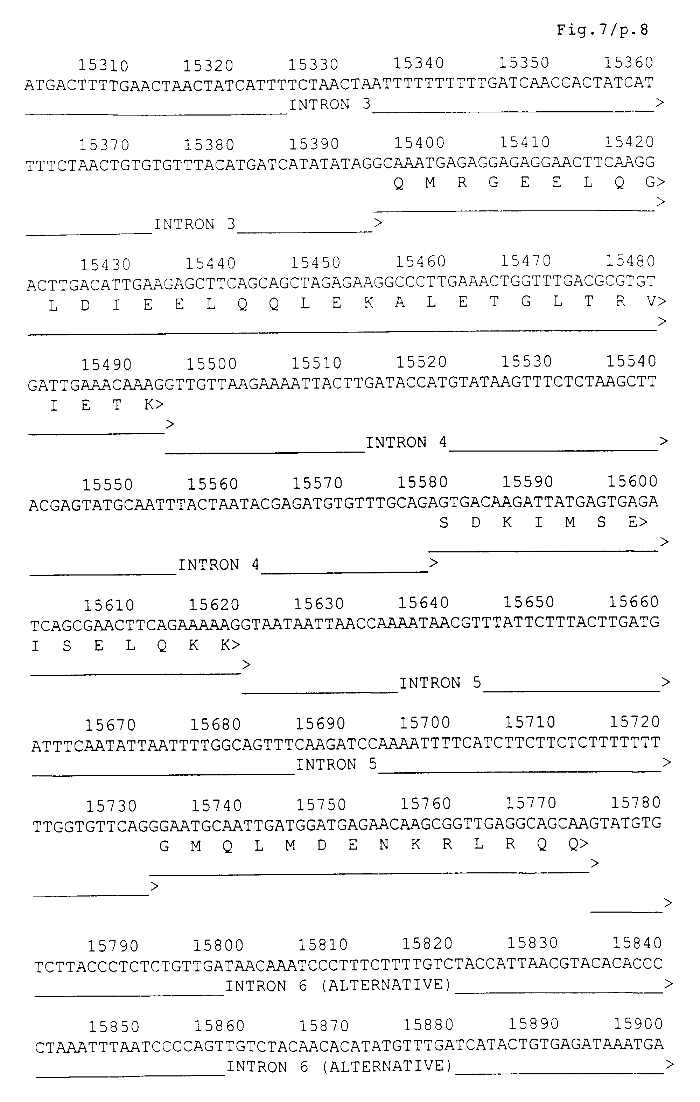

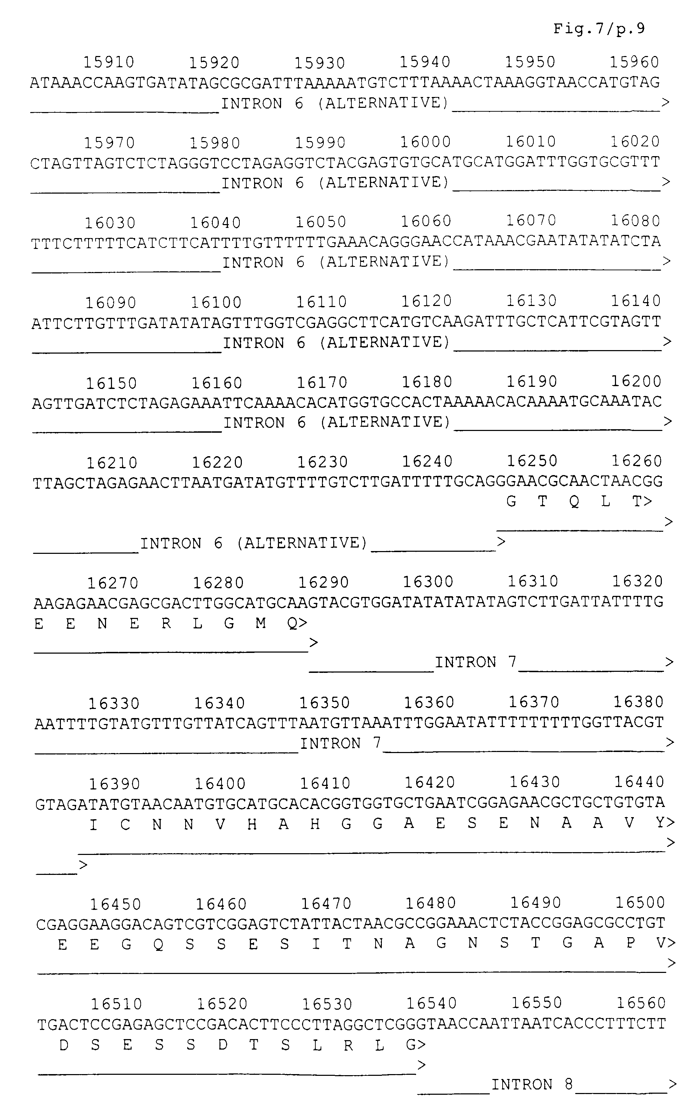

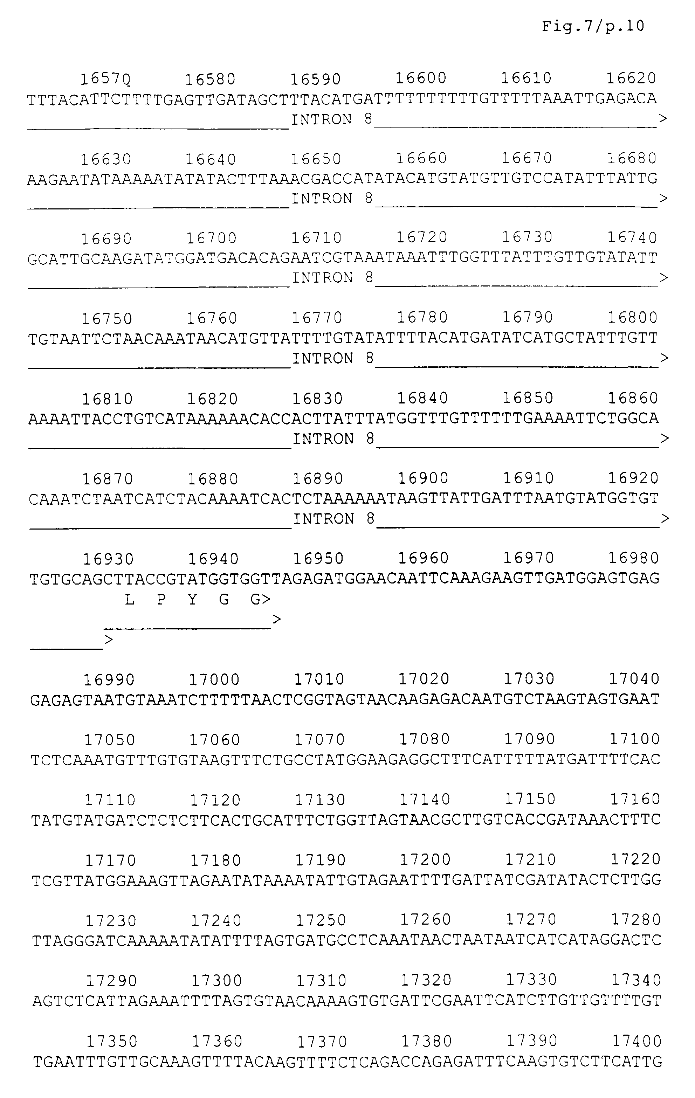

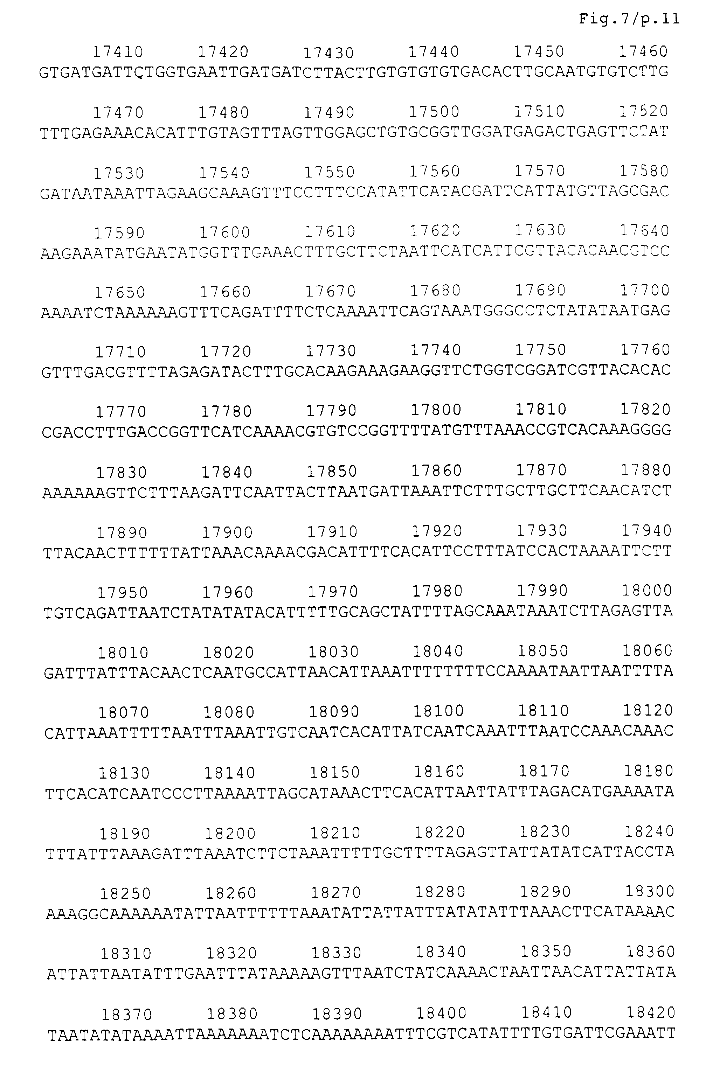

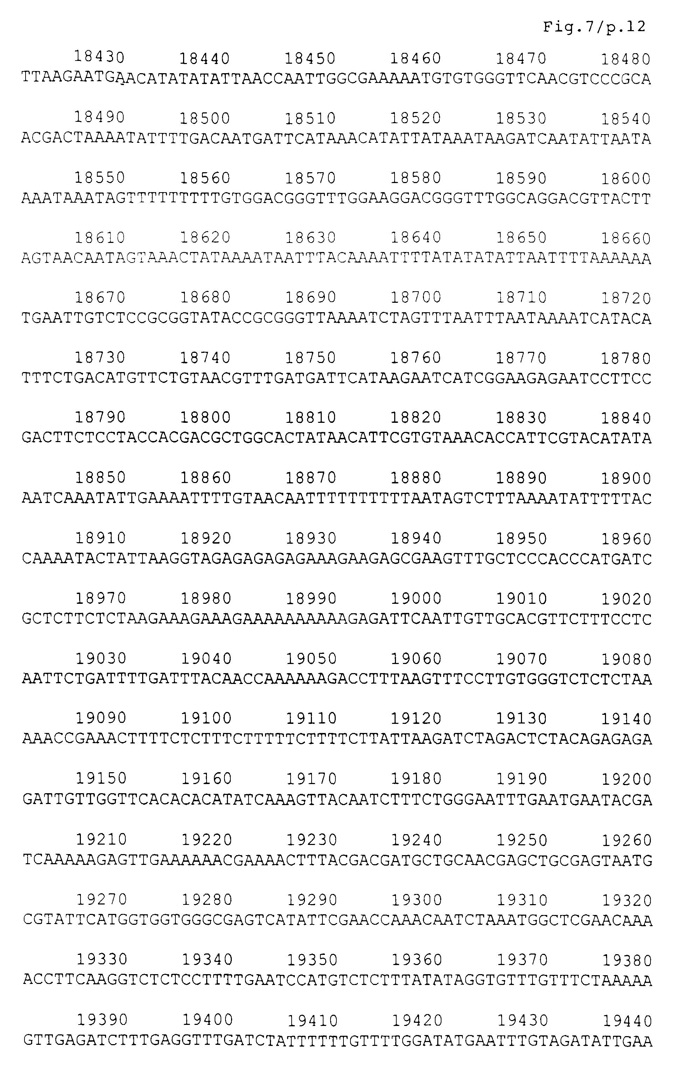

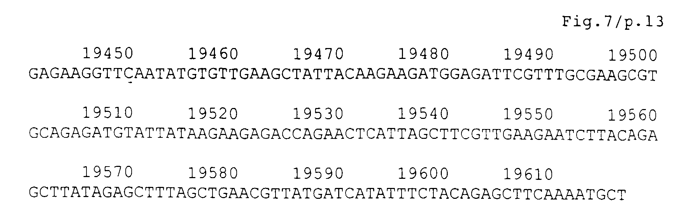

Figure 7 depicts a larger portion of the *SVP* genomic sequence (with indicated *SVP* exon/intron structure leading to the expression of the larger SVP protein; see Figure 6) including also portions of the 5' and 3' untranscribed regions (SEQ ID NO. 7).

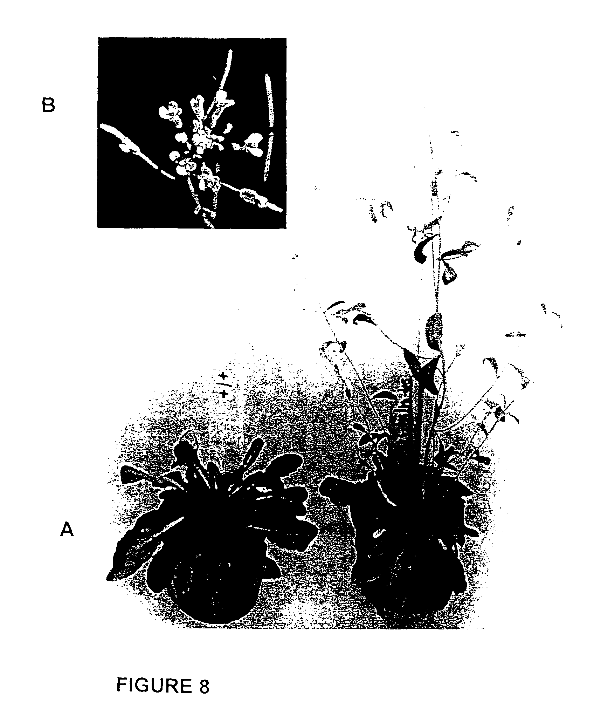

Figure 8 is a photograph showing the phenotype of homozygous svp mutant and *A. thaliana* Columbia wildtype plants.

(A) The homozygous *SVP* (allele 41) mutant plant (right) flowers earlier and with a reduced total number of leaves (TLN) compared with the Columbia wildtype reference plant (left). Both plants are the same age and were grown under the same environmental conditions (short day (SD); 22°C).

(B) Inflorescence and flower morphologie is not altered in the mutant plant.

## EXAMPLES

### SUMMARY

The *SVP* gene could be cloned through transposon tagging and was found to be expressed mainly in vegetative tissues like roots, leaves and stem. No or strongly reduced expression could be detected in inflorescences, flowers and siliques. Due to alternative splicing of the *SVP* gene, a new member of the MADS-box class of transcription factors, encodes at least two proteins differing in their C-terminal sequences.

1) Identification and morphological description of a *svp* mutant

An *En-1* mutagenized *A. thaliana* population previously described by Wisman et al., 1998, was screened for early flowering mutants. For the evaluation of flowering time (FT) the total leaf number (TLN) was determined. TLN is the sum of primary rosette and cauline leaves and correlates well with FT (Napp-Zinn, 1969; Koornneef et al., 1991). By visual inspection of a collection of about 30000 mutagenized plants 13 early flowering mutants could be identified. The mutant which is described here was named *svp* for "short vegetative phase". It was selected from the other mutants because it showed one of the most severe early flowering phenotypes among the identified mutants. Under long day (LD) conditions *svp* flowered with significantly lower TLN than Col wildtype control plants. Under the particular conditions used, the average TLN of Col wildtype was observed to be 15 and the average TLN of *svp* mutant plants was observed to be 9.

2) Molecular cloning and characterization of the *SVP* gene

Because the *svp* mutant was found to have multiple copies of the *En-1* transposon inserted in its genome, the causal relationship between the observed *svp* phenotype and a particular insertion had to be established. Phenotypic restoration of the wild type phenotype upon excision of *En-1* from the locus provided this proof.

Nine phenotypic revertants were obtained from the offspring (540 S1 plants grown in a greenhouse) of the original *svp* plant identified in the mutant screen. Two revertant plants (20010 and 20012) displaying a TLN of 10 and 11, respectively, were selected for further characterization. Wildtype plants (Col 0) grown under the same conditions displayed a TLN of 13, *svp* mutants of 8. Progeny (S1) of the selected revertants were grown under short day (SD) conditions to create a segregating population with respect to flowering time. 4 out of a total of 22 plants derived from 20010 and 5 out of 17 plants derived from 20012 displayed an early flowering phenotype which is in good correlation with the expected one fourth of the progeny being early. To identify the transposon insertion responsible for the early flowering phenotype southern analysis was performed with the S1 progeny of the two selected revertants. A probe corresponding to the 3' end of the *En-1* transposon detected a single *EcoRV* fragment of about 2.8kb which cosegregated with plants showing the early flowering phenotype. A second hybridizing fragment was present in (almost) all plants regardless of their flowering phenotype.

Using a PCR based procedure (see materials and methods) DNA flanking the tagged *SVP* locus was isolated from genomic DNA of the originally identified *svp* mutant. A database search (Altschul et al., 1990; Gish et al., 1993) with this flanking DNA fragment revealed that a central portion of *SVP* corresponds to a portion of EST T21923. This EST displays high homology to genes of the MADS-box gene family although the expected MADS-box in the N-terminus is missing in T21923.

To isolate full-length cDNA clones a cDNA library prepared from poly A(+) RNA derived mainly from green parts of *A. thaliana* plants ecotype Columbia was screened. Sequence comparison of the EST sequence with the cDNA clones derived from the cDNA library screen revealed that the latter contains an additional intron. The genomic sequence of the *SVP* gene was determined by sequencing two out of four IGF-BAC clones (14M13 and 12H6; obtained from the Resource Center of the German Human Genome Project, Berlin, Germany) which gave positive signals after hybridisation with the originally isolated *SVP* fragment.

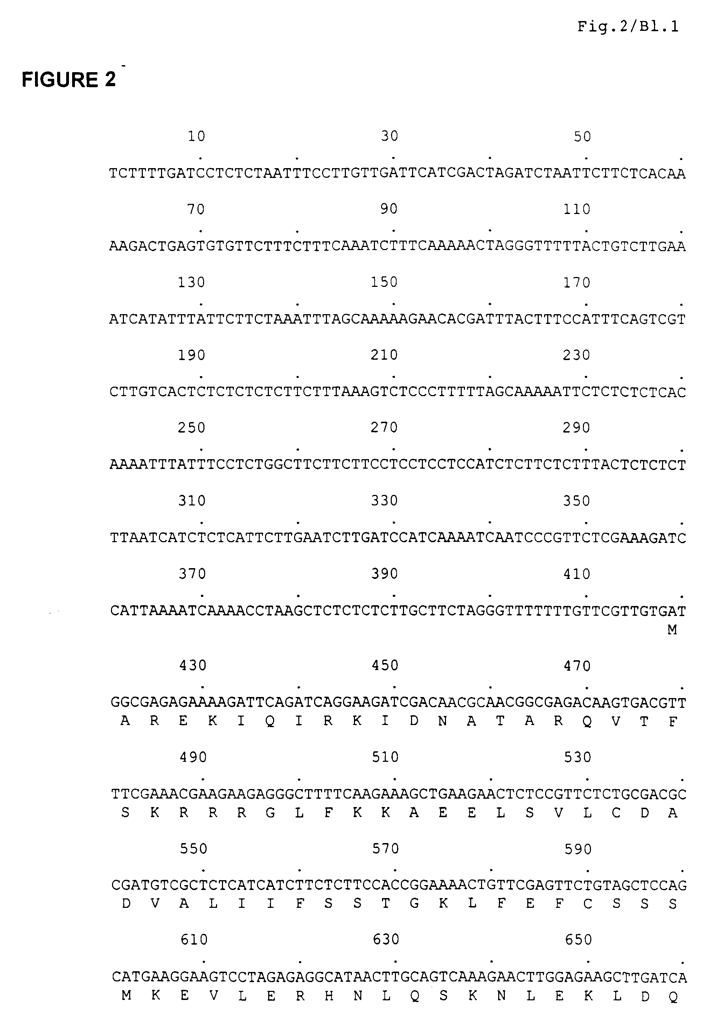

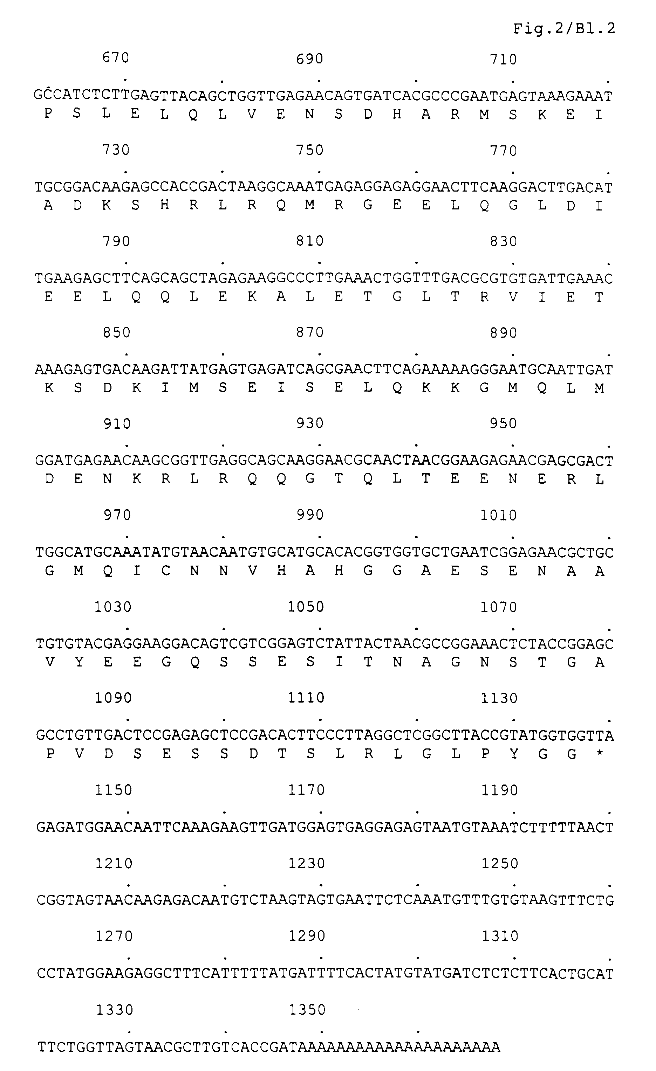

While this work was in progress the sequence of the IGF-BAC clone 14M13 became partly available. The intron-exon structure of *SVP* was derived from comparison of the cDNA sequences and the BAC sequence information. The transcribed region according to the cDNA obtained from the screening of the cDNA library consists of 9 exons and 8 introns (Fig. 2). As deduced from the cDNA nucleotide sequence, the *SVP* gene encodes a 240 amino acid residue protein. When deduced from the EST sequence the transcribed region consists of 8 exons and 7 introns only and the resulting protein consists of 210 amino acid residues. The first 170 amino acid residues are identical in the two potential proteins. A stop codon 5' of the ATG start codon verifies this ATG to be the correct start codon. The conserved MADS-box and K-box domains are present in the protein as well as the more variable I and C-terminal domains. From four different cDNA clones at least three different polyadenylation sites were found.

Analysis of the mutant *svp:En-1* allele revealed that the *En-1* transposon is integrated into the second exon of the gene which contains the sequence for the I-domain. A three bp target site duplication which is characteristic for the *En-1* transposable element was found.

The map position of the *SVP* gene was determined by hybridisation of the SVP probe to the IGF-BAC library. Positive signals could be obtained with four different BAC clones (14M13, 12H6, 21A23, 7022, all obtained from the Resource Center of the German Human Genome Project, Berlin, Germany) which are all anchored to chromosome 2 between the markers mi148 and mi238.

3) Evaluation of the flowering time of heterozygous and homozygous *svp* mutants, respectively:

From the *Arabidopsis thaliana* revertant 20012 obtained above offspring (S1 progeny) was produced. From the S1 plants, one plant was found to be heterozygous carrying one wildtype *SVP* allel and one mutated svp allel carrying a 2 bp deletion (frameshift mutation). Offspring of this heterozygous *svp* mutant, segregating for homozygous wildtype plants, heterozygous as well as homozygous *svp* mutants, was subsequently grown under long day and short day conditions, respectively, and the bolting time, the flowering time as well as the number of leaves on the main stem before the first flower have been determined. The results are shown in the Tables 1 and 2 below.

Table 1:

| *Arabidopsis thaliana* plants grown under long day conditions (16 hrs light/8 hrs dark) at 22°C | | | |
|---|---|---|---|
| | bolting time (days after germination) | flowering time (days after germination) | number of leaves on main stem before first flower |
| *SVP/SVP:* homozygous *SVP* wildtype in *A. thaliana* (Ecotype Col) background | 18 | 22 | 16 |
| *SVP/svp:* heterozygous *svp* mutant in *A. thaliana* (Col) background | 16 | 20 | 14 |
| *svp/svp:* homozygous *svp* mutant in *A. thaliana* (Col.) background | 14 | 18 | 11 |

The given numbers are average values obtained from the evaluation of 25 to 50 plants each.

Table 2:

| *Arabidopsis thaliana* plants grown under short day conditions (8 hrs light/16 hrs dark) at 22°C | | | |
|---|---|---|---|
| | bolting time (days after germination) | flowering time (days after germination) | number of leaves on main stem before first flower |
| *SVP*/*SVP:* homozygous *SVP* wildtype in *A. thaliana* (Ecotype Col) background | 59 | 67 | 63 |
| *SVP*/*syp:* heterozygous *svp* mutant in *A. thaliana* (Col) background | 53 | 60 | 53 |
| *svp*/*svp*: homozygous *svp* mutant in *A. thaliana* (Col.) background | 33 | 43 | 24 |

The given numbers are average values obtained from the evaluation of 9 to 15 plants each.

Materials and Methods

DNA sequencing and sequence analysis

Sequences were determined on Applied Biosystems 377 sequencers (Applied Biosystems, Weiterstadt, Germany) by using dye terminator chemistry. The analysis of DNA sequences was performed using the GCG program package (Version 9.0) from the Genetics Computer Group, Inc., Madison, Wisconsin. Oligonucleotides were purchased from Life Technologies, Eggenstein, Germany.

Isolation of genomic DNA and Southern blot analysis

Genomic DNA was prepared from *Arabidopsis* leaves by using a CTAB-based extraction procedure. The leaf material (1 to 1.5g) was ground with mortar and pistil and the resulting powder resuspended in 10ml of CTAB extraction buffer (100mM Tris/HCl pH 8.0, 1.4M NaCl, 20mM EDTA, 2% CTAB). After incubation at 60°C for 30 min the mixture was extracted once with 1 Vol of chloroform. Nucleic acids were precipitated from the aqueous phase by addition of 0.1 Vol of 3M NaOAc and 0.8 Vol of isopropanol. After incubation at RT for 5 min the precipitate was collected by centrifugation. The pellet was washed once with 70% ethanol and dissolved in 1880μl TE pH 8.0. After RNase treatment (final concentration of 2μg/ml) at 65°C for 15 min 120μl of 5M NaCl were added to the solution. The genomic DNA was purified by applying the solution onto a QIAGEN tip 20 column (QIAGEN, Hilden, Germany) according to the supplier's protocol. After washing twice with lml buffer QC (QIAGEN) the genomic DNA was eluted from the column with 800μl buffer QF (QIAGEN) and precipitated by addition of 500μl isopropanol. The precipitated DNA was pelleted, washed with 70% ethonol and dissolved in 100-200μl TE buffer. Genomic DNA was cleaved with restriction endonucleases and the fragments were separated overnight on a 0.8% agarose gel. DNA was blotted to Hybond N(+) according to Koetsier et al. 1993 and hybridised to a probe corresponding to the 3' end of the *En-1* transposon as well as probes comprising the *En-1* flanking sequences as mentioned above.

Hybridisation of IGF-BAC-filters and isolation of BAC-DNA

IGF-BAC-filters and clones were obtained from the Resource Center of the German Human Genome Project (Berlin, Germany). Hybridisation of filters were performed according to the supplier's protocol. The correct identification was confirmed by DNA-blot analysis using *EcoRV*-restricted DNA isolated from candidate-bacteria lines. Information about the genomic localization was obtained from Internet resources (http://www.mpimp-golm.mpg.de/101/mpi_mp_map/bac.html). BAC-DNA was isolated by using the QIAGEN Plasmid Maxi Kit (tip 500) according to the supplier's protocol with some modifications. A 5ml starter culture (LB, 50μg/ml kanamycin) was inoculated with a single colony and incubated overnight at 37°C. 400ml medium were inoculated with lml of the starter culture and incubated at 37°C for 16 hours. The following modifications were carried out: 50ml instead of 10ml P1, P2 and P3 buffers were used. DNA was eluted from the tip 500 with 5 x 3ml prewarmed (65°C) buffer QF. The DNA was dissolved in 300-500μg

10mM Tris/HCl pH 8.5. BAC-DNA was directly sequenced.

Standard molecular biology techniques

Basic molecular biology techniques were applied according to Sambrook et al. 1989. Radiolabelled probes were derived from gel-purified DNA fragments or PCR products.

Isolation of *En-1* flanking regions

After a *Csp6*I restriction of 1µg of genomic DNA derived from the originally identified svp mutant the sample was purified using the QIAquick spin PCR Purification kit (QIAGEN) according to the suppliers protocol. The DNA was eluted from the column with 50µl of prewarmed (55°C) elution buffer. 2.5µl of the purified DNA was used for a linker ligation reaction using 12.5 pmoles of linker. The linker was prepared by annealing of the oligonucleotides 5'-ACTCGATTCTCAACCCGAAAGTATAGATCCCA-3' (LR32) and 5'-p-TATGGGATCACATTAA-amino-3' (APL 16). After ligation at 16°C overnight a primer extension reaction was carried out. PCR conditions were as follows: 50µl total volume, 4µl of linker ligated genomic DNA, 20 pmoles of *En-1* specific primer (5'-AGAAGCACGACGGCTGTAGAATAGGA-3'; En205R), 2.5 U Taq polymerase, cycle program: 2 min, 94°C, 40x (35 sec, 94°C; 1 min, 64°C; 1 min 30 sec, 73°C), 3 min 73°C). The product of the primer extension reaction was diluted 1:100 and used as a template (1µl) for a second PCR using the *En-1* specific primer 5'-TGCAGCAAAACCCACACTTTTACTTC-3' (En91R) and the linker specific primer 5'-ACTCGATTCTCAACCCGAAAGTATAG-3' (LR26). PCR conditions were as described above. The two resulting bands were subcloned into pCR-TOPO-2.1 (Invitrogen NV, Leek, The Netherlands) and sequenced. One clone corresponded to the *SVP* locus.

RNA isolation and Northern blot analysis

For isolation of poly(A)+ RNA *Arabidopsis* plants were grown either under short (8h light, 16h dark) or long (16h light, 8h dark) day conditions in a phytochamber as described by Cardon et. al, 1997. RNA was isolated as described by Logemann et al, 1987. Poly(A)+ RNA enrichment with Oligo$(dT)_{25}$-Dynabeads (Dynal, Oslo, Norway) was performed as recommended by the manufacturer. For isolation of RNA from different plant organs *Arabidopsis thaliana* plants ecotype Columbia were grown for 12 weeks under short day conditions in a phytochamber. Total RNA was prepared as described by Feldbrügge et al, 1994. RNA was fractionated in 1% agarose gels containing formaldehyde, blotted on Hybond N+ (Amersham Buchler, Braunschweig, Germany) and hybridized to sequence specific probes for detection.

*Agrobacterium* binary vector construction, *Agrobacterium* transformation and subsequent transformation of *Arabidopsis thalia*na plants

For the production of transgenic plants expressing a transgene encoding the 210 amino acid residue *SVP* gene product (SEQ ID NO. 2; Fig. 3), gene fusions between the CaMV 35S promoter and the *SVP* ORF in sense and antisense orientation were constructed in the *Agrobacterium* binary vector pBAR-35S. A *XbaI* fragment of *SVP* carrying its endogenous start and stop codon was generated by PCR with *SV*P specific primers containing *XbaI* restriction sites at their 5' ends (primer oU9 5'-GCGTCTAGAATGGCGAGAGAAAAGATTCAGATCAG-3' and oU10 5'-GCGTCTAGATTATCTCACAGTATGATCAAACATATGTG-3'). The PCR was performed using *Pfu* turbo DNA polymerase (Stratagene, Amsterdam, The Netherlands). The template for the PCR reaction was a cDNA pool generated by using the SMART PCR cDNA Synthesis Kit (Clontech, Heidelberg, Germany) with minor modifications. The first strand synthesis was performed using 500µg polyA (+) RNA of 2 week old *Arabidopsis* plants grown under short day conditions and primers S313 5'-CAGCTAGTCAAGGATCCGAATTCGAGCGGG-3' and GC361 5'-AAGCAGTGGTAACAACGCAGAGTAC$(T)_{23}$-3'. PCR with the first strand reaction as template was carried out to generate the cDNA pool described above using primers GC362 5'-AGCTAGTCAAGGATCCGAATTCGA-3' and GC360 5'-GCAGTGGTAACAACGCAGAGTACTTT-3'. The PCR was performed using *Pfu* turbo DNA polymerase (Stratagene) and the following program: 4 min 95°C / 30 s 95°C, 30 s 60°C, 5 min 30 s 72°C, 15x / 10 min 72°C. The final PCR product was digested with *XbaI* and cloned in sense orientation into the unique *XbaI* site of the polylinker of the CaMV 35S promoter/terminator cassette of pBAR-35S. The resulting plasmid was named pBAR-35S::*SVP*se. The corresponding *SVP* antisense plasmid was constructed that way that it does not contain the conserved MADS-domain. PCR was carried out using the cDNA pool described above and primer oH13 5'-GCGTCTAGAATGAAGGAAGTCCTAGAGAGGCATAAC-3' and primer oU10. After restriction with *XbaI* the resulting PCR fragment was cloned in antisense orientation into pBAR-35S.

For the production of transgenic plants expressing a transgene encoding the larger 240 amino acid residue *SVP* gene product (SEQ ID NO. 4; Fig. 4), a binary vector construction was carried as outlined above with the exception that the primer oU10 was replaced by the primer oU26: 5'-GCGTCTAGACTAACCACCATACGGTAAGCCGAGCCTAAG-3' both times.

The transformation of *Agrobacterium tumefaciens* with any of the resulting binary vector constructs, respectively, was carried out by electroporation according to Cardon et al. 1997.

With the resulting transformed *agrobacteria, Arabidopsis thaliana* ecotype Columbia wildtype plants were transformed according to Bechthold et al., 1993, with minor modifications.

References:


Altschul, S.F., Gish, W., Miller, W., Myers, E.W., Lipman, D.J. (1990). Basic local alignment search tool. Journal of Molecular Biology 215 (3), 403-410.

Amasino, R.M. (1996). Control of flowering time in plants. Current Opinion in Genetics & Development 6(N4), 480-487.

An, G., Kang, H.G. and Gupta, H. (1997). Rice mads box genes controlling flowering time. Plant Physiology 114, 213-213.

Baumann, E., Lewald, J., Saedler, H., Schulz, B. and Wisman, E. (1998). Successful PCR-based reverse genetic screens using an *En-1*-mutagenised *Arabidopsis thaliana* population generated via single-seed descent. Theor. & Appl. Gen. 97, 729-734.

Bechthold, N., Ellis, J. and Pelletier, G. (1993). *In planta* Agrobacterium-mediated gene transfer by infiltration of adult *Arabidopsis thaliana* plants. C.R. Acad. Sci. Ser III Sci. Vie 316, 1194-1199.

Cardon, G.H., Hoehmann, S., Nettesheim, K., Saedler, H. and Huijser, P. (1997). Functional analysis of the *Arabidopsis thaliana* SBP-box gene *SPL3* - a novel gene involved in the floral transition. Plant Journal 12 (2), 367-377.

Chung, Y.Y., Kim, S.R., Finkel, D., Yanofsky, M.F. and An, G. (1994). Early flowering and reduced apical dominance result from ectopic expression of a rice MADS box gene. Plant Molecular Biology 26, 657-65.

Colasanti, J. and Sundaresan, V. (1996). Control of the transition to flowering. Current Opinion in Biotechnology 7(N2), 145-149.

Faske, M., Backhausen, J.E., Sendker, M., Singer-Bayrle, M., Scheibe, R. and von Schaewen, A. (1997). Transgenic tobacco plants expressing pea chloroplast *Nmdh* cDNA in sense and antisense orientation: Effects on NADP-MDH level, stability of transformants and plant growth. Plant Physiol. 115, 705-715.

Feldbrügge, M., Sprenger, M., Dinkelbach, M., Yazaki, K., Harter, K., Weisshaar, B. (1994). Functional Analysis of a Light-Responsive Plant bZIP Transcriptional Regulator. Plant Cell 6 (11), 1607-1621.

Gish, W., States. D.J. (1993). Identification of protein coding regions by database similarity search. Nature Genetics 3 (3), 266-272.

Hicks, K.A., Sundas, A. and Meeks-Wagner, D.R. (1996). *Arabidopsis* early flowering mutants reveal multiple levels of regulation in the vegetative-to-floral transition. Seminars in Cell & Developmental Biology 7(N3), 409-418.

Kang, H.G., Jang, S., Chung, J.E., Cho, Y.G. and An, G. (1997). Characterization of 2 rice mads box genes that control flowering time. Molecules and Cells 7, 559-566. (see also WO 98/54328)

Koetsier, P.A., Schorr, J., Doerfler, W. (1993). A rapid optimized protocol for downward alkaline southern blotting of DNA. Biotechniques 15 (2), 260-262.

Koornneef, M., Hanhart, C.J., and van der Veen, Jh. (1991). Molecular & General Genetics, 229, 57-66.

Laurie, D.A. (1997). Comparative genetics of flowering time. Plant Molecular Biology 35, 167-177.

Levy and Dean (1998). Plant Cell 10, 1973 - 1989.

Logemann, J., Schell, J., Willmitzer, L. (1987). Improved method for the isolation of RNA from plant tissues. Analytical Biochemistry 163 (1), 16-20.

Macknight, R., Bancroft, I., Page, T., Lister, C., Schmidt, R., Love, K., Westphal, L., Murphy, G., Sherson, S., Cobbett, C. and Dean, C. (1997). Fca, a gene controlling flowering time in *Arabidopsis,* encodes a protein containing RNA binding domains. Cell 89, 737-745.

Mandel, M.A. and Yanofsky, M.F. (1995). A gene triggering flower formation in *Arabidopsis.* Nature 377, 522-524. (see also U.S. patent No. 5,811,536 issued on Sep. 22, 1998).

Menzel, G., Apel, K., Melzer, S. (1996). Identification of two MADS box genes that are expressed in the apical meristem of the long-day plant *Sinapis alba* in transition to flowering. Plant Journal 9 (3), 399-408.

Mozo, T., Fischer, S., Shizuya, H., Altmann, T. (1998). Construction and characterization of the IGF *Arabidopsis* BAC library. Mol. Gen. Genet. 258, 562-570.

Napp-Zinn, K. (1969) Cornell University Press, Ithaca N.Y., 0, 291-304.

Nilsson, O. and Weigel, D. (1997). Modulating the timing of flowering. Current Opinion in Biotechnology 8, 195-199.

Puchta, H. and Hohn, B. (1996). From centiMorgans to base pairs: homologous recombination in plants. Plant Sci. 1, 340-348.

Putterill, J., Robson, F., Lee, K., Simon, R. and Coupland, G. (1995). The *CONSTANS* gene of *Arabidopsis* promotes flowering and encodes a protein showing similarities to zinc finger transcription factors. Cell 80, 847-857.
Sambrook, J., Fritsch, E.F., Maniatis, T. (1989). Molecular cloning: a laboratory manual (2nd ed.), Cold Spring Harbor Laboratory, Cold Spring Harbor, NY
Schwarz-Sommer, Zs., Huijser, P., Nacken, W., Saedler, H. and Sommer, H. (1990). Genetic control of flower development by homeotic genes in *Antirrhinum majus*. Science (Washington D.C.) 250, 931-936.
Sheldon, C.C., Burn, J.E., Perez, P.P., Metzger, J., Edwards, J.A., Peacock, W.J., Dennis (1999). The FLF MADS box gene. A repressor of flowering in *Arabidopsis* regulated by vernalization and methylation. Plant Cell 1999, 11(3), 445-458.
Simon, R., Igeno, M.I. and Coupland, G. (1996). Activation of floral meristem identity genes in *arabidopsis.* Nature 384(N6604), 59-62.
Taylor, C.B. (1997). Comprehending cosuppression. Plant Cell 9, 1245-1249.
Weigel, D. and Nilsson, O. (1995). A developmental switch sufficient for flower initiation in diverse plants. Nature 377, 495-500. (see also U.S. patent No. 5,637,785 issued on June 10, 1997).
Wisman, E., Hartmann, U., Sagasser, M., Baumann, E., Palme, K., Hahlbrock, K., Saedler, H., Weisshaar, B. (1998). Knock-out mutants from an *En-1* mutagenized *Arabidopsis thaliana* population generated phenylpropanoid biosynthesis phenotypes. Proc. Natl. Acad. of Sciences of the U.S.A. 95(21), 12432-12437.

## Claims

1. A plant comprising a genetic modification resulting in an altered flowering time of said plant when compared to a corresponding wild-type plant or a corresponding non-wild-type plant without said genetic modification wherein said genetic modification results in an altered expression of

   - a *SVP* gene comprising a nucleotide sequence as set forth in any one of SEQ ID NOS. 5 or 6; or
   - a *SVP* gene comprising a nucleotide sequence exhibiting, within the coding regions thereof, at least 75% homology to the coding regions of the *SVP* gene having the nucleotide sequence as set forth in any one of SEQ ID NOS. 5 or 6; or
   - a DNA sequence encoding any one of the *SVP* gene products comprising an amino acid sequence as set forth in any one of SEQ ID. NOS. 21 and 22;
   - a DNA sequence encoding a SVP protein comprising an amino acid sequence exhibiting at least 85% homology to any one of the *SVP* gene products having an amino acid sequence as set forth in any one of SEQ ID. NOS. 21 and 22; or
   - an effective portion of any one of said *SVP* genes or DNA sequences;

   in the plant when compared to the wild-type plant or the non-wild-type plant without said genetic modification.

2. The genetically modified plant according to claim 1 exhibiting an early flowering time wherein said genetic modification results in a reduced, lacking or qualitatively altered expression of

   - said *SVP* gene comprising a nucleotide sequence as set forth in any one of SEQ ID NOS. 5 or 6, or
   - said *SVP* gene comprising a nucleotide sequence exhibiting, within the coding regions thereof, at least 75% homology to the coding regions of the *SVP* gene having the nucleotide sequence as set forth in any one of SEQ ID NOS. 5 or 6 in said plant; or
   - said DNA sequence encoding any one of the *SVP* gene products comprising an amino acid sequence as set forth in any one of SEQ ID. NOS. 21 and 22; or
   - said DNA sequence encoding a SVP protein comprising an amino acid sequence exhibiting at least 85% homology to any one of the *SVP* gene products having an amino acid sequence as set forth in any one of SEQ ID. NOS. 21 and 22; or
   - an effective portion of any one of said *SVP* genes or DNA sequences;

   wherein the qualitatively altered expression of any one of said *SVP* genes, DNA sequences or effective portions thereof results in a reduced or lacking activity of the gene product of said *SVP* gene, DNA sequence or effective portion thereof, respectively, in said plant.

3. The genetically modified plant of claim 2 exhibiting early flowering wherein said genetic modification consists in

   (1) a mutation of any one of said *SVP* genes, DNA sequences or effective portions thereof in said plant, wherein said mutation results in loss of function of said *SVP* gene, DNA sequence or effective portion thereof;
   (2) a mutation of any one of said *SVP* genes, DNA sequences or effective portions thereof in said plant, wherein said mutation results in a reduced activity of said *SVP* gene, DNA sequence or effective portion thereof;
   (3) an expression of an "antisense" nucleotide molecule with respect to any one of said *SVP* genes, DNA sequences or effective portions thereof; and/or
   (4) an expression of a "sense" nucleotide molecule with respect to any one of said *SVP* genes, DNA sequences or effective portions thereof, wherein said expression of said "sense" nucleotide molecule results in cosuppression of said *SVP* gene, DNA sequence or effective portion thereof.

4. The genetically modified plant according to claim 1 exhibiting a late flowering time wherein said genetic modification results in a novel, enhanced or qualitatively altered expression of

   - said *SVP* gene comprising a nucleotide sequence as set forth in any one of SEQ ID NOS. 5 or 6; or
   - said *SVP* gene comprising a nucleotide sequence exhibiting, within the coding regions thereof, at least 75% homology to the coding regions of the *SVP* gene having the nucleotide sequence as set forth in any one of SEQ ID NOS. 5 or 6 in said plant; or
   - a DNA sequence encoding any one of the *SVP* gene products comprising an amino acid sequence as set forth in any one of SEQ ID. NOS. 21 and 22; or

- a DNA sequence encoding a *SVP* protein comprising an amino acid sequence exhibiting at least 85% homology to any one of the *SVP* gene products comprising an amino acid sequence as set forth in any one of SEQ ID NOS. 21 and 22; or
- an effective portion of any one of said *SVP* genes or DNA sequences;

wherein the qualitatively altered expression of any one of said *SVP* genes, DNA sequences or effective portions thereof results in an enhanced activity of a gene product of said *SVP* gene, DNA sequence or effective portion thereof, respectively, in said plant.

**5.** The genetically modified plant of claim 4 exhibiting late flowering wherein said genetic modification consists in

- introducing at least one transgene into the plant, said transgene comprising the nucleotide sequence of any one of said *SVP* genes, DNA sequences or effective portions thereof; and/or
- introducing a mutation into any one of said *SVP* genes, DNA sequences or effective portions thereof, wherein said mutation results in an enhanced activity of a gene product of said *SVP* gene, DNA sequence or effective portion thereof, respectively, in said plant.

**6.** A genetically modified plant part, plant cell or seed obtainable from a plant according to any one of claims 1 to 5.

**7.** A method for altering the flowering time in a plant comprising the step of introducing at least one genetic modification into said plant said genetic modification resulting in an altered expression of a *SVP* gene within said plant, said *SVP* gene

(1) comprising a nucleotide sequence as set forth in any one of SEQ ID NOS. 5 or 6; or
(2) comprising a nucleotide sequence exhibiting, within the coding regions thereof, at least 75% homology to the coding regions of the *SVP* gene having the nucleotide sequence as set forth in any one of SEQ ID NOS. 5 or 6, or
(3) being transcribable into mRNA giving rise to a cDNA comprising a nucleotide sequence as set forth in any one of SEQ ID NOS. 1 and 3 or a cDNA comprising a nucleotide sequence exhibiting at least 75% homology to any one of said sequences of SEQ ID NOS. 1 and 3; or
(4) giving rise upon expression to a *SVP* gene product comprising an amino acid sequence as set forth in any one of SEQ ID NOS. 21 and 22; or
(5) giving rise upon expression to a *SVP* gene product comprising an amino acid sequence exhibiting at least 85% homology to any one of the *SVP* gene products having an amino acid sequence as set forth in any one of SEQ ID NOS. 21 and 22; or
(6) being an effective portion of any one of the *SVP* genes characterized in any one of (1) to (5).

**8.** A kit for use in the method for altering the flowering time in a plant as claimed in claim 7, said kit comprising

(a) a construct, preferably a vector, comprising a nucleotide sequence being transcribable into an "antisense" nucleotide molecule with respect to an endogenous *SVP* gene comprising a nucleotide sequence as set forth in any one of SEQ ID NOS. 5 or 6 or an endogenous *SVP* gene comprising a nucleotide sequence exhibiting, within the coding regions thereof, at least 75% homology to the coding regions of the *SVP* gene having the nucleotide sequence as set forth in any one of SEQ ID NOS. 5 or 6 or an effective portion thereof; and/or
(b) a construct, preferably a vector, comprising a nucleotide sequence being transcribable into a "sense" nucleotide molecule with respect to an endogenous *SVP* gene comprising a nucleotide sequence as set forth in any one of SEQ ID NOS. 5 or 6 or an endogenous *SVP* gene comprising a nucleotide sequence exhibiting, within the coding regions thereof, at least 75% homology to the coding regions of the *SVP* gene having the nucleotide sequence as set forth in any one of SEQ ID NOS. 5 or 6 or an effective portion thereof said "sense" nucleotide molecule being suitable to induce a cosuppression of the *SVP* gene; and/or
(c) a construct, preferably a vector, comprising, preferably operably linked to a plant-functional promoter, a transgene, said transgene being

(i) a *SVP* gene comprising a nucleotide sequence as set forth in any one of SEQ ID NOS. 5 or 6 or a *SVP* gene comprising a nucleotide sequence exhibiting, within the coding regions thereof, at least 75% homology to the coding regions of the *SVP* gene having the nucleotide sequence as set forth in any one of SEQ ID NOS. 5 or 6; and/or
(ii) a DNA molecule essentially consisting of or comprising a nucleotide sequence as set forth in any one of SEQ ID NO. 1 or SEQ ID NO. 3 or a nucleotide sequence exhibiting a homology of at least 75% to any one of said sequences, or a DNA molecule derived from said DNA molecule by nucleotide substitution, addition, insertion, deletion and/or modification, which hybrididizes with the latter; and/or
(iii) a DNA molecule encoding any one of the *SVP* gene products comprising an amino acid sequence as set forth in any one of SEQ ID NOS. 21 and 22; and/or
(iv) a DNA molecule encoding a SVP protein or polypeptide comprising an amino acid sequence exhibiting at least 85% homology to any one of the *SVP* gene products having an amino acid sequence as set forth in any one of SEQ ID NOS. 21 and 22; and/or
(v) an effective portion of any one of the *SVP* genes or DNA sequences defined in any one of (i) to (iv).

**9.** A DNA molecule essentially consisting of or comprising a nucleotide sequence as set forth in any one of SEQ ID NO. 1 or SEQ ID NO. 3 or a nucleotide sequence having a homology of at least 75% to any one of said sequences, or a sequence complementary thereto.

**10.** A DNA molecule derived from the DNA molecule as claimed in claim 9 by nucleotide substitution, addition, insertion, deletion and/or modification, which hybrididizes with the latter.

**11.** A DNA molecule encoding any one of the proteins having an amino acid sequence as set forth in any one of SEQ ID. NOS. 21 and 22.

**12.** An antisense DNA molecule having an antisense nucleotide sequence with respect to the nucleotide sequence of any one of the DNA molecules as claimed in any one of claims 9 to 11.

**13.** A fragment of a DNA molecule as claimed in any one of claims 9 to 12.

**14.** A DNA construct comprising a DNA molecule or fragment according to any one of claims 9 to 13.

**15.** The DNA construct according to claim 14, said DNA construct being a vector comprising at least one DNA molecule or fragment as claimed in any one of claims 9 to 13.

**16.** A microorganism transformed with a DNA molecule, fragment or construct as claimed in any one of claims 9 to 15.

**17.** A protein or polypeptide encoded by any one of the DNA molecules as claimed in any one of claims 9 or 10.

**18.** A polypeptide fragment of the protein or polypeptide as claimed in claim 17.

**19.** A protein or polypeptide, in particular fusion protein, comprising a protein, polypeptide or polypeptide fragment as claimed in any one of claims 17 or 18.

**20.** A monoclonal or polyclonal antibody which specifically recognizes and binds a protein, polypeptide or a polypeptide fragment as claimed in any one of claims 17 or 18.

Fig.1/Bl.1

# FIGURE 1

```
       10                 30                 50
        .        .        .        .        .        .
ATCCTCTCTAATTTCCTTGTTGATTCATCGACTAGATCTAATTCTTCTCACAAAAGACTG

       70                 90                 110
        .        .        .        .        .        .
AGTGTGTTCTTTCTTTCAAATCTTTCAAAAACTAGGGTTTTTACTGTCTTGAAATCATAT

       130                150                170
        .        .        .        .        .        .
TTATTCTTCTAAATTTAGCAAAAAGAACACGATTTACTTTCCATTTCAGTCGTCTTGTCA

       190                210                230
        .        .        .        .        .        .
CTCTCTCTCTCTTCTTTAAAGTCTCCCTTTTTAGCAAAAATTCTCTCTCTCACAAAATTT

       250                270                290
        .        .        .        .        .        .
ATTTCCTCTGGCTTCTTCTTCCTCCTCCTCCATCTCTTCTCTTTACTCTCTCTTTAATCA

       310                330                350
        .        .        .        .        .        .
TCTCTCATTCTTGAATCTTGATCCATCAAAATCAATCCCGTTCTCGAAAGATCCATTAAA

       370                390                410
        .        .        .        .        .        .
ATCAAAACCTAAGCTCTCTCTCTTGCTTCTAGGGTTTTTTTGTTCGTTGTGATGGCGAGA
                                                     M  A  R

       430                450                470
        .        .        .        .        .        .
GAAAAGATTCAGATCAGGAAGATCGACAACGCAACGGCGAGACAAGTGACGTTTTCGAAA
E  K  I  Q  I  R  K  I  D  N  A  T  A  R  Q  V  T  F  S  K

       490                510                530
        .        .        .        .        .        .
CGAAGAAGAGGGCTTTTCAAGAAAGCTGAAGAACTCTCCGTTCTCTGCGACGCCGATGTC
R  R  R  G  L  F  K  K  A  E  E  L  S  V  L  C  D  A  D  V

       550                570                590
        .        .        .        .        .        .
GCTCTCATCATCTTCTCTTCCACCGGAAAACTGTTCGAGTTCTGTAGCTCCAGCATGAAG
A  L  I  I  F  S  S  T  G  K  L  F  E  F  C  S  S  S  M  K

       610                630                650
        .        .        .        .        .        .
GAAGTCCTAGAGAGGCATAACTTGCAGTCAAAGAACTTGGAGAAGCTTGATCAGCCATCT
E  V  L  E  R  H  N  L  Q  S  K  N  L  E  K  L  D  Q  P  S
```

Fig.1/Bl.2

```
        670                 690                 710
          .         .         .         .         .         .
CTTGAGTTACAGCTGGTTGAGAACAGTGATCACGCCCGAATGAGTAAAGAAATTGCGGAC
L  E  L  Q  L  V  E  N  S  D  H  A  R  M  S  K  E  I  A  D

        730                 750                 770
          .         .         .         .         .         .
AAGAGCCACCGACTAAGGCAAATGAGAGGAGAGGAACTTCAAGGACTTGACATTGAAGAG
K  S  H  R  L  R  Q  M  R  G  E  E  L  Q  G  L  D  I  E  E

        790                 810                 830
          .         .         .         .         .         .
CTTCAGCAGCTAGAGAAGGCCCTTGAAACTGGTTTGACGCGTGTGATTGAAACAAAGAGT
L  Q  Q  L  E  K  A  L  E  T  G  L  T  R  V  I  E  T  K  S

        850                 870                 890
          .         .         .         .         .         .
GACAAGATTATGAGTGAGATCAGCGAACTTCAGAAAAAGGGAATGCAATTGATGGATGAG
D  K  I  M  S  E  I  S  E  L  Q  K  K  G  M  Q  L  M  D  E

        910                 930                 950
          .         .         .         .         .         .
AACAAGCGGTTGAGGCAGCAAGTATGTGTCTTACCCTCTCTGTTGATAACAAATCCCTTT
N  K  R  L  R  Q  Q  V  C  V  L  P  S  L  L  I  T  N  P  F

        970                 990                1010
          .         .         .         .         .         .
CTTTTGTCTACCATTAACGTACACACCCCTAAATTTAATCCCCAGTTGTCTACAACACAT
L  L  S  T  I  N  V  H  T  P  K  F  N  P  Q  L  S  T  T  H

       1030                1050                1070
          .         .         .         .         .         .
ATGTTTGATCATACTGTGAGATAAATGAATAAACCAAGTGATATAGCGCGATTTAAAAAT
M  F  D  H  T  V  R  *

       1090                1110                1130
          .         .         .         .         .         .
GTCTTTAAAACTAAAGGTAACCATGTAGCTAGTTAGTCTCTAGGGTCCTAGAGGTCTACG

       1150                1170                1190
          .         .         .         .         .         .
AGTGTGCATGCATGGATTTGGTGCGTTTTTTCTTTTTCATCTTCATTTTGTTTTTTGAAA

       1210                1230                1250
          .         .         .         .         .         .
CAGGGAACCATAAACGAATATATATCTAATTCTTGTTTGATATATAGTTTGGTCGAGGCT

       1270                1290                1310
          .         .         .         .         .         .
TCATGTCAAGATTTGCTCATTCGTAGTTAGTTGATCTCTAGAGAAATTCAAAACACATGG

       1330                1350                1370
          .         .         .         .         .         .
TGCCACTAAAAACACAAAATGCAAATACTTAGCTAGAGAACTTAATGATATGTTTTGTCT
```

Fig.1/Bl.3

```
      1390              1410              1430
        .        .        .        .        .        .
TGATTTTTGCAGGGAACGCAACTAACGGAAGAGAACGAGCGACTTGGCATGCAAATATGT

      1450              1470              1490
        .        .        .        .        .        .
AACAATGTGCATGCACACGGTGGTGCTGAATCGGAGAACGCTGCTGTGTACGAGGAAGGA

      1510              1530              1550
        .        .        .        .        .        .
CAGTCGTCGGAGTCTATTACTAACGCCGGAAACTCTACCGGAGCGCCTGTTGACTCCGAG

      1570              1590              1610
        .        .        .        .        .        .
AGCTCCGACACTTCCCTTAGGCTCGGCTTACCGTATGGTGGTTAGAGATGGAACAATTCA

      1630              1650              1670
        .        .        .        .        .        .
AAGAAGTTGATGGAGTGAGGAGAGTAATGTAAATCTTTTTAACTCGGTAGTAACAAGAGA

      1690              1710              1730
        .        .        .        .        .        .
CAATGTCTAAGTAGTGAATTCTCAAATGTTTGTGTAAGTTTCTGCCTATGGAAGAGGCTT

      1750              1770
        .        .        .
TCATTTTTATGATTAAAAAAAAAAAAAAAAA
```

Fig.2/Bl.1

## FIGURE 2

```
          10                  30                  50
           .         .         .         .         .         .
TCTTTTGATCCTCTCTAATTTCCTTGTTGATTCATCGACTAGATCTAATTCTTCTCACAA

          70                  90                 110
           .         .         .         .         .         .
AAGACTGAGTGTGTTCTTTCTTTCAAATCTTTCAAAAACTAGGGTTTTTACTGTCTTGAA

         130                 150                 170
           .         .         .         .         .         .
ATCATATTTATTCTTCTAAATTTAGCAAAAAGAACACGATTTACTTTCCATTTCAGTCGT

         190                 210                 230
           .         .         .         .         .         .
CTTGTCACTCTCTCTCTCTTCTTTAAAGTCTCCCTTTTTAGCAAAAATTCTCTCTCTCAC

         250                 270                 290
           .         .         .         .         .         .
AAAATTTATTTCCTCTGGCTTCTTCTTCCTCCTCCTCCATCTCTTCTCTTTACTCTCTCT

         310                 330                 350
           .         .         .         .         .         .
TTAATCATCTCTCATTCTTGAATCTTGATCCATCAAAATCAATCCCGTTCTCGAAAGATC

         370                 390                 410
           .         .         .         .         .         .
CATTAAAATCAAAACCTAAGCTCTCTCTCTTGCTTCTAGGGTTTTTTTGTTCGTTGTGAT
                                                           M

         430                 450                 470
           .         .         .         .         .         .
GGCGAGAGAAAAGATTCAGATCAGGAAGATCGACAACGCAACGGCGAGACAAGTGACGTT
 A  R  E  K  I  Q  I  R  K  I  D  N  A  T  A  R  Q  V  T  F

         490                 510                 530
           .         .         .         .         .         .
TTCGAAACGAAGAAGAGGGCTTTTCAAGAAAGCTGAAGAACTCTCCGTTCTCTGCGACGC
 S  K  R  R  R  G  L  F  K  K  A  E  E  L  S  V  L  C  D  A

         550                 570                 590
           .         .         .         .         .         .
CGATGTCGCTCTCATCATCTTCTCTTCCACCGGAAAACTGTTCGAGTTCTGTAGCTCCAG
 D  V  A  L  I  I  F  S  S  T  G  K  L  F  E  F  C  S  S  S

         610                 630                 650
           .         .         .         .         .         .
CATGAAGGAAGTCCTAGAGAGGCATAACTTGCAGTCAAAGAACTTGGAGAAGCTTGATCA
 M  K  E  V  L  E  R  H  N  L  Q  S  K  N  L  E  K  L  D  Q
```

Fig.2/Bl.2

```
       670                 690                 710
         .         .         .         .         .         .
GCCATCTCTTGAGTTACAGCTGGTTGAGAACAGTGATCACGCCCGAATGAGTAAAGAAAT
 P  S  L  E  L  Q  L  V  E  N  S  D  H  A  R  M  S  K  E  I

       730                 750                 770
         .         .         .         .         .         .
TGCGGACAAGAGCCACCGACTAAGGCAAATGAGAGGAGAGGAACTTCAAGGACTTGACAT
 A  D  K  S  H  R  L  R  Q  M  R  G  E  E  L  Q  G  L  D  I

       790                 810                 830
         .         .         .         .         .         .
TGAAGAGCTTCAGCAGCTAGAGAAGGCCCTTGAAACTGGTTTGACGCGTGTGATTGAAAC
 E  E  L  Q  Q  L  E  K  A  L  E  T  G  L  T  R  V  I  E  T

       850                 870                 890
         .         .         .         .         .         .
AAAGAGTGACAAGATTATGAGTGAGATCAGCGAACTTCAGAAAAAGGGAATGCAATTGAT
 K  S  D  K  I  M  S  E  I  S  E  L  Q  K  K  G  M  Q  L  M

       910                 930                 950
         .         .         .         .         .         .
GGATGAGAACAAGCGGTTGAGGCAGCAAGGAACGCAACTAACGGAAGAGAACGAGCGACT
 D  E  N  K  R  L  R  Q  Q  G  T  Q  L  T  E  E  N  E  R  L

       970                 990                1010
         .         .         .         .         .         .
TGGCATGCAAATATGTAACAATGTGCATGCACACGGTGGTGCTGAATCGGAGAACGCTGC
 G  M  Q  I  C  N  N  V  H  A  H  G  G  A  E  S  E  N  A  A

      1030                1050                1070
         .         .         .         .         .         .
TGTGTACGAGGAAGGACAGTCGTCGGAGTCTATTACTAACGCCGGAAACTCTACCGGAGC
 V  Y  E  E  G  Q  S  S  E  S  I  T  N  A  G  N  S  T  G  A

      1090                1110                1130
         .         .         .         .         .         .
GCCTGTTGACTCCGAGAGCTCCGACACTTCCCTTAGGCTCGGCTTACCGTATGGTGGTTA
 P  V  D  S  E  S  S  D  T  S  L  R  L  G  L  P  Y  G  G  *

      1150                1170                1190
         .         .         .         .         .         .
GAGATGGAACAATTCAAAGAAGTTGATGGAGTGAGGAGAGTAATGTAAATCTTTTTAACT

      1210                1230                1250
         .         .         .         .         .         .
CGGTAGTAACAAGAGACAATGTCTAAGTAGTGAATTCTCAAATGTTTGTGTAAGTTTCTG

      1270                1290                1310
         .         .         .         .         .         .
CCTATGGAAGAGGCTTTCATTTTTATGATTTTCACTATGTATGATCTCTCTTCACTGCAT

      1330                1350
         .         .         .         .
TTCTGGTTAGTAACGCTTGTCACCGATAAAAAAAAAAAAAAAAAAAA
```

## FIGURE 3:

```
  1  MAREKIQIRK IDNATARQVT FSKRRRGLFK KAEELSVLCD ADVALIIFSS
 51  TGKLFEFCSS SMKEVLERHN LQSKNLEKLD QPSLELQLVE NSDHARMSKE
101  IADKSHRLRQ MRGEELQGLD IEELQQLEKA LETGLTRVIE TKSDKIMSEI
151  SELQKKGMQL MDENKRLRQQ VCVLPSLLIT NPFLLSTINV HTPKFNPQLS
201  TTHMFDHTVR *
```

## FIGURE 4:

```
  1  MAREKIQIRK IDNATARQVT FSKRRRGLFK KAEELSVLCD ADVALIIFSS
 51  TGKLFEFCSS SMKEVLERHN LQSKNLEKLD QPSLELQLVE NSDHARMSKE
101  IADKSHRLRQ MRGEELQGLD IEELQQLEKA LETGLTRVIE TKSDKIMSEI
151  SELQKKGMQL MDENKRLRQQ GTQLTEENER LGMQICNNVH AHGGAESENA
201  AVYEEGQSSE SITNAGNSTG APVDSESSDT SLRLGLPYGG *
```

Fig.5/Bl.1

## FIGURE 5.

```
                 13770     13780     13790     13800
            ATGGCGAGAGAAAAGATTCAGATCAGGAAGATCGACAACGC
             M  A  R  E  K  I  Q  I  R  K  I  D  N  A>
            ______________PROTEIN SVP________________>

      13810     13820     13830     13840     13850     13860
AACGGCGAGACAAGTGACGTTTTCGAAACGAAGAAGAGGGCTTTTCAAGAAAGCTGAAGA
  T  A  R  Q  V  T  F  S  K  R  R  R  G  L  F  K  K  A  E  E>
________________________PROTEIN SVP________________________>

      13870     13880     13890     13900     13910     13920
ACTCTCCGTTCTCTGCGACGCCGATGTCGCTCTCATCATCTTCTCTTCCACCGGAAAACT
  L  S  V  L  C  D  A  D  V  A  L  I  I  F  S  S  T  G  K  L>
________________________PROTEIN SVP________________________>

      13930     13940     13950     13960     13970     13980
GTTCGAGTTCTGTAGCTCCAGGTCTTTCTTTCTCTCTAACTTCCCTCTCTATAGATTT
  F  E  F  C  S  S  S>
____PROTEIN SVP______>
                      _______________INTRON 1________________>

      13990     14000     14010     14020     14030     14040
CTCATAACTCATCGAAGGAATCTTGTCTAGATCCAGACAAAAAACTTTAAAGAGTTTTTA
_________________________INTRON 1__________________________>

      14050     14060     14070     14080     14090     14100
GATGTATATCTGATACATAGGAGTTTACTGTATCAATCTTTATAGGACCACTAACTATTT
_________________________INTRON 1__________________________>

      14110     14120     14130     14140     14150     14160
ATATAATTAAAATAGTTGTTAGAAACATTAATCATGACCATAAATGACATATATAAAGTG
_________________________INTRON 1__________________________>

      14170     14180     14190     14200     14210     14220
TATAGTAAAACTCTGTATTTAGATAAATTAAGGTATCTAACTACGGTAATATTCAAAAAG
_________________________INTRON 1__________________________>

      14230     14240     14250     14260     14270     14280
ATGTAAATCTGGATATGCATATATGTATATTATTAGTATATAAATACATGCTCTATAGTA
_________________________INTRON 1__________________________>

      14290     14300     14310     14320     14330     14340
GGTATTTGTGTCAACCATGTATAAATCTATGTATATAGATATTGTGGTATGATATGTTTA
_________________________INTRON 1__________________________>

      14350     14360     14370     14380     14390     14400
AGCCGTCAATGTCATATTTATATAGAAATATGTGGGTACCATAACATGAGGAAGTATCTA
_________________________INTRON 1__________________________>
```

Fig.5/Bl.2

```
    14410     14420     14430     14440     14450     14460
TATGTGTGGATGTATAAAGCTTTCCCTTTGAAGAAGTAATCTAAAAATAATATATATATA
_________________________INTRON 1__________________________>

    14470     14480     14490     14500     14510     14520
TATATGTATATGTATAGATATGTTGGAATCTTTATTAGTGTTGGGAAAAGTCATTTAGAG
_________________________INTRON 1__________________________>

    14530     14540     14550     14560     14570     14580
AGATATTATTGATATTAGGGATCTAAAATGACTTATCGTATTACAGAGATACGATTTTGG
_________________________INTRON 1__________________________>

    14590     14600     14610     14620     14630     14640
ATTTTTGACCCACTAGTTATCAGCTCAGTTCCTATCTTCGGGGACATACACACTTTCACA
_________________________INTRON 1__________________________>

    14650     14660     14670     14680     14690     14700
GATAATTGTGTATATATGTAACTGAAAACGATAGTGTTAACATGAAATAATGTACATGTT
_________________________INTRON 1__________________________>

    14710     14720     14730     14740     14750     14760
TGGGATTAAATGTGTTTTGTGGATTTGGTTTGCATCTTTTGATTTTAGATTTTGGTATAT
_________________________INTRON 1__________________________>

    14770     14780     14790     14800     14810     14820
TGTCGGTGTTTACATATGCACATTGTTAATATCAACAGTATAGTTGTTTATAATAAGTTA
_________________________INTRON 1__________________________>

    14830     14840     14850     14860     14870     14880
TTTATTGGAATGTGTTTATATTATGAAGCATGAAGGAAGTCCTAGAGAGGCATAACTTGC
                              M  K  E  V  L  E  R  H  N  L>
                             ______________________________>
_________INTRON 1__________>

    14890     14900     14910     14920     14930     14940
AGTCAAAGAACTTGGAGAAGCTTGATCAGCCATCTCTTGAGTTACAGGTTAGCTACATTC
Q  S  K  N  L  E  K  L  D  Q  P  S  L  E  L  Q>
_______________________________________________>
                                                ____________>

    14950     14960     14970     14980     14990     15000
TCGAAACGACCACACATTTTCTTTCCCGATTTCTGTAACTTGCAAAATCGAGTATTACTC
_________________________INTRON 2__________________________>

    15010     15020     15030     15040     15050     15060
CGTTGAATTACCAATATGTTTTAGATTGTTGTATTTATTGACCAAGAATCTCTTAAAACT
_________________________INTRON 2__________________________>

    15070     15080     15090     15100     15110     15120
TTGTATTAATAGGTACAAAACTTTATATTATTGCATATGATTAATTAGACTCGATCCATG
_________________________INTRON 2__________________________>
```

Fig.5/Bl.3

```
    15130     15140     15150     15160     15170     15180
TAGTAGTCATGTAGAGTAGTCCTGTGTAGAGAGTTGAGCTTTAGATCATTATGGATATGA
_________________________INTRON 2___________________________>

    15190     15200     15210     15220     15230     15240
TTAAGAGCTTAAATCAATGTTTTATTCTGTTAGCTGGTTGAGAACAGTGATCACGCCCGA
                                  L  V  E  N  S  D  H  A  R>
                                  ___________________________>
____________INTRON 2_____________>

    15250     15260     15270     15280     15290     15300
ATGAGTAAAGAAATTGCGGACAAGAGCCACCGACTAAGGTACGTTATATATGTATATTCT
 M  S  K  E  I  A  D  K  S  H  R  L  R>
______________________________________>
                                      ______INTRON 3________>

    15310     15320     15330     15340     15350     15360
ATGACTTTTGAACTAACTATCATTTTCTAACTAATTTTTTTTTGATCAACCACTATCAT
_________________________INTRON 3___________________________>

    15370     15380     15390     15400     15410     15420
TTTCTAACTGTGTGTTTACATGATCATATATAGGCAAATGAGAGGAGAGGAACTTCAAGG
                                   Q  M  R  G  E  E  L  Q  G>
                                  ___________________________>
____________INTRON 3_____________>

    15430     15440     15450     15460     15470     15480
ACTTGACATTGAAGAGCTTCAGCAGCTAGAGAAGGCCCTTGAAACTGGTTTGACGCGTGT
  L  D  I  E  E  L  Q  Q  L  E  K  A  L  E  T  G  L  T  R  V>
____________________________________________________________>

    15490     15500     15510     15520     15530     15540
GATTGAAACAAAGGTTGTTAAGAAAATTACTTGATACCATGTATAAGTTTCTCTAAGCTT
  I  E  T  K>
_____________>
             ___________________INTRON 4____________________>

    15550     15560     15570     15580     15590     15600
ACGAGTATGCAATTTACTAATACGAGATGTGTTTGCAGAGTGACAAGATTATGAGTGAGA
                                       S  D  K  I  M  S  E>
                                      ______________________>
______________INTRON 4_______________>

    15610     15620     15630     15640     15650     15660
TCAGCGAACTTCAGAAAAAGGTAATAATTAACCAAAATAACGTTTATTCTTTACTTGATG
I  S  E  L  Q  K  K>
____________________>
                    _______________INTRON 5_________________>

    15670     15680     15690     15700     15710     15720
ATTTCAATATTAATTTTGGCAGTTTCAAGATCCAAAATTTTCATCTTCTTCTCTTTTTTT
_________________________INTRON 5___________________________>
```

Fig.5/Bl.4

```
     15730     15740     15750     15760     15770     15780
TTGGTGTTCAGGGAATGCAATTGATGGATGAGAACAAGCGGTTGAGGCAGCAAGTATGTG
           G  M  Q  L  M  D  E  N  K  R  L  R  Q  Q  V  C>
           ________________________________________________>
__________>

     15790     15800     15810     15820     15830     15840
TCTTACCCTCTCTGTTGATAACAAATCCCTTTCTTTTGTCTACCATTAACGTACACACCC
V  L  P  S  L  L  I  T  N  P  F  L  L  S  T  I  N  V  H  T>
___________________________________________________________>

     15850     15860     15870     15880     15890
CTAAATTTAATCCCCAGTTGTCTACAACACATATGTTTGATCATACTGTGAGA
P  K  F  N  P  Q  L  S  T  T  H  M  F  D  H  T  V  R>
_____________________________________________________>
```

Fig.6/Bl.1

**FIGURE 6**

```
                    13770     13780     13790     13800
                 ATGGCGAGAGAAAAGATTCAGATCAGGAAGATCGACAACGC
                  M  A  R  E  K  I  Q  I  R  K  I  D  N  A>
                 ______________PROTEIN SVP________________>

     13810     13820     13830     13840     13850     13860
AACGGCGAGACAAGTGACGTTTTCGAAACGAAGAAGAGGGCTTTTCAAGAAAGCTGAAGA
  T  A  R  Q  V  T  F  S  K  R  R  R  G  L  F  K  K  A  E  E>
________________________PROTEIN SVP_________________________>

     13870     13880     13890     13900     13910     13920
ACTCTCCGTTCTCTGCGACGCCGATGTCGCTCTCATCATCTTCTCTTCCACCGGAAAACT
  L  S  V  L  C  D  A  D  V  A  L  I  I  F  S  S  T  G  K  L>
________________________PROTEIN SVP_________________________>

     13930     13940     13950     13960     13970     13980
GTTCGAGTTCTGTAGCTCCAGGTCTTTCTTTCTCTCTCTAACTTCCCTCTCTATAGATTT
  F  E  F  C  S  S  S>
____PROTEIN SVP______>
                     _______________INTRON 1_________________>

     13990     14000     14010     14020     14030     14040
CTCATAACTCATCGAAGGAATCTTGTCTAGATCCAGACAAAAAACTTTAAAGAGTTTTTA
_________________________INTRON 1___________________________>

     14050     14060     14070     14080     14090     14100
GATGTATATCTGATACATAGGAGTTTACTGTATCAATCTTTATAGGACCACTAACTATTT
_________________________INTRON 1___________________________>

     14110     14120     14130     14140     14150     14160
ATATAATTAAAATAGTTGTTAGAAACATTAATCATGACCATAAATGACATATATAAAGTG
_________________________INTRON 1___________________________>

     14170     14180     14190     14200     14210     14220
TATAGTAAAACTCTGTATTTAGATAAATTAAGGTATCTAACTACGGTAATATTCAAAAAG
_________________________INTRON 1___________________________>

     14230     14240     14250     14260     14270     14280
ATGTAAATCTGGATATGCATATATGTATATTATTAGTATATAAATACATGCTCTATAGTA
_________________________INTRON 1___________________________>

     14290     14300     14310     14320     14330     14340
GGTATTTGTGTCAACCATGTATAAATCTATGTATATAGATATTGTGGTATGATATGTTTA
_________________________INTRON 1___________________________>

     14350     14360     14370     14380     14390     14400
AGCCGTCAATGTCATATTTATATAGAAATATGTGGGTACCATAACATGAGGAAGTATCTA
_________________________INTRON 1___________________________>
```

Fig.6/Bl.2

```
     1441Q      14420      14430      14440      14450      14460
TATGTGTGGATGTATAAAGCTTTCCCTTTGAAGAAGTAATCTAAAAATAATATATATATA
_________________________INTRON 1___________________________>

     14470      14480      14490      14500      14510      14520
TATATGTATATGTATAGATATGTTGGAATCTTTATTAGTGTTGGGAAAAGTCATTTAGAG
_________________________INTRON 1___________________________>

     14530      14540      14550      14560      14570      14580
AGATATTATTGATATTAGGGATCTAAAATGACTTATCGTATTACAGAGATACGATTTTGG
_________________________INTRON 1___________________________>

     14590      14600      14610      14620      14630      14640
ATTTTTGACCCACTAGTTATCAGCTCAGTTCCTATCTTCGGGGACATACACACTTTCACA
_________________________INTRON 1___________________________>

     14650      14660      14670      14680      14690      14700
GATAATTGTGTATATATGTAACTGAAAACGATAGTGTTAACATGAAATAATGTACATGTT
_________________________INTRON 1___________________________>

     14710      14720      14730      14740      14750      14760
TGGGATTAAATGTGTTTTGTGGATTTGGTTTGCATCTTTTGATTTTAGATTTTGGTATAT
_________________________INTRON 1___________________________>

     14770      14780      14790      14800      14810      14820
TGTCGGTGTTTACATATGCACATTGTTAATATCAACAGTATAGTTGTTTATAATAAGTTA
_________________________INTRON 1___________________________>

     14830      14840      14850      14860      14870      14880
TTTATTGGAATGTGTTTATATTATGAAGCATGAAGGAAGTCCTAGAGAGGCATAACTTGC
                              M  K  E  V  L  E  R  H  N  L>
                             _______________________________>
_________INTRON 1___________>

     14890      14900      14910      14920      14930      14940
AGTCAAAGAACTTGGAGAAGCTTGATCAGCCATCTCTTGAGTTACAGGTTAGCTACATTC
Q  S  K  N  L  E  K  L  D  Q  P  S  L  E  L  Q>
________________________________________________>
                                                 ___________>

     14950      14960      14970      14980      14990      15000
TCGAAACGACCACACATTTTCTTTCCCGATTTCTGTAACTTGCAAAATCGAGTATTACTC
_________________________INTRON 2___________________________>

     15010      15020      15030      15040      15050      15060
CGTTGAATTACCAATATGTTTTAGATTGTTGTATTTATTGACCAAGAATCTCTTAAAACT
_________________________INTRON 2___________________________>

     15070      15080      15090      15100      15110      15120
TTGTATTAATAGGTACAAAACTTTATATTATTGCATATGATTAATTAGACTCGATCCATG
_________________________INTRON 2___________________________>
```

Fig.6/Bl.3

```
     15130      15140      15150      15160      15170      15180
TAGTAGTCATGTAGAGTAGTCCTGTGTAGAGAGTTGAGCTTTAGATCATTATGGATATGA
_________________________________INTRON 2____________________________>

     15190      15200      15210      15220      15230      15240
TTAAGAGCTTAAATCAATGTTTTATTCTGTTAGCTGGTTGAGAACAGTGATCACGCCCGA
                                  L  V  E  N  S  D  H  A  R>
                                  ____________________________>
________________INTRON 2_________________>

     15250      15260      15270      15280      15290      15300
ATGAGTAAAGAAATTGCGGACAAGAGCCACCGACTAAGGTACGTTATATATGTATATTCT
 M  S  K  E  I  A  D  K  S  H  R  L  R>
______________________________________>
                                      ______INTRON 3_________>

     15310      15320      15330      15340      15350      15360
ATGACTTTTGAACTAACTATCATTTTCTAACTAATTTTTTTTTGATCAACCACTATCAT
_________________________________INTRON 3____________________________>

     15370      15380      15390      15400      15410      15420
TTTCTAACTGTGTGTTTACATGATCATATATAGGCAAATGAGAGGAGAGGAACTTCAAGG
                                  Q  M  R  G  E  E  L  Q  G>
                                  ____________________________>
________________INTRON 3_________________>

     15430      15440      15450      15460      15470      15480
ACTTGACATTGAAGAGCTTCAGCAGCTAGAGAAGGCCCTTGAAACTGGTTTGACGCGTGT
  L  D  I  E  E  L  Q  Q  L  E  K  A  L  E  T  G  L  T  R  V>
_____________________________________________________________>

     15490      15500      15510      15520      15530      15540
GATTGAAACAAAGGTTGTTAAGAAAATTACTTGATACCATGTATAAGTTTCTCTAAGCTT
  I  E  T  K>
_____________>
             ____________________INTRON 4_____________________>

     15550      15560      15570      15580      15590      15600
ACGAGTATGCAATTTACTAATACGAGATGTGTTTGCAGAGTGACAAGATTATGAGTGAGA
                                       S  D  K  I  M  S  E>
                                       _______________________>
_______________INTRON 4_________________>

     15610      15620      15630      15640      15650      15660
TCAGCGAACTTCAGAAAAAGGTAATAATTAACCAAAATAACGTTTATTCTTTACTTGATG
I  S  E  L  Q  K  K>
_____________________>
                     _______________INTRON 5__________________>
```

Fig.6/Bl.4

```
     15670     15680     15690     15700     15710     15720
ATTTCAATATTAATTTTGGCAGTTTCAAGATCCAAAATTTTCATCTTCTTCTCTTTTTTT
_____________________________INTRON 5________________________________>

     15730     15740     15750     15760     15770     15780
TTGGTGTTCAGGGAATGCAATTGATGGATGAGAACAAGCGGTTGAGGCAGCAAGTATGTG
               G  M  Q  L  M  D  E  N  K  R  L  R  Q  Q>
             _________________________________________________>
_____________>
                                                      ________>

     15790     15800     15810     15820     15830     15840
TCTTACCCTCTCTGTTGATAACAAATCCCTTTCTTTGTCTACCATTAACGTACACACCC
_____________________INTRON 6 (ALTERNATIVE)_______________________>

     15850     15860     15870     15880     15890     15900
CTAAATTTAATCCCCAGTTGTCTACAACACATATGTTTGATCATACTGTGAGATAAATGA
_____________________INTRON 6 (ALTERNATIVE)_______________________>

     15910     15920     15930     15940     15950     15960
ATAAACCAAGTGATATAGCGCGATTTAAAAATGTCTTTAAAACTAAAGGTAACCATGTAG
_____________________INTRON 6 (ALTERNATIVE)_______________________>

     15970     15980     15990     16000     16010     16020
CTAGTTAGTCTCTAGGGTCCTAGAGGTCTACGAGTGTGCATGCATGGATTTGGTGCGTTT
_____________________INTRON 6 (ALTERNATIVE)_______________________>

     16030     16040     16050     16060     16070     16080
TTTCTTTTTCATCTTCATTTTGTTTTTTGAAACAGGGAACCATAAACGAATATATATCTA
_____________________INTRON 6 (ALTERNATIVE)_______________________>

     16090     16100     16110     16120     16130     16140
ATTCTTGTTTGATATATAGTTTGGTCGAGGCTTCATGTCAAGATTTGCTCATTCGTAGTT
_____________________INTRON 6 (ALTERNATIVE)_______________________>

     16150     16160     16170     16180     16190     16200
AGTTGATCTCTAGAGAAATTCAAAACACATGGTGCCACTAAAAACACAAAATGCAAATAC
_____________________INTRON 6 (ALTERNATIVE)_______________________>

     16210     16220     16230     16240     16250     16260
TTAGCTAGAGAACTTAATGATATGTTTTGTCTTGATTTTTGCAGGGAACGCAACTAACGG
                                                 G  T  Q  L  T>
                                                ________________>
___________INTRON 6 (ALTERNATIVE)_____________>

     16270     16280     16290     16300     16310     16320
AAGAGAACGAGCGACTTGGCATGCAAGTACGTGGATATATATATAGTCTTGATTATTTTG
E  E  N  E  R  L  G  M  Q>
______________________________>
                              _____________INTRON 7______________>
```

Fig.6/Bl.5

```
    16330     16340     16350     16360     16370     16380
AATTTTGTATGTTTGTTATCAGTTTAATGTTAAATTTGGAATATTTTTTTTGGTTACGT
_________________________INTRON 7__________________________>

    16390     16400     16410     16420     16430     16440
GTAGATATGTAACAATGTGCATGCACACGGTGGTGCTGAATCGGAGAACGCTGCTGTGTA
    I  C  N  N  V  H  A  H  G  G  A  E  S  E  N  A  A  V  Y>
    _______________________________________________________>
____>

    16450     16460     16470     16480     16490     16500
CGAGGAAGGACAGTCGTCGGAGTCTATTACTAACGCCGGAAACTCTACCGGAGCGCCTGT
  E  E  G  Q  S  S  E  S  I  T  N  A  G  N  S  T  G  A  P  V>
___________________________________________________________>

    16510     16520     16530     16540     16550     16560
TGACTCCGAGAGCTCCGACACTTCCCTTAGGCTCGGGTAACCAATTAATCACCCTTTCTT
  D  S  E  S  S  D  T  S  L  R  L  G>
___________________________________>
                                   _______INTRON 8_________>

    16570     16580     16590     16600     16610     16620
TTTACATTCTTTTGAGTTGATAGCTTTACATGATTTTTTTTTGTTTTTAAATTGAGACA
_________________________INTRON 8__________________________>

    16630     16640     16650     16660     16670     16680
AAGAATATAAAAATATATACTTTAAACGACCATATACATGTATGTTGTCCATATTTATTG
_________________________INTRON 8__________________________>

    16690     16700     16710     16720     16730     16740
GCATTGCAAGATATGGATGACACAGAATCGTAAATAAATTTGGTTTATTTGTTGTATATT
_________________________INTRON 8__________________________>

    16750     16760     16770     16780     16790     16800
TGTAATTCTAACAAATAACATGTTATTTTGTATATTTTACATGATATCATGCTATTTGTT
_________________________INTRON 8__________________________>

    16810     16820     16830     16840     16850     16860
AAAATTACCTGTCATAAAAAACACCACTTATTTATGGTTTGTTTTTGAAAATTCTGGCA
_________________________INTRON 8__________________________>

    16870     16880     16890     16900     16910     16920
CAAATCTAATCATCTACAAAATCACTCTAAAAAATAAGTTATTGATTTAATGTATGGTGT
_________________________INTRON 8__________________________>

    16930     16940
TGTGCAGCTTACCGTATGGTGGT
         L  P  Y  G  G>
       ________________>
_______>
```

Fig.7/Bl.1

# FIGURE 7 -

```
        9010      9020      9030      9040      9050      9060
CAATAAAGAGCATTAAGGAGTCAGTTTCTTCATATGACGACCCTCCGTGATTTCCATTCT

        9070      9080      9090      9100      9110      9120
CAGTCATGCCATGATCACTGACTATTATCTGTATATGTAACAAAAATAAGATTGACTCTT

        9130      9140      9150      9160      9170      9180
GACATATGAAAGTCAAATGGACCTTCAAATACGAAAAGTCACTGACCAGAAGAGTCTGTC

        9190      9200      9210      9220      9230      9240
CTTGATCATGGCTGCGATCCATCATGGCTCTTAAATGCATTGTTCTAACTATATCATCCA

        9250      9260      9270      9280      9290      9300
TTTCTTTAAGTTTTGCAGGCATCAAGGGGCTAGTATAGAGAAMCAAATAATAAGATGAAT

        9310      9320      9330      9340      9350      9360
AAGCCATGCAACTAGTAACCTTTCTACTCACAAATGAGTTATGAAACTAACAAAGACCTG

        9370      9380      9390      9400      9410      9420
TTACGGCCGCCAGTATGTCCAACGTGATCTAAACCAAGGTAATGAAGGATCTGATAAGGG

        9430      9440      9450      9460      9470      9480
GTAGATCTAGTAAGAATAAAGCACAGAAAAGCTCAATTGATATACAAGAGGAATTTTTGT

        9490      9500      9510      9520      9530      9540
TTAAGTCAGTATATATTTTAAGGCAGAGAACAATCCATTGGCGTTAAATGAACCAGACGC

        9550      9560      9570      9580      9590      9600
CAATACTGGGGAATAAAAGAAAAAGTAAAAACTAACCAAGAGATTCCAATCATCACTGTT

        9610      9620      9630      9640      9650      9660
TAGCTCATCGGGCAAGTGTCGAGAAACATTTCTGTCTACCTGTACTGTATCTTTGACCTG

        9670      9680      9690      9700      9710      9720
TCCAAAAAAACATTTTTTGTTCAGATGACCATGATGTCGATAAGAAAAGCAATCAACAAA

        9730      9740      9750      9760      9770      9780
ACCAAACATCACATTAACTCACAAAGAAACTGCTAACACCGTCATGTCTCATAAATAGCC

        9790      9800      9810      9820      9830      9840
CTGGAAATAACTTGAGCCACGTCTCATCACCGAGCATCACCATTTTCCAACCAATCCTGA

        9850      9860      9870      9880      9890      9900
AAAACTGACCTGGTAAATTTGGACCAATGTCACATAGCTTTTCTTTCAATGTTTGCAAGC

        9910      9920      9930      9940      9950      9960
ATGAAAAATTTCTCATTGTTGAAAACTAATAGTTACCAAGAATATTATCATCTAAGAGAG
```

Fig.7/Bl.2

```
     9970       9980       9990      10000      10010      10020
CTTGTGTGTTAAAATTAAAAGCAACATCCAAGAAACCACCAATTGCCCCAGAAACCATTG

    10030      10040      10050      10060      10070      10080
CCTGAAAAAAACAATGAATTCTATCAACTAAGCAAGCAAATAAATTCATTTAGACTGTGA

    10090      10100      10110      10120      10130      10140
TCATCCACTTAATCACAACTATACACACACCTTTAACCTCGGCATGGTAACAGTTGGAGG

    10150      10160      10170      10180      10190      10200
AGCTGCTTTAGCATGGTAACCAATAGCATCTCCATTAGCTAACAGTGACTGAGTATAAGG

    10210      10220      10230      10240      10250      10260
CATAGACTCTTTCAAAACCTTCTCTGGAGGTTTACCATCTTTCCCAAGAACGAACTCCGC

    10270      10280      10290      10300      10310      10320
AGGTAGCCCATCAATAACCTAAACTAGCATCGCTTTAACATCCTCAAAAACACTCTCTCA

    10330      10340      10350      10360      10370      10380
CAAAGATCAAAATCAAAATTTTAACAAAGTGCAACATACCATTAAAATCAGTCGATCATA

    10390      10400      10410      10420      10430      10440
TTTGGAAGAGATTCCAGATAACTCCTACAAATTCACCACTATCAAACTCAAAAGGGAACT

    10450      10460      10470      10480      10490      10500
TTACACATTTGTTTTTGGATTCACAAAAGAGACAAATACCTGGTACAGCAATCTCAGTTT

    10510      10520      10530      10540      10550      10560
CTCTGGATGATGAAGCTCCGATTCGTTCGAAATCAGAGAAGAATCACAGAATGGATCTCG

    10570      10580      10590      10600      10610      10620
ATAGCTCTCCGAGCCACTGCCATTTCAAATAAACAAATCAATAGAGCGTAGATTCTTCTT

    10630      10640      10650      10660      10670      10680
CTTCGACTTCAAATGTTATTGATTTAGTAGTACCTGACGCCGGAGAGAGTCGGCTTGACG

    10690      10700      10710      10720      10730      10740
GGGAAGAAACCGAATACGAAAATGGAGAGGCCAATAATCTGAAGTAATATTCCGGCCACC

    10750      10760      10770      10780      10790      10800
GTAAATATCGTCAGTCTCGTACAAGTCATCGCCGCCGTCGTCATTTTCAGGATCCGGCGA

    10810      10820      10830      10840      10850      10860
GAAACTGAACCAAAATAATACTTATTTTACTCGTAAGGAAAATTTGGGCCTAATAAAAGC

    10870      10880      10890      10900      10910      10920
CCAATAATAATAAAAAGCCCATTAGGGACTCCGCTTTATGATAACGGTGACTGTAGTTTC

    10930      10940      10950      10960      10970      10980
CTTGATGTGTCAGAGAGAGTGTGTAGTGTAGGGACTGTGTAGAAAGAAAGAAGCCTAAAA
```

Fig.7/p.3

```
     10990     11000     11010     11020     11030     11040
TGGCTAAAAGGTTAGGTGCAATGTTTCATTAGAGAGGCTTGGAACTGTTAAGGGAAAGGT

     11050     11060     11070     11080     11090     11100
CACGAGTCGTCTACTCATAAAAACTCTGACACTTTGACCAATCAAAACTCAAAGACCTCA

     11110     11120     11130     11140     11150     11160
CCAGTTGTGTCACGTGCGCCTCTAAACACTATTCAATTTCAAATATAAATGATTCATGCG

     11170     11180     11190     11200     11210     11220
GTTCCAAACGCCAATTGATGGATGTTCTACCAAATTTAATCTACTTTTACCAAACCATGA

     11230     11240     11250     11260     11270     11280
CAAATATGAATAAACATTACTTGATAATAATTTTGTGAGTGAACAAACTTTTTTTTTTTC

     11290     11300     11310     11320     11330     11340
GAAACCAAACCAAGCTGAAAAAAACTCAACGATTTTCTTTGTTTAAAATACGTTAGAAAG

     11350     11360     11370     11380     11390     11400
GAATATGTATTATGCCGAAATAAGTAATATCGATCAGGCCACCTCTCTTATAGTTATTCT

     11410     11420     11430     11440     11450     11460
CCTAGCAACTTTAACCACTAGAAGGTTTTGTTTTCTAGTGTTTTCTAATATACGTCATCA

     11470     11480     11490     11500     11510     11520
AAATTTTCAAAAAATACTACATTTTTGTTTTAAAAACTTCCATAATTCCATTACTCGTAG

     11530     11540     11550     11560     11570     11580
AACACAAACGCAAACCATATTAATATTTTGTTGTCAACAAAAAATTTCAAATTATAATTCA

     11590     11600     11610     11620     11630     11640
ACTATATTTGCTTGATTACCCAATTAGATAGAAAAGAGTTAAAGAAGAAAAGAAAAGAGT

     11650     11660     11670     11680     11690     11700
TTACAGTAAATTAACGCAAACCATAATTATATTTAACACCGTATTAATCACATCAACCAT

     11710     11720     11730     11740     11750     11760
ATGACTTTTTTACCGTTTGCAACTTCATAATTCATATAGTATCATAATAAATTCGCAATA

     11770     11780     11790     11800     11810     11820
ATACAACACAAGAGTTTCGTCGGAAGAGTAAATAATACTCAAATAGGGGGTGAGTGATAC

     11830     11840     11850     11860     11870     11880
GAGCCACATGTATTCTTGAAGGGTAGATTATTGCAAACTTGGAGTAATAAAGAGAAGAAG

     11890     11900     11910     11920     11930     11940
AATGGGTTTGTAGTAGTTGCGTGGAGTATCTTTATTTGGGTAAAACTTTAATTTAGAAAT

     11950     11960     11970     11980     11990     12000
AAAATTCTGTACGGACAATGGATCGTGTCCCAATCAGATTTCTTGTGGCTGCTTCGGGTC
```

Fig.7/p.4

```
    12010     12020     12030     12040     12050     12060
TGGTTTTGGGTCCCTTTGAAAAATTTTAGTGGTCGACACTTTTTATTTTACTCTGGCTCG

    12070     12080     12090     12100     12110     12120
TGCCTCGAGGGTCCCTCTATTCACTGTTTCTTCGTATGAAGGTATGCTTAAACATTATTT

    12130     12140     12150     12160     12170     12180
TATTTTTAAAAACCCTTTAATTTTATTTTCTTACCTTTAATCACGGTTTTGTAAATTGCT

    12190     12200     12210     12220     12230     12240
TTTTAGTCTATGGAATGATGATTGTGGCGATTGAAATCATATGTTTGGTTCTGTTGTTGA

    12250     12260     12270     12280     12290     12300
CGTTGGTGAAGTATATGTGATTTGTAATGTTGAGCTTATGTATTAAAATGTTAAATGATA

    12310     12320     12330     12340     12350     12360
AATAACCTCGTAAGAAAGTGATTTCATTTAAATTTTATTTTGAGTTACATATTCAATTGG

    12370     12380     12390     12400     12410     12420
TTTTATAAAAAAATACTTCAGTGATGATTGATACCCCCATTGTGTGTGTAATTGTTACTG

    12430     12440     12450     12460     12470     12480
GGATTGAACAAAATTTATTTGTGCATGACAAACTTTCCAAATTAGTGCATAGATTGTAAT

    12490     12500     12510     12520     12530     12540
TGTATAATGGACTACATGTATCTGAGTAGATATGGTTCATTAGGTTACAAACCTCTTTTT

    12550     12560     12570     12580     12590     12600
TTAAGGACACAATTTTTCGACAAGTTATATGCCACATGATTGACTACTAAATTTTCAAAA

    12610     12620     12630     12640     12650     12660
ATTATTGCACTAATGTCTTTGAAATTAACAAATTATTTTGTCATTTCCGAGTTGGATTCT

    12670     12680     12690     12700     12710     12720
TACAAACCAAGGCCGAACTCACAAACTTATTTCTTTCAGTAAAAACAAAACATTGTCCTC

    12730     12740     12750     12760     12770     12780
AGAAAAATTCTGAAATGTCATCTTCCCAAATGTTTTTACATAAATAAAAATAATATACAG

    12790     12800     12810     12820     12830     12840
TTGATATTATTTTGTTCTTTCTGAATTTTGTTATGAGGTACCATTACCATATAGTACGTA

    12850     12860     12870     12880     12890     12900
GATTTACAAAAATGAAAATACGTTGTAGCCCTTGATGTTCTTCAGGTCTTCTAGTTAGTT

    12910     12920     12930     12940     12950     12960
TTTGCAGTAAATACCAACCAATTAGTTACAAGGAGTATAAGTGAACAAAGTGAGACAACT

    12970     12980     12990     13000     13010     13020
CATTTTATGCTTCCCTATAAAAAGAAATTCCCCAYTGACCCAAACACACACTTCTCTTCT
```

Fig.7/p.5

```
    13030     13040     13050     13060     13070     13080
CTCTCTCATCTCATTGGAGACTTATAAATCCTATTACCTCACCATATCCAATAACCACCA

    13090     13100     13110     13120     13130     13140
CACACAGACCAATATCCTAAAAAAAAACTAAAACTAAAAATATAATATATTTCGTTTTCT

    13150     13160     13170     13180     13190     13200
TTCCAAAAATAATCATTTAAGAAACCCCATCATCTTGATAGTATTATAAAATTAATAAAC

    13210     13220     13230     13240     13250     13260
CTCTCCCTGAAAATATCTCATCCTTCACCAATCAAAACCTTCTCATGTCTTCTTCTCTCC

    13270     13280     13290     13300     13310     13320
TCGACCTTTGAGGTGGAAAATTAAATATATTCCCTTAGCTTTTTTCTCCTTTAGTTTTC

    13330     13340     13350     13360     13370     13380
TTCTTCTTCTTGAGTTTTTTTCTTTTGATCCTCTCTAATTTCCTTGTTGATTCATCGAC

    13390     13400     13410     13420     13430     13440
TAGATCTAATTCTTCTCACAAAAGACTGAGTGTGTTCTTTCTTTCAAATCTTTCAAAAAC

    13450     13460     13470     13480     13490     13500
TAGGGTTTTTACTGTCTTGAAATCATATTTATTCTTCTAAATTTAGCAAAAAGAACACGA

    13510     13520     13530     13540     13550     13560
TTTACTTTCCATTTCAGTCGTCTTGTCACTCTCTCTCTCTTCTTTAAAGTCTCCCTTTTT

    13570     13580     13590     13600     13610     13620
AGCAAAAAATTCTCTCTCTCACAAAATTTATTTCCTCTGGCTTCTTCTTCCTCCTCCTCCA

    13630     13640     13650     13660     13670     13680
TCTCTTCTCTTTACTCTCTCTTTAATCATCTCTCATTCTTGAATCTTGATCCATCAAAAT

    13690     13700     13710     13720     13730     13740
CAATCCCGTTCTCGAAAGATCCATTAAAATCAAAACCTAAGCTCTCTCTCTTGCTTCTAG

    13750     13760     13770     13780     13790     13800
GGTTTTTTTGTTCGTTGTGATGGCGAGAGAAAAGATTCAGATCAGGAAGATCGACAACGC
                      M  A  R  E  K  I  Q  I  R  K  I  D  N  A>
                     ______________PROTEIN SVP_______________>

    13810     13820     13830     13840     13850     13860
AACGGCGAGACAAGTGACGTTTTCGAAACGAAGAAGAGGGCTTTTCAAGAAAGCTGAAGA
  T  A  R  Q  V  T  F  S  K  R  R  R  G  L  F  K  K  A  E  E>
________________________PROTEIN SVP_________________________>

    13870     13880     13890     13900     13910     13920
ACTCTCCGTTCTCTGCGACGCCGATGTCGCTCTCATCATCTTCTCTTCCACCGGAAAACT
  L  S  V  L  C  D  A  D  V  A  L  I  I  F  S  S  T  G  K  L>
________________________PROTEIN SVP_________________________>
```

Fig.7/p.6

```
     13930     13940     13950     13960     13970     13980
GTTCGAGTTCTGTAGCTCCAGGTCTTTCTTTCTCTCTCTAACTTCCCTCTCTATAGATTT
 F  E  F  C  S  S  S>
_____PROTEIN SVP_______>
                   ___________________INTRON 1_________________>

     13990     14000     14010     14020     14030     14040
CTCATAACTCATCGAAGGAATCTTGTCTAGATCCAGACAAAAAACTTTAAAGAGTTTTTA
______________________________INTRON 1_________________________>

     14050     14060     14070     14080     14090     14100
GATGTATATCTGATACATAGGAGTTTACTGTATCAATCTTTATAGGACCACTAACTATTT
______________________________INTRON 1_________________________>

     14110     14120     14130     14140     14150     14160
ATATAATTAAAATAGTTGTTAGAAACATTAATCATGACCATAAATGACATATATAAAGTG
______________________________INTRON 1_________________________>

     14170     14180     14190     14200     14210     14220
TATAGTAAAACTCTGTATTTAGATAAATTAAGGTATCTAACTACGGTAATATTCAAAAAG
______________________________INTRON 1_________________________>

     14230     14240     14250     14260     14270     14280
ATGTAAATCTGGATATGCATATATGTATATTATTAGTATATAAATACATGCTCTATAGTA
______________________________INTRON 1_________________________>

     14290     14300     14310     14320     14330     14340
GGTATTTGTGTCAACCATGTATAAATCTATGTATATAGATATTGTGGTATGATATGTTTA
______________________________INTRON 1_________________________>

     14350     14360     14370     14380     14390     14400
AGCCGTCAATGTCATATTTATATAGAAATATGTGGGTACCATAACATGAGGAAGTATCTA
______________________________INTRON 1_________________________>

     14410     14420     14430     14440     14450     14460
TATGTGTGGATGTATAAAGCTTTCCCTTTGAAGAAGTAATCTAAAAATAATATATATATA
______________________________INTRON 1_________________________>

     14470     14480     14490     14500     14510     14520
TATATGTATATGTATAGATATGTTGGAATCTTTATTAGTGTTGGGAAAAGTCATTTAGAG
______________________________INTRON 1_________________________>

     14530     14540     14550     14560     14570     14580
AGATATTATTGATATTAGGGATCTAAAATGACTTATCGTATTACAGAGATACGATTTTGG
______________________________INTRON 1_________________________>

     14590     14600     14610     14620     14630     14640
ATTTTTGACCCACTAGTTATCAGCTCAGTTCCTATCTTCGGGGACATACACACTTTCACA
______________________________INTRON 1_________________________>
```

Fig.7/p.7

```
    14650     14660     14670     14680     14690     14700
GATAATTGTGTATATATGTAACTGAAAACGATAGTGTTAACATGAAATAATGTACATGTT
__________________________INTRON 1___________________________>

    14710     14720     14730     14740     14750     14760
TGGGATTAAATGTGTTTTGTGGATTTGGTTTGCATCTTTTGATTTTAGATTTTGGTATAT
__________________________INTRON 1___________________________>

    14770     14780     14790     14800     14810     14820
TGTCGGTGTTTACATATGCACATTGTTAATATCAACAGTATAGTTGTTTATAATAAGTTA
__________________________INTRON 1___________________________>

    14830     14840     14850     14860     14870     14880
TTTATTGGAATGTGTTTATATTATGAAGCATGAAGGAAGTCCTAGAGAGGCATAACTTGC
                               M  K  E  V  L  E  R  H  N  L>
                             _______________________________>
_________INTRON 1___________>

    14890     14900     14910     14920     14930     14940
AGTCAAAGAACTTGGAGAAGCTTGATCAGCCATCTCTTGAGTTACAGGTTAGCTACATTC
Q  S  K  N  L  E  K  L  D  Q  P  S  L  E  L  Q>
_______________________________________________>
                                                ____________>

    14950     14960     14970     14980     14990     15000
TCGAAACGACCACACATTTTCTTTCCCGATTTCTGTAACTTGCAAAATCGAGTATTACTC
__________________________INTRON 2___________________________>

    15010     15020     15030     15040     15050     15060
CGTTGAATTACCAATATGTTTTAGATTGTTGTATTTATTGACCAAGAATCTCTTAAAACT
__________________________INTRON 2___________________________>

    15070     15080     15090     15100     15110     15120
TTGTATTAATAGGTACAAAACTTTATATTATTGCATATGATTAATTAGACTCGATCCATG
__________________________INTRON 2___________________________>

    15130     15140     15150     15160     15170     15180
TAGTAGTCATGTAGAGTAGTCCTGTGTAGAGAGTTGAGCTTTAGATCATTATGGATATGA
__________________________INTRON 2___________________________>

    15190     15200     15210     15220     15230     15240
TTAAGAGCTTAAATCAATGTTTTATTCTGTTAGCTGGTTGAGAACAGTGATCACGCCCGA
                                  L  V  E  N  S  D  H  A  R>
                                  ___________________________>
____________INTRON 2_____________>

    15250     15260     15270     15280     15290     15300
ATGAGTAAAGAAATTGCGGACAAGAGCCACCGACTAAGGTACGTTATATATGTATATTCT
 M  S  K  E  I  A  D  K  S  H  R  L  R>
_____________________________________>
                                      _____INTRON 3________>
```

Fig.7/p.8

```
     15310      15320      15330      15340      15350      15360
ATGACTTTTGAACTAACTATCATTTTCTAACTAATTTTTTTTTGATCAACCACTATCAT
__________________________INTRON 3____________________________>

     15370      15380      15390      15400      15410      15420
TTTCTAACTGTGTGTTTACATGATCATATATAGGCAAATGAGAGGAGAGGAACTTCAAGG
                                     Q  M  R  G  E  E  L  Q  G>
                                     ____________________________>
____________INTRON 3_____________>

     15430      15440      15450      15460      15470      15480
ACTTGACATTGAAGAGCTTCAGCAGCTAGAGAAGGCCCTTGAAACTGGTTTGACGCGTGT
  L  D  I  E  E  L  Q  Q  L  E  K  A  L  E  T  G  L  T  R  V>
______________________________________________________________>

     15490      15500      15510      15520      15530      15540
GATTGAAACAAAGGTTGTTAAGAAAATTACTTGATACCATGTATAAGTTTCTCTAAGCTT
  I  E  T  K>
_____________>
             ____________________INTRON 4_____________________>

     15550      15560      15570      15580      15590      15600
ACGAGTATGCAATTTACTAATACGAGATGTGTTTGCAGAGTGACAAGATTATGAGTGAGA
                                          S  D  K  I  M  S  E>
                                          _____________________>
______________INTRON 4________________>

     15610      15620      15630      15640      15650      15660
TCAGCGAACTTCAGAAAAAGGTAATAATTAACCAAAATAACGTTTATTCTTTACTTGATG
I  S  E  L  Q  K  K>
_____________________>
                     _______________INTRON 5_________________>

     15670      15680      15690      15700      15710      15720
ATTTCAATATTAATTTTGGCAGTTTCAAGATCCAAAATTTTCATCTTCTTCTCTTTTTTT
__________________________INTRON 5____________________________>

     15730      15740      15750      15760      15770      15780
TTGGTGTTCAGGGAATGCAATTGATGGATGAGAACAAGCGGTTGAGGCAGCAAGTATGTG
            G  M  Q  L  M  D  E  N  K  R  L  R  Q  Q>
            ____________________________________________>
___________>
                                                        _______>

     15790      15800      15810      15820      15830      15840
TCTTACCCTCTCTGTTGATAACAAATCCCTTTCTTTTGTCTACCATTAACGTACACACCC
___________________INTRON 6 (ALTERNATIVE)____________________>

     15850      15860      15870      15880      15890      15900
CTAAATTTAATCCCCAGTTGTCTACAACACATATGTTTGATCATACTGTGAGATAAATGA
___________________INTRON 6 (ALTERNATIVE)____________________>
```

Fig.7/p.9

```
    15910     15920     15930     15940     15950     15960
ATAAACCAAGTGATATAGCGCGATTTAAAAATGTCTTTAAAACTAAAGGTAACCATGTAG
___________________INTRON 6 (ALTERNATIVE)_____________________>

    15970     15980     15990     16000     16010     16020
CTAGTTAGTCTCTAGGGTCCTAGAGGTCTACGAGTGTGCATGCATGGATTTGGTGCGTTT
___________________INTRON 6 (ALTERNATIVE)_____________________>

    16030     16040     16050     16060     16070     16080
TTTCTTTTTCATCTTCATTTTGTTTTTGAAACAGGGAACCATAAACGAATATATATCTA
___________________INTRON 6 (ALTERNATIVE)_____________________>

    16090     16100     16110     16120     16130     16140
ATTCTTGTTTGATATATAGTTTGGTCGAGGCTTCATGTCAAGATTTGCTCATTCGTAGTT
___________________INTRON 6 (ALTERNATIVE)_____________________>

    16150     16160     16170     16180     16190     16200
AGTTGATCTCTAGAGAAATTCAAAACACATGGTGCCACTAAAAACACAAAATGCAAATAC
___________________INTRON 6 (ALTERNATIVE)_____________________>

    16210     16220     16230     16240     16250     16260
TTAGCTAGAGAACTTAATGATATGTTTTGTCTTGATTTTTGCAGGGAACGCAACTAACGG
                                              G  T  Q  L  T>
                                             ________________>
__________INTRON 6 (ALTERNATIVE)____________>

    16270     16280     16290     16300     16310     16320
AAGAGAACGAGCGACTTGGCATGCAAGTACGTGGATATATATATAGTCTTGATTATTTTG
E  E  N  E  R  L  G  M  Q>
___________________________>
                           ____________INTRON 7______________>

    16330     16340     16350     16360     16370     16380
AATTTTGTATGTTTGTTATCAGTTTAATGTTAAATTTGGAATATTTTTTTTTGGTTACGT
__________________________INTRON 7____________________________>

    16390     16400     16410     16420     16430     16440
GTAGATATGTAACAATGTGCATGCACACGGTGGTGCTGAATCGGAGAACGCTGCTGTGTA
    I  C  N  N  V  H  A  H  G  G  A  E  S  E  N  A  A  V  Y>
    __________________________________________________________>
____>

    16450     16460     16470     16480     16490     16500
CGAGGAAGGACAGTCGTCGGAGTCTATTACTAACGCCGGAAACTCTACCGGAGCGCCTGT
  E  E  G  Q  S  S  E  S  I  T  N  A  G  N  S  T  G  A  P  V>
______________________________________________________________>

    16510     16520     16530     16540     16550     16560
TGACTCCGAGAGCTCCGACACTTCCCTTAGGCTCGGGTAACCAATTAATCACCCTTTCTT
  D  S  E  S  S  D  T  S  L  R  L  G>
_____________________________________>
                                     _______INTRON 8_________>
```

Fig.7/p.10

```
    16570     16580     16590     16600     16610     16620
TTTACATTCTTTTGAGTTGATAGCTTTACATGATTTTTTTTTGTTTTTAAATTGAGACA
_____________________________INTRON 8______________________________>

    16630     16640     16650     16660     16670     16680
AAGAATATAAAAATATATACTTTAAACGACCATATACATGTATGTTGTCCATATTTATTG
_____________________________INTRON 8______________________________>

    16690     16700     16710     16720     16730     16740
GCATTGCAAGATATGGATGACACAGAATCGTAAATAAATTTGGTTTATTTGTTGTATATT
_____________________________INTRON 8______________________________>

    16750     16760     16770     16780     16790     16800
TGTAATTCTAACAAATAACATGTTATTTTGTATATTTTACATGATATCATGCTATTTGTT
_____________________________INTRON 8______________________________>

    16810     16820     16830     16840     16850     16860
AAAATTACCTGTCATAAAAAACACCACTTATTTATGGTTTGTTTTTTGAAAATTCTGGCA
_____________________________INTRON 8______________________________>

    16870     16880     16890     16900     16910     16920
CAAATCTAATCATCTACAAAATCACTCTAAAAAATAAGTTATTGATTTAATGTATGGTGT
_____________________________INTRON 8______________________________>

    16930     16940     16950     16960     16970     16980
TGTGCAGCTTACCGTATGGTGGTTAGAGATGGAACAATTCAAAGAAGTTGATGGAGTGAG
          L  P  Y  G  G>
         __________________>
________>

    16990     17000     17010     17020     17030     17040
GAGAGTAATGTAAATCTTTTTAACTCGGTAGTAACAAGAGACAATGTCTAAGTAGTGAAT

    17050     17060     17070     17080     17090     17100
TCTCAAATGTTTGTGTAAGTTTCTGCCTATGGAAGAGGCTTTCATTTTTATGATTTTCAC

    17110     17120     17130     17140     17150     17160
TATGTATGATCTCTCTTCACTGCATTTCTGGTTAGTAACGCTTGTCACCGATAAACTTTC

    17170     17180     17190     17200     17210     17220
TCGTTATGGAAAGTTAGAATATAAAATATTGTAGAATTTTGATTATCGATATACTCTTGG

    17230     17240     17250     17260     17270     17280
TTAGGGATCAAAAATATATTTAGTGATGCCTCAAATAACTAATAATCATCATAGGACTC

    17290     17300     17310     17320     17330     17340
AGTCTCATTAGAAATTTTAGTGTAACAAAAGTGTGATTCGAATTCATCTTGTTGTTTTGT

    17350     17360     17370     17380     17390     17400
TGAATTTGTTGCAAAGTTTTACAAGTTTTCTCAGACCAGAGATTTCAAGTGTCTTCATTG
```

Fig.7/p.11

```
     17410     17420     17430     17440     17450     17460
GTGATGATTCTGGTGAATTGATGATCTTACTTGTGTGTGTGACACTTGCAATGTGTCTTG

     17470     17480     17490     17500     17510     17520
TTTGAGAAACACATTTGTAGTTTAGTTGGAGCTGTGCGGTTGGATGAGACTGAGTTCTAT

     17530     17540     17550     17560     17570     17580
GATAATAAATTAGAAGCAAAGTTTCCTTTCCATATTCATACGATTCATTATGTTAGCGAC

     17590     17600     17610     17620     17630     17640
AAGAAATATGAATATGGTTTGAAACTTTGCTTCTAATTCATCATTCGTTACACAACGTCC

     17650     17660     17670     17680     17690     17700
AAAATCTAAAAAAGTTTCAGATTTTCTCAAAATTCAGTAAATGGGCCTCTATATAATGAG

     17710     17720     17730     17740     17750     17760
GTTTGACGTTTTAGAGATACTTTGCACAAGAAAGAAGGTTCTGGTCGGATCGTTACACAC

     17770     17780     17790     17800     17810     17820
CGACCTTTGACCGGTTCATCAAAACGTGTCCGGTTTTATGTTTAAACCGTCACAAAGGGG

     17830     17840     17850     17860     17870     17880
AAAAAAGTTCTTTAAGATTCAATTACTTAATGATTAAATTCTTTGCTTGCTTCAACATCT

     17890     17900     17910     17920     17930     17940
TTACAACTTTTTTATTAAACAAAACGACATTTTCACATTCCTTTATCCACTAAAATTCTT

     17950     17960     17970     17980     17990     18000
TGTCAGATTAATCTATATATACATTTTTGCAGCTATTTTAGCAAATAAATCTTAGAGTTA

     18010     18020     18030     18040     18050     18060
GATTTATTTACAACTCAATGCCATTAACATTAAATTTTTTTCCAAAATAATTAATTTTA

     18070     18080     18090     18100     18110     18120
CATTAAATTTTTAATTTAAATTGTCAATCACATTATCAATCAAATTTAATCCAAACAAAC

     18130     18140     18150     18160     18170     18180
TTCACATCAATCCCTTAAAATTAGCATAAACTTCACATTAATTATTTAGACATGAAAATA

     18190     18200     18210     18220     18230     18240
TTTATTTAAAGATTTAAATCTTCTAAATTTTTGCTTTTAGAGTTATTATATCATTACCTA

     18250     18260     18270     18280     18290     18300
AAAGGCAAAAAATATTAATTTTTTAAATATTATTATTTATATATTTAAACTTCATAAAAC

     18310     18320     18330     18340     18350     18360
ATTATTAATATTTGAATTTATAAAAAGTTTAATCTATCAAAACTAATTAACATTATTATA

     18370     18380     18390     18400     18410     18420
TAATATATAAAATTAAAAAAATCTCAAAAAAAATTTCGTCATATTTTGTGATTCGAAATT
```

Fig.7/p.12

```
    18430     18440     18450     18460     18470     18480
TTAAGAATGAACATATATATTAACCAATTGGCGAAAAATGTGTGGGTTCAACGTCCCGCA

    18490     18500     18510     18520     18530     18540
ACGACTAAAATATTTTGACAATGATTCATAAACATATTATAAATAAGATCAATATTAATA

    18550     18560     18570     18580     18590     18600
AAATAAATAGTTTTTTTTTGTGGACGGGTTTGGAAGGACGGGTTTGGCAGGACGTTACTT

    18610     18620     18630     18640     18650     18660
AGTAACAATAGTAAACTATAAAATAATTTACAAAATTTTATATATATTAATTTTAAAAAA

    18670     18680     18690     18700     18710     18720
TGAATTGTCTCCGCGGTATACCGCGGGTTAAAATCTAGTTTAATTTAATAAAATCATACA

    18730     18740     18750     18760     18770     18780
TTTCTGACATGTTCTGTAACGTTTGATGATTCATAAGAATCATCGGAAGAGAATCCTTCC

    18790     18800     18810     18820     18830     18840
GACTTCTCCTACCACGACGCTGGCACTATAACATTCGTGTAAACACCATTCGTACATATA

    18850     18860     18870     18880     18890     18900
AATCAAATATTGAAAATTTTGTAACAATTTTTTTTTAATAGTCTTTAAAATATTTTTAC

    18910     18920     18930     18940     18950     18960
CAAAATACTATTAAGGTAGAGAGAGAGAAAGAAGAGCGAAGTTTGCTCCCACCCATGATC

    18970     18980     18990     19000     19010     19020
GCTCTTCTCTAAGAAAGAAAGAAAAAAAAAAGAGATTCAATTGTTGCACGTTCTTTCCTC

    19030     19040     19050     19060     19070     19080
AATTCTGATTTTGATTTACAACCAAAAAAGACCTTTAAGTTTCCTTGTGGGTCTCTCTAA

    19090     19100     19110     19120     19130     19140
AAACCGAAACTTTTCTCTTTCTTTTTCTTTTCTTATTAAGATCTAGACTCTACAGAGAGA

    19150     19160     19170     19180     19190     19200
GATTGTTGGTTCACACACATATCAAAGTTACAATCTTTCTGGGAATTTGAATGAATACGA

    19210     19220     19230     19240     19250     19260
TCAAAAAGAGTTGAAAAAACGAAAACTTTACGACGATGCTGCAACGAGCTGCGAGTAATG

    19270     19280     19290     19300     19310     19320
CGTATTCATGGTGGTGGGCGAGTCATATTCGAACCAAACAATCTAAATGGCTCGAACAAA

    19330     19340     19350     19360     19370     19380
ACCTTCAAGGTCTCTCCTTTTGAATCCATGTCTCTTTATATAGGTGTTTGTTTCTAAAAA

    19390     19400     19410     19420     19430     19440
GTTGAGATCTTTGAGGTTTGATCTATTTTTTGTTTTGGATATGAATTTGTAGATATTGAA
```

Fig.7/p.13

```
     19450       19460       19470       19480       19490       19500
GAGAAGGTTCAATATGTGTTGAAGCTATTACAAGAAGATGGAGATTCGTTTGCGAAGCGT

     19510       19520       19530       19540       19550       19560
GCAGAGATGTATTATAAGAAGAGACCAGAACTCATTAGCTTCGTTGAAGAATCTTACAGA

     19570       19580       19590       19600       19610
GCTTATAGAGCTTTAGCTGAACGTTATGATCATATTTCTACAGAGCTTCAAAATGCT
```

B

A

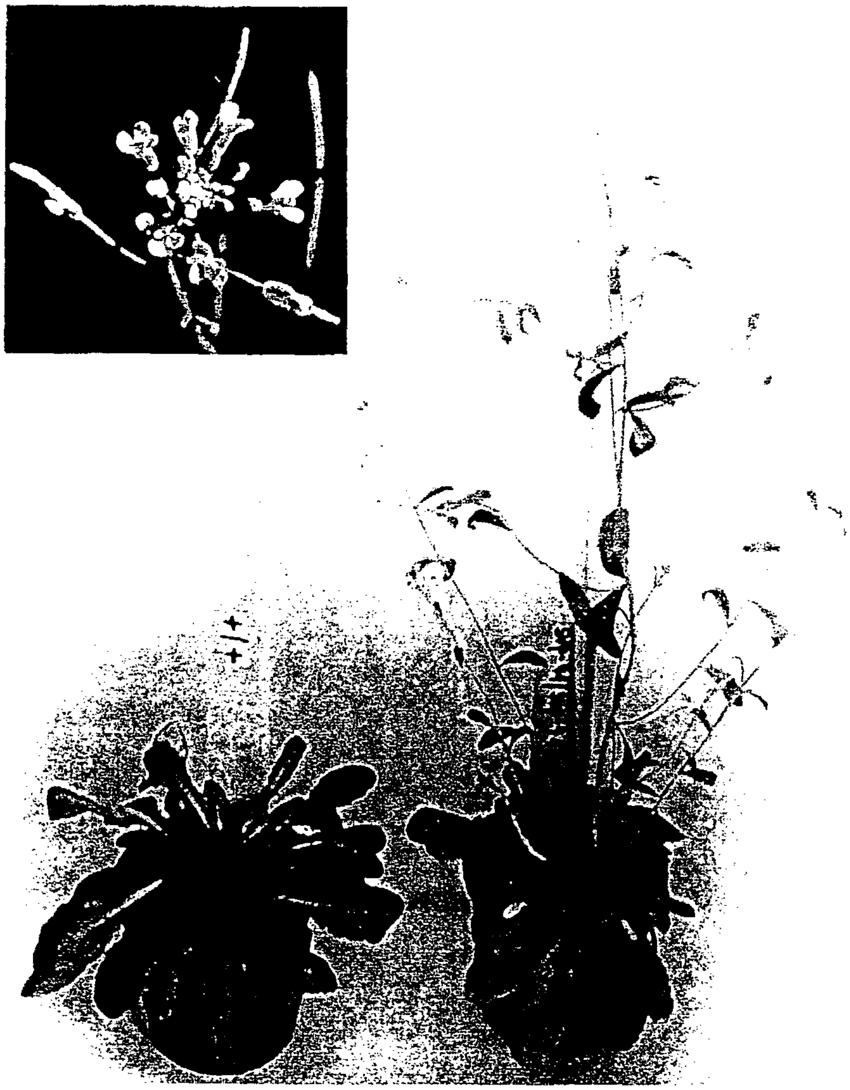

FIGURE 8

European Patent Office

# EUROPEAN SEARCH REPORT

Application Number
EP 99 10 9751

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | NEWMAN T. ET AL.: "EST AC R65130" EMBL DATABASE,4 June 1995 (1995-06-04), XP002119915 Heidelberg * the whole document * | 9,10, 13-16 | C12N15/29 C12N15/11 C12N15/62 C12N15/82 C12N5/10 C12N1/21 C07K14/415 C07K16/16 A01H5/00 A01H5/10 |
| X | NEWMAN T. ET AL.: "EST AA585750" EMBL DATABASE, 13 September 1997 (1997-09-13), XP002119916 Heidelberg * the whole document * | 9,10, 13-16 | |
| X | LIN X. ET AL.: "AC AC006592" EMBL DATABASE,11 March 1999 (1999-03-11), XP002119917 Heidelberg * the whole document * | 9-13, 16-19 | |
| A | MICHAELS S D ET AL: "FLOWERING LOCUS C encodes a novel MADS domain protein that acts as a repressor of flowering 'see comments!." PLANT CELL, (1999 MAY) 11 (5) 949-56. , XP002119918 * the whole document * | 1-20 | TECHNICAL FIELDS SEARCHED (Int.Cl.7) C12N C07K A01H |
| A,D | SHELDON C. ET AL.: "The FLF MADS box gene: a repressor of flowering in Arabidopsis regulated by vernalization and methylation" PLANT CELL, vol. 11, March 1999 (1999-03), pages 445-458, XP002119919 * the whole document * | 1-20 | |

-/--

The present search report has been drawn up for all claims

| Place of search | Date of completion of the search | Examiner |
|---|---|---|
| THE HAGUE | 22 October 1999 | Kania, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 99 10 9751

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WO 97 27287 A (UNIV CALIFORNIA) 31 July 1997 (1997-07-31) * the whole document * --- | 1-20 | |
| A | WO 99 04003 A (CT VOOR PLANTENVEREDELINGS EN ;BUSSCHER MARCO (NL); FRANKEN JOHN () 28 January 1999 (1999-01-28) * the whole document * --- | 1-20 | |
| A | WO 98 54328 A (UNIV WASHINGTON) 3 December 1998 (1998-12-03) * the whole document * --- | 1-20 | |
| A,D | MOZO T ET AL: "Construction and characterization of the IGF Arabidopsis BAC library." MOLECULAR AND GENERAL GENETICS, (1998 JUN) 258 (5) 562-70. , XP002119920 * the whole document * ----- | 1-20 | |

TECHNICAL FIELDS SEARCHED (Int.Cl.7)

The present search report has been drawn up for all claims

| Place of search | Date of completion of the search | Examiner |
|---|---|---|
| THE HAGUE | 22 October 1999 | Kania, T |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 99 10 9751

This annex lists the patent family members relating to the patent documents cited in the above-mentioned European search report.
The members are as contained in the European Patent Office EDP file on
The European Patent Office is in no way liable for these particulars which are merely given for the purpose of information.

22-10-1999

| Patent document cited in search report | Publication date | Patent family member(s) | Publication date |
|---|---|---|---|
| WO 9727287 A | 31-07-1997 | NONE | |
| WO 9904003 A | 28-01-1999 | AU 3465597 A | 10-02-1999 |
| WO 9854328 A | 03-12-1998 | AU 7718298 A | 30-12-1998 |
| | | CA 2224407 A | 02-12-1998 |

EPO FORM P0459

For more details about this annex : see Official Journal of the European Patent Office, No. 12/82

Annex to the application documents - subsequently filed sequences listing

```
                        SEQUENCE LISTING

<110> Max-Planck-Gesellschaft zur Förderung der Wissenschaften

<120> Transgenic plants exhibiting an altered flowering time

<130> 82253seq

<140>
<141>

<160> 22

<170> PatentIn Ver. 2.1

<210> 1
<211> 1771
<212> DNA
<213> Arabidopsis thaliana

<220>
<221> CDS
<222> (412)..(1041)

<400> 1
atcctctcta atttccttgt tgattcatcg actagatcta attcttctca caaaagactg 60

agtgtgttct ttctttcaaa tctttcaaaa actagggttt ttactgtctt gaaatcatat 120

ttattcttct aaatttagca aaaagaacac gatttacttt ccatttcagt cgtcttgtca 180

ctctctctct cttctttaaa gtctcccttt ttagcaaaaa ttctctctct cacaaaattt 240

atttcctctg gcttcttctt cctcctcctc catctcttct ctttactctc tctttaatca 300

tctctcattc ttgaatcttg atccatcaaa atcaatcccg ttctcgaaag atccattaaa 360

atcaaaacct aagctctctc tcttgcttct agggtttttt tgttcgttgt g atg gcg  417
                                                         Met Ala
                                                           1

aga gaa aag att cag atc agg aag atc gac aac gca acg gcg aga caa   465
Arg Glu Lys Ile Gln Ile Arg Lys Ile Asp Asn Ala Thr Ala Arg Gln
          5                  10                  15

gtg acg ttt tcg aaa cga aga aga ggg ctt ttc aag aaa gct gaa gaa   513
Val Thr Phe Ser Lys Arg Arg Arg Gly Leu Phe Lys Lys Ala Glu Glu
     20                  25                  30

ctc tcc gtt ctc tgc gac gcc gat gtc gct ctc atc atc ttc tct tcc   561
Leu Ser Val Leu Cys Asp Ala Asp Val Ala Leu Ile Ile Phe Ser Ser
 35                  40                  45                  50

acc gga aaa ctg ttc gag ttc tgt agc tcc agc atg aag gaa gtc cta   609
Thr Gly Lys Leu Phe Glu Phe Cys Ser Ser Ser Met Lys Glu Val Leu
                 55                  60                  65

gag agg cat aac ttg cag tca aag aac ttg gag aag ctt gat cag cca   657
Glu Arg His Asn Leu Gln Ser Lys Asn Leu Glu Lys Leu Asp Gln Pro
             70                  75                  80
```

```
tct ctt gag tta cag ctg gtt gag aac agt gat cac gcc cga atg agt    705
Ser Leu Glu Leu Gln Leu Val Glu Asn Ser Asp His Ala Arg Met Ser
        85                  90                  95

aaa gaa att gcg gac aag agc cac cga cta agg caa atg aga gga gag    753
Lys Glu Ile Ala Asp Lys Ser His Arg Leu Arg Gln Met Arg Gly Glu
    100                 105                 110

gaa ctt caa gga ctt gac att gaa gag ctt cag cag cta gag aag gcc    801
Glu Leu Gln Gly Leu Asp Ile Glu Glu Leu Gln Gln Leu Glu Lys Ala
115                 120                 125                 130

ctt gaa act ggt ttg acg cgt gtg att gaa aca aag agt gac aag att    849
Leu Glu Thr Gly Leu Thr Arg Val Ile Glu Thr Lys Ser Asp Lys Ile
                135                 140                 145

atg agt gag atc agc gaa ctt cag aaa aag gga atg caa ttg atg gat    897
Met Ser Glu Ile Ser Glu Leu Gln Lys Lys Gly Met Gln Leu Met Asp
            150                 155                 160

gag aac aag cgg ttg agg cag caa gta tgt gtc tta ccc tct ctg ttg    945
Glu Asn Lys Arg Leu Arg Gln Gln Val Cys Val Leu Pro Ser Leu Leu
        165                 170                 175

ata aca aat ccc ttt ctt ttg tct acc att aac gta cac acc cct aaa    993
Ile Thr Asn Pro Phe Leu Leu Ser Thr Ile Asn Val His Thr Pro Lys
    180                 185                 190

ttt aat ccc cag ttg tct aca aca cat atg ttt gat cat act gtg aga   1041
Phe Asn Pro Gln Leu Ser Thr Thr His Met Phe Asp His Thr Val Arg
195                 200                 205                 210

taaatgaata aaccaagtga tatagcgcga tttaaaaatg tctttaaaac taaaggtaac 1101

catgtagcta gttagtctct agggtcctag aggtctacga gtgtgcatgc atggatttgg 1161

tgcgtttttt ctttttcatc ttcattttgt tttttgaaac agggaaccat aaacgaatat 1221

atatctaatt cttgtttgat atatagtttg gtcgaggctt catgtcaaga tttgctcatt 1281

cgtagttagt tgatctctag agaaattcaa aacacatggt gccactaaaa acacaaaatg 1341

caaatactta gctagagaac ttaatgatat gttttgtctt gatttttgca gggaacgcaa 1401

ctaacggaag agaacgagcg acttggcatg caaatatgta acaatgtgca tgcacacggt 1461

ggtgctgaat cggagaacgc tgctgtgtac gaggaaggac agtcgtcgga gtctattact 1521

aacgccggaa actctaccgg agcgcctgtt gactccgaga gctccgacac ttcccttagg 1581

ctcggcttac cgtatggtgg ttagagatgg aacaattcaa agaagttgat ggagtgagga 1641

gagtaatgta aatcttttta actcggtagt aacaagagac aatgtctaag tagtgaattc 1701

tcaaatgttt gtgtaagttt ctgcctatgg aagaggcttt catttttatg attaaaaaaa 1761

aaaaaaaaaa                                                        1771
```

<210> 2
<211> 90
<212> PRT
<213> Arabidopsis thaliana

<400> 2
Ile Glu Glu Leu Gln Gln Leu Glu Lys Ala Leu Glu Thr Gly Leu Thr
1 5 10 15

Arg Val Ile Glu Thr Lys Ser Asp Lys Ile Met Ser Glu Ile Ser Glu
20 25 30

Leu Gln Lys Lys Gly Met Gln Leu Met Asp Glu Asn Lys Arg Leu Arg
35 40 45

Gln Gln Val Cys Val Leu Pro Ser Leu Leu Ile Thr Asn Pro Phe Leu
50 55 60

Leu Ser Thr Ile Asn Val His Thr Pro Lys Phe Asn Pro Gln Leu Ser
65 70 75 80

Thr Thr His Met Phe Asp His Thr Val Arg
85 90

<210> 3
<211> 1368
<212> DNA
<213> Arabidopsis thaliana

<220>
<221> CDS
<222> (419)..(1138)

<400> 3
tcttttgatc ctctctaatt tccttgttga ttcatcgact agatctaatt cttctcacaa 60

aagactgagt gtgttctttc tttcaaatct ttcaaaaact agggttttta ctgtcttgaa 120

atcatattta ttcttctaaa tttagcaaaa agaacacgat ttactttcca tttcagtcgt 180

cttgtcactc tctctctctt ctttaaagtc tccctttttta gcaaaaattc tctctctcac 240

aaaatttatt tcctctggct tcttcttcct cctcctccat ctcttctctt tactctctct 300

ttaatcatct ctcattcttg aatcttgatc catcaaaatc aatcccgttc tcgaaagatc 360

cattaaaatc aaaacctaag ctctctctct tgcttctagg gtttttttgt tcgttgtg 418

atg gcg aga gaa aag att cag atc agg aag atc gac aac gca acg gcg 466
Met Ala Arg Glu Lys Ile Gln Ile Arg Lys Ile Asp Asn Ala Thr Ala
1 5 10 15

aga caa gtg acg ttt tcg aaa cga aga aga ggg ctt ttc aag aaa gct 514
Arg Gln Val Thr Phe Ser Lys Arg Arg Arg Gly Leu Phe Lys Lys Ala
20 25 30

gaa gaa ctc tcc gtt ctc tgc gac gcc gat gtc gct ctc atc atc ttc 562
Glu Glu Leu Ser Val Leu Cys Asp Ala Asp Val Ala Leu Ile Ile Phe
35 40 45

tct tcc acc gga aaa ctg ttc gag ttc tgt agc tcc agc atg aag gaa 610
Ser Ser Thr Gly Lys Leu Phe Glu Phe Cys Ser Ser Ser Met Lys Glu
50 55 60

```
gtc cta gag agg cat aac ttg cag tca aag aac ttg gag aag ctt gat   658
Val Leu Glu Arg His Asn Leu Gln Ser Lys Asn Leu Glu Lys Leu Asp
 65                 70                  75                  80

cag cca tct ctt gag tta cag ctg gtt gag aac agt gat cac gcc cga   706
Gln Pro Ser Leu Glu Leu Gln Leu Val Glu Asn Ser Asp His Ala Arg
                85                  90                  95

atg agt aaa gaa att gcg gac aag agc cac cga cta agg caa atg aga   754
Met Ser Lys Glu Ile Ala Asp Lys Ser His Arg Leu Arg Gln Met Arg
            100                 105                 110

gga gag gaa ctt caa gga ctt gac att gaa gag ctt cag cag cta gag   802
Gly Glu Glu Leu Gln Gly Leu Asp Ile Glu Glu Leu Gln Gln Leu Glu
        115                 120                 125

aag gcc ctt gaa act ggt ttg acg cgt gtg att gaa aca aag agt gac   850
Lys Ala Leu Glu Thr Gly Leu Thr Arg Val Ile Glu Thr Lys Ser Asp
    130                 135                 140

aag att atg agt gag atc agc gaa ctt cag aaa aag gga atg caa ttg   898
Lys Ile Met Ser Glu Ile Ser Glu Leu Gln Lys Lys Gly Met Gln Leu
145                 150                 155                 160

atg gat gag aac aag cgg ttg agg cag caa gga acg caa cta acg gaa   946
Met Asp Glu Asn Lys Arg Leu Arg Gln Gln Gly Thr Gln Leu Thr Glu
                165                 170                 175

gag aac gag cga ctt ggc atg caa ata tgt aac aat gtg cat gca cac   994
Glu Asn Glu Arg Leu Gly Met Gln Ile Cys Asn Asn Val His Ala His
            180                 185                 190

ggt ggt gct gaa tcg gag aac gct gct gtg tac gag gaa gga cag tcg   1042
Gly Gly Ala Glu Ser Glu Asn Ala Ala Val Tyr Glu Glu Gly Gln Ser
        195                 200                 205

tcg gag tct att act aac gcc gga aac tct acc gga gcg cct gtt gac   1090
Ser Glu Ser Ile Thr Asn Ala Gly Asn Ser Thr Gly Ala Pro Val Asp
    210                 215                 220

tcc gag agc tcc gac act tcc ctt agg ctc ggc tta ccg tat ggt ggt   1138
Ser Glu Ser Ser Asp Thr Ser Leu Arg Leu Gly Leu Pro Tyr Gly Gly
225                 230                 235                 240

tagagatgga acaattcaaa gaagttgatg gagtgaggag agtaatgtaa atctttttaa 1198

ctcggtagta acaagagaca atgtctaagt agtgaattct caaatgtttg tgtaagtttc 1258

tgcctatgga agaggctttc atttttatga ttttcactat gtatgatctc tcttcactgc 1318

atttctggtt agtaacgctt gtcaccgata aaaaaaaaaa aaaaaaaaaa           1368
```

<210> 4
<211> 101
<212> PRT
<213> Arabidopsis thaliana

<400> 4

```
Glu Thr Lys Ser Asp Lys Ile Met Ser Glu Ile Ser Glu Leu Gln Lys
 1               5                  10                  15
```

```
Lys Gly Met Gln Leu Met Asp Glu Asn Lys Arg Leu Arg Gln Gln Gly
            20                  25                  30

Thr Gln Leu Thr Glu Glu Asn Glu Arg Leu Gly Met Gln Ile Cys Asn
        35                  40                  45

Asn Val His Ala His Gly Gly Ala Glu Ser Glu Asn Ala Ala Val Tyr
    50                  55                  60

Glu Glu Gly Gln Ser Ser Glu Ser Ile Thr Asn Ala Gly Asn Ser Thr
65                  70                  75                  80

Gly Ala Pro Val Asp Ser Glu Ser Ser Asp Thr Ser Leu Arg Leu Gly
                85                  90                  95

Leu Pro Tyr Gly Gly
            100
```

```
<210> 5
<211> 2134
<212> DNA
<213> Arabidopsis thaliana

<220>
<221> exon
<222> (1)..(183)

<220>
<221> intron
<222> (184)..(1090)

<220>
<221> exon
<222> (1091)..(1168)

<220>
<221> intron
<222> (1169)..(1454)

<220>
<221> exon
<222> (1455)..(1520)

<220>
<221> intron
<222> (1521)..(1635)

<220>
<221> exon
<222> (1636)..(1734)

<220>
<221> intron
<222> (1735)..(1819)
```

<220>
<221> exon
<222> (1820)..(1861)

<220>
<221> intron
<222> (1862)..(1972)

<220>
<221> exon
<222> (1973)..(2134)

<400> 5
atg gcg aga gaa aag att cag atc agg aag atc gac aac gca acg gcg 48

aga caa gtg acg ttt tcg aaa cga aga aga ggg ctt ttc aag aaa gct 96

gaa gaa ctc tcc gtt ctc tgc gac gcc gat gtc gct ctc atc atc ttc 144

tct tcc acc gga aaa ctg ttc gag ttc tgt agc tcc agg tctttctttc 193

tctctctaac ttccctctct atagatttct cataactcat cgaaggaatc ttgtctagat 253

ccagacaaaa aactttaaag agtttttaga tgtatatctg atacatagga gtttactgta 313

tcaatcttta taggaccact aactatttat ataattaaaa tagttgttag aaacattaat 373

catgaccata aatgacatat ataaagtgta tagtaaaact ctgtatttag ataaattaag 433

gtatctaact acggtaatat tcaaaaagat gtaaatctgg atatgcatat atgtatatta 493

ttagtatata aatacatgct ctatagtagg tatttgtgtc aaccatgtat aaatctatgt 553

atatagatat tgtggtatga tatgtttaag ccgtcaatgt catatttata tagaaatatg 613

tgggtaccat aacatgagga agtatctata tgtgtggatg tataaagctt tccctttgaa 673

gaagtaatct aaaaataata tatatatata tatgtatatg tatagatatg ttggaatctt 733

tattagtgtt gggaaaagtc atttagagag atattattga tattagggat ctaaaatgac 793

ttatcgtatt acagagatac gattttggat ttttgaccca ctagttatca gctcagttcc 853

tatcttcggg gacatacaca ctttcacaga taattgtgta tatatgtaac tgaaaacgat 913

agtgttaaca tgaaataatg tacatgtttg ggattaaatg tgttttgtgg atttggtttg 973

catcttttga ttttagattt tggtatattg tcggtgttta catatgcaca ttgttaatat 1033

caacagtata gttgtttata ataagttatt tattggaatg tgtttatatt atgaagc 1090

atg aag gaa gtc cta gag agg cat aac ttg cag tca aag aac ttg gag 1138

aag ctt gat cag cca tct ctt gag tta cag gttagctaca ttctcgaaac 1188

gaccacacat tttctttccc gatttctgta acttgcaaaa tcgagtatta ctccgttgaa 1248

ttaccaatat gttttagatt gttgtattta ttgaccaaga atctcttaaa actttgtatt 1308

aataggtaca aaactttata ttattgcata tgattaatta gactcgatcc atgtagtagt 1368

catgtagagt agtcctgtgt agagagttga gctttagatc attatggata tgattaagag 1428

cttaaatcaa tgttttattc tgttag ctg gtt gag aac agt gat cac gcc cga 1481

atg agt aaa gaa att gcg gac aag agc cac cga cta agg tacgttatat 1530

atgtatattc tatgactttt gaactaacta tcattttcta actaattttt tttttgatca 1590

accactatca ttttctaact gtgtgtttac atgatcatat atagg caa atg aga gga 1647

gag gaa ctt caa gga ctt gac att gaa gag ctt cag cag cta gag aag 1695

gcc ctt gaa act ggt ttg acg cgt gtg att gaa aca aag gttgttaaga 1744

aaattacttg ataccatgta taagtttctc taagcttacg agtatgcaat ttactaatac 1804

gagatgtgtt tgcag agt gac aag att atg agt gag atc agc gaa ctt cag 1855

aaa aag gtaataatta accaaaataa cgtttattct ttacttgatg atttcaatat 1911

taattttggc agtttcaaga tccaaaattt tcatcttctt ctcttttttt ttggtgttca 1971

g gga atg caa ttg atg gat gag aac aag cgg ttg agg cag caa gta tgt 2020

gtc tta ccc tct ctg ttg ata aca aat ccc ttt ctt ttg tct acc att 2068

aac gta cac acc cct aaa ttt aat ccc cag ttg tct aca aca cat atg 2116

ttt gat cat act gtg aga 2134

<210> 6
<211> 3184
<212> DNA
<213> Arabidopsis thaliana

<220>
<221> exon
<222> (1)..(183)

<220>
<221> intron
<222> (184)..(1090)

<220>
<221> exon
<222> (1091)..(1168)

<220>
<221> intron
<222> (1169)..(1454)

<220>
<221> exon
<222> (1455)..(1520)

<220>
<221> intron
<222> (1521)..(1635)

<220>
<221> exon
<222> (1636)..(1734)

<220>

<221> intron
<222> (1735)..(1819)

<220>
<221> exon
<222> (1820)..(1861)

<220>
<221> intron
<222> (1862)..(1972)

<220>
<221> exon
<222> (1973)..(2014)

<220>
<221> intron
<222> (2015)..(2485)

<220>
<221> exon
<222> (2486)..(2527)

<220>
<221> intron
<222> (2528)..(2625)

<220>
<221> exon
<222> (2626)..(2778)

<220>
<221> intron
<222> (2779)..(3169)

<220>
<221> exon
<222> (3170)..(3184)

<400> 6
atg gcg aga gaa aag att cag atc agg aag atc gac aac gca acg gcg 48

aga caa gtg acg ttt tcg aaa cga aga aga ggg ctt ttc aag aaa gct 96

gaa gaa ctc tcc gtt ctc tgc gac gcc gat gtc gct ctc atc atc ttc 144

tct tcc acc gga aaa ctg ttc gag ttc tgt agc tcc agg tctttctttc 193

tctctctaac ttccctctct atagatttct cataactcat cgaaggaatc ttgtctagat 253

ccagacaaaa aactttaaag agtttttaga tgtatatctg atacatagga gtttactgta 313

tcaatcttta taggaccact aactatttat ataattaaaa tagttgttag aaacattaat 373

catgaccata aatgacatat ataaagtgta tagtaaaact ctgtatttag ataaattaag 433

gtatctaact acggtaatat tcaaaaagat gtaaatctgg atatgcatat atgtatatta 493

ttagtatata aatacatgct ctatagtagg tatttgtgtc aaccatgtat aaatctatgt 553

atatagatat tgtggtatga tatgtttaag ccgtcaatgt catatttata tagaaatatg 613

tgggtaccat aacatgagga agtatctata tgtgtggatg tataaagctt tccctttgaa 673

gaagtaatct aaaaataata tatatatata tatgtatatg tatagatatg ttggaatctt 733
tattagtgtt gggaaaagtc atttagagag atattattga tattagggat ctaaaatgac 793
ttatcgtatt acagagatac gattttggat ttttgaccca ctagttatca gctcagttcc 853
tatcttcggg gacatacaca ctttcacaga taattgtgta tatatgtaac tgaaaacgat 913
agtgttaaca tgaaataatg tacatgtttg ggattaaatg tgttttgtgg atttggtttg 973
catcttttga ttttagattt tggtatattg tcggtgttta catatgcaca ttgttaatat 1033
caacagtata gttgtttata ataagttatt tattggaatg tgtttatatt atgaagc 1090
atg aag gaa gtc cta gag agg cat aac ttg cag tca aag aac ttg gag 1138
aag ctt gat cag cca tct ctt gag tta cag gttagctaca ttctcgaaac 1188
gaccacacat tttctttccc gatttctgta acttgcaaaa tcgagtatta ctccgttgaa 1248
ttaccaatat gttttagatt gttgtattta ttgaccaaga atctcttaaa actttgtatt 1308
aataggtaca aaactttata ttattgcata tgattaatta gactcgatcc atgtagtagt 1368
catgtagagt agtcctgtgt agagagttga gctttagatc attatggata tgattaagag 1428
cttaaatcaa tgttttattc tgttag ctg gtt gag aac agt gat cac gcc cga 1481
atg agt aaa gaa att gcg gac aag agc cac cga cta agg tacgttatat 1530
atgtatattc tatgactttt gaactaacta tcattttcta actaattttt tttttgatca 1590
accactatca ttttctaact gtgtgtttac atgatcatat atagg caa atg aga gga 1647
gag gaa ctt caa gga ctt gac att gaa gag ctt cag cag cta gag aag 1695
gcc ctt gaa act ggt ttg acg cgt gtg att gaa aca aag gttgttaaga 1744
aaattacttg ataccatgta taagtttctc taagcttacg agtatgcaat ttactaatac 1804
gagatgtgtt tgcag agt gac aag att atg agt gag atc agc gaa ctt cag 1855
aaa aag gtaataatta accaaaataa cgtttattct ttacttgatg atttcaatat 1911
taattttggc agtttcaaga tccaaaattt tcatcttctt ctcttttttt ttggtgttca 1971
g gga atg caa ttg atg gat gag aac aag cgg ttg agg cag caa 2014
gtatgtgtct taccctctct gttgataaca aatccctttc ttttgtctac cattaacgta 2074
cacaccccta aatttaatcc ccagttgtct acaacacata tgtttgatca tactgtgaga 2134
taaatgaata aaccaagtga tatagcgcga tttaaaaatg tctttaaaac taaaggtaac 2194
catgtagcta gttagtctct agggtcctag aggtctacga gtgtgcatgc atggatttgg 2254
tgcgtttttt ctttttcatc ttcattttgt tttttgaaac agggaaccat aaacgaatat 2314
atatctaatt cttgtttgat atatagtttg gtcgaggctt catgtcaaga tttgctcatt 2374
cgtagttagt tgatctctag agaaattcaa aacacatggt gccactaaaa acacaaaatg 2434

caaatactta gctagagaac ttaatgatat gttttgtctt gatttttgca g gga acg 2491

caa cta acg gaa gag aac gag cga ctt ggc atg caa gtacgtggat 2537

atatatatag tcttgattat tttgaatttt gtatgtttgt tatcagttta atgttaaatt 2597

tggaatattt ttttttggtt acgtgtag ata tgt aac aat gtg cat gca cac 2649

ggt ggt gct gaa tcg gag aac gct gct gtg tac gag gaa gga cag tcg 2697

tcg gag tct att act aac gcc gga aac tct acc gga gcg cct gtt gac 2745

tcc gag agc tcc gac act tcc ctt agg ctc ggg taaccaatta atcaccettt 2798

ctttttacat tcttttgagt tgatagcttt acatgatttt ttttttgttt ttaaattgag 2858

acaaagaata taaaaatata tactttaaac gaccatatac atgtatgttg tccatattta 2918

ttggcattgc aagatatgga tgacacagaa tcgtaaataa atttggttta tttgttgtat 2978

atttgtaatt ctaacaaata acatgttatt ttgtatattt tacatgatat catgctattt 3038

gttaaaatta cctgtcataa aaaacaccac ttatttatgg tttgtttttt gaaaattctg 3098

gcacaaatct aatcatctac aaaatcactc taaaaaataa gttattgatt taatgtatgg 3158

tgttgtgcag c tta ccg tat ggt ggt 3184

<210> 7
<211> 10617
<212> DNA
<213> Arabidopsis thaliana

<220>
<221> exon
<222> (4760)..(4942)

<220>
<221> intron
<222> (4943)..(5849)

<220>
<221> exon
<222> (5850)..(5927)

<220>
<221> intron
<222> (5928)..(6213)

<220>
<221> exon
<222> (6214)..(6279)

<220>
<221> intron
<222> (6280)..(6394)

<220>
<221> exon
<222> (6395)..(6493)

<220>

<221> intron
<222> (6494)..(6578)

<220>
<221> exon
<222> (6579)..(6620)

<220>
<221> intron
<222> (6621)..(6731)

<220>
<221> exon
<222> (6732)..(6773)

<220>
<221> intron
<222> (6774)..(7244)

<220>
<221> exon
<222> (7245)..(7286)

<220>
<221> intron
<222> (7287)..(7384)

<220>
<221> exon
<222> (7385)..(7537)

<220>
<221> intron
<222> (7538)..(7928)

<220>
<221> exon
<222> (7929)..(7943)

<400> 7
caataaagag cattaaggag tcagtttctt catatgacga ccctccgtga tttccattct 60

cagtcatgcc atgatcactg actattatct gtatatgtaa caaaaataag attgactctt 120

gacatatgaa agtcaaatgg accttcaaat acgaaaagtc actgaccaga agagtctgtc 180

cttgatcatg gctgcgatcc atcatggctc ttaaatgcat tgttctaact atatcatcca 240

tttctttaag ttttgcaggc atcaaggggc tagtatagag aamcaaataa taagatgaat 300

aagccatgca actagtaacc tttctactca caaatgagtt atgaaactaa caaagacctg 360

ttacggccgc cagtatgtcc aacgtgatct aaaccaaggt aatgaaggat ctgataaggg 420

gtagatctag taagaataaa gcacagaaaa gctcaattga tatacaagag gaatttttgt 480

ttaagtcagt atatatttta aggcagagaa caatccattg gcgttaaatg aaccagacgc 540

caatactggg gaataaaaga aaaagtaaaa actaaccaag agattccaat catcactgtt 600

tagctcatcg ggcaagtgtc gagaaacatt tctgtctacc tgtactgtat ctttgacctg 660

tccaaaaaaa catttttgt tcagatgacc atgatgtcga taagaaaagc aatcaacaaa 720

accaaacatc acattaactc acaaagaaac tgctaacacc gtcatgtctc ataaatagcc 780
ctggaaataa cttgagccac gtctcatcac cgagcatcac cattttccaa ccaatcctga 840
aaaactgacc tggtaaattt ggaccaatgt cacatagctt ttctttcaat gtttgcaagc 900
atgaaaaatt tctcattgtt gaaaactaat agttaccaag aatattatca tctaagagag 960
cttgtgtgtt aaaattaaaa gcaacatcca agaaaccacc aattgcccca gaaaccattg 1020
cctgaaaaaa acaatgaatt ctatcaacta agcaagcaaa taaattcatt tagactgtga 1080
tcatccactt aatcacaact atacacacac ctttaacctc ggcatggtaa cagttggagg 1140
agctgcttta gcatggtaac caatagcatc tccattagct aacagtgact gagtataagg 1200
catagactct ttcaaaacct tctctggagg tttaccatct ttcccaagaa cgaactccgc 1260
aggtagccca tcaataacct aaactagcat cgctttaaca tcctcaaaaa cactctctca 1320
caaagatcaa aatcaaaatt ttaacaaagt gcaacatacc attaaaatca gtcgatcata 1380
tttggaagag attccagata actcctacaa attcaccact atcaaactca aaagggaact 1440
ttacacattt gtttttggat tcacaaaaga gacaaatacc tggtacagca atctcagttt 1500
ctctggatga tgaagctccg attcgttcga aatcagagaa gaatcacaga atggatctcg 1560
atagctctcc gagccactgc catttcaaat aaacaaatca atagagcgta gattcttctt 1620
cttcgacttc aaatgttatt gatttagtag tacctgacgc cggagagagt cggcttgacg 1680
gggaagaaac cgaatacgaa aatggagagg ccaataatct gaagtaatat tccggccacc 1740
gtaaatatcg tcagtctcgt acaagtcatc gccgccgtcg tcattttcag gatccggcga 1800
gaaactgaac caaaataata cttattttac tcgtaaggaa aatttgggcc taataaaagc 1860
ccaataataa taaaaagccc attagggact ccgctttatg ataacggtga ctgtagtttc 1920
cttgatgtgt cagagagagt gtgtagtgta gggactgtgt agaaagaaag aagcctaaaa 1980
tggctaaaag gttaggtgca atgtttcatt agagaggctt ggaactgtta agggaaaggt 2040
cacgagtcgt ctactcataa aaactctgac actttgacca atcaaaactc aaagacctca 2100
ccagttgtgt cacgtgcgcc tctaaacact attcaatttc aaatataaat gattcatgcg 2160
gttccaaacg ccaattgatg gatgttctac caaatttaat ctacttttac caaaccatga 2220
caaatatgaa taaacattac ttgataataa ttttgtgagt gaacaaactt tttttttttc 2280
gaaaccaaac caagctgaaa aaaactcaac gattttcttt gtttaaaata cgttagaaag 2340
gaatatgtat tatgccgaaa taagtaatat cgatcaggcc acctctctta tagttattct 2400
cctagcaact ttaaccacta gaaggttttg ttttctagtg ttttctaata tacgtcatca 2460
aaattttcaa aaaatactac atttttgttt taaaaacttc cataattcca ttactcgtag 2520
aacacaaacg caaaccatat taatattttg ttgtcaacaa aaatttcaaa ttataattca 2580

actatatttg cttgattacc caattagata gaaaagagtt aaagaagaaa agaaaagagt 2640
ttacagtaaa ttaacgcaaa ccataattat atttaacacc gtattaatca catcaaccat 2700
atgacttttt taccgtttgc aacttcataa ttcatatagt atcataataa attcgcaata 2760
atacaacaca agagtttcgt cggaagagta aataatactc aaataggggg tgagtgatac 2820
gagccacatg tattcttgaa gggtagatta ttgcaaactt ggagtaataa agagaagaag 2880
aatgggtttg tagtagttgc gtggagtatc tttatttggg taaaacttta atttagaaat 2940
aaaattctgt acggacaatg gatcgtgtcc caatcagatt tcttgtggct gcttcgggtc 3000
tggttttggg tccctttgaa aaattttagt ggtcgacact ttttatttta ctctggctcg 3060
tgcctcgagg gtccctctat tcactgtttc ttcgtatgaa ggtatgctta aacattattt 3120
tatttttaaa aaccctttaa ttttattttc ttacctttaa tcacggtttt gtaaattgct 3180
ttttagtcta tggaatgatg attgtggcga ttgaaatcat atgtttggtt ctgttgttga 3240
cgttggtgaa gtatatgtga tttgtaatgt tgagcttatg tattaaaatg ttaaatgata 3300
aataacctcg taagaaagtg atttcattta aattttattt tgagttacat attcaattgg 3360
ttttataaaa aaatacttca gtgatgattg atacccccat tgtgtgtgta attgttactg 3420
ggattgaaca aaatttattt gtgcatgaca aactttccaa attagtgcat agattgtaat 3480
tgtataatgg actacatgta tctgagtaga tatggttcat taggttacaa acctcttttt 3540
ttaaggacac aatttttcga caagttatat gccacatgat tgactactaa attttcaaaa 3600
attattgcac taatgtcttt gaaattaaca aattattttg tcatttccga gttggattct 3660
tacaaaccaa ggccgaactc acaaacttat ttctttcagt aaaaacaaaa cattgtcctc 3720
agaaaaattc tgaaatgtca tcttcccaaa tgtttttaca taaataaaaa taatatacag 3780
ttgatattat tttgttcttt ctgaattttg ttatgaggta ccattaccat atagtacgta 3840
gatttacaaa aatgaaaata cgttgtagcc cttgatgttc ttcaggtctt ctagttagtt 3900
tttgcagtaa ataccaacca attagttaca aggagtataa gtgaacaaag tgagacaact 3960
cattttatgc ttccctataa aaagaaattc cccaytgacc caaacacaca cttctcttct 4020
ctctctcatc tcattggaga cttataaatc ctattacctc accatatcca ataaccacca 4080
cacacagacc aatatcctaa aaaaaaacta aaactaaaaa tataatatat ttcgttttct 4140
ttccaaaaat aatcatttaa gaaaccccat catcttgata gtattataaa attaataaac 4200
ctctccctga aaatatctca tccttcacca atcaaaacct tctcatgtct tcttctctcc 4260
tcgacctttg aggtggaaaa ttaaatatat tcccttagct ttttttctcc tttagttttc 4320
ttcttcttct tgagtttttt ttcttttgat cctctctaat ttccttgttg attcatcgac 4380
tagatctaat tcttctcaca aaagactgag tgtgttcttt ctttcaaatc tttcaaaaac 4440
tagggttttt actgtcttga aatcatattt attcttctaa atttagcaaa aagaacacga 4500

tttactttcc atttcagtcg tcttgtcact ctctctctct tctttaaagt ctccctttt 4560
agcaaaaatt ctctctctca caaaatttat ttcctctggc ttcttcttcc tcctcctcca 4620
tctcttctct ttactctctc tttaatcatc tctcattctt gaatcttgat ccatcaaaat 4680
caatcccgtt ctcgaaagat ccattaaaat caaaacctaa gctctctctc ttgcttctag 4740
ggttttttg ttcgttgtg atg gcg aga gaa aag att cag atc agg aag atc 4792
gac aac gca acg gcg aga caa gtg acg ttt tcg aaa cga aga aga ggg 4840
ctt ttc aag aaa gct gaa gaa ctc tcc gtt ctc tgc gac gcc gat gtc 4888
gct ctc atc atc ttc tct tcc acc gga aaa ctg ttc gag ttc tgt agc 4936
tcc agg tctttctttc tctctctaac ttccctctct atagatttct cataactcat 4992
cgaaggaatc ttgtctagat ccagacaaaa aactttaaag agtttttaga tgtatatctg 5052
atacatagga gtttactgta tcaatcttta taggaccact aactatttat ataattaaaa 5112
tagttgttag aaacattaat catgaccata aatgacatat ataaagtgta tagtaaaact 5172
ctgtatttag ataaattaag gtatctaact acggtaatat tcaaaaagat gtaaatctgg 5232
atatgcatat atgtatatta ttagtatata aatacatgct ctatagtagg tatttgtgtc 5292
aaccatgtat aaatctatgt atatagatat tgtggtatga tatgtttaag ccgtcaatgt 5352
catatttata tagaaatatg tgggtaccat aacatgagga agtatctata tgtgtggatg 5412
tataaagctt tccctttgaa gaagtaatct aaaaataata tatatatata tatgtatatg 5472
tatagatatg ttggaatctt tattagtgtt gggaaaagtc atttagagag atattattga 5532
tattagggat ctaaaatgac ttatcgtatt acagagatac gattttggat ttttgaccca 5592
ctagttatca gctcagttcc tatcttcggg gacatacaca ctttcacaga taattgtgta 5652
tatatgtaac tgaaaacgat agtgttaaca tgaaataatg tacatgtttg ggattaaatg 5712
tgttttgtgg atttggtttg catcttttga ttttagattt tggtatattg tcggtgttta 5772
catatgcaca ttgttaatat caacagtata gttgtttata ataagttatt tattggaatg 5832
tgtttatatt atgaagc atg aag gaa gtc cta gag agg cat aac ttg cag 5882
tca aag aac ttg gag aag ctt gat cag cca tct ctt gag tta cag 5927
gttagctaca ttctcgaaac gaccacacat tttctttccc gatttctgta acttgcaaaa 5987
tcgagtatta ctccgttgaa ttaccaatat gttttagatt gttgtattta ttgaccaaga 6047
atctcttaaa actttgtatt aataggtaca aaactttata ttattgcata tgattaatta 6107
gactcgatcc atgtagtagt catgtagagt agtcctgtgt agagagttga gctttagatc 6167
attatggata tgattaagag cttaaatcaa tgttttattc tgttag ctg gtt gag 6222
aac agt gat cac gcc cga atg agt aaa gaa att gcg gac aag agc cac 6270

cga cta agg tacgttatat atgtatattc tatgactttt gaactaacta 6319

tcattttcta actaattttt tttttgatca accactatca ttttctaact gtgtgtttac 6379

atgatcatat atagg caa atg aga gga gag gaa ctt caa gga ctt gac att 6430

gaa gag ctt cag cag cta gag aag gcc ctt gaa act ggt ttg acg cgt 6478

gtg att gaa aca aag gttgttaaga aaattacttg ataccatgta taagtttctc 6533

taagcttacg agtatgcaat ttactaatac gagatgtgtt tgcag agt gac aag att 6590

atg agt gag atc agc gaa ctt cag aaa aag gtaataatta accaaaataa 6640

cgtttattct ttacttgatg atttcaatat taattttggc agtttcaaga tccaaaattt 6700

tcatcttctt ctcttttttt ttggtgttca g gga atg caa ttg atg gat gag 6752

aac aag cgg ttg agg cag caa gtatgtgtct taccctctct gttgataaca 6803

aatccctttc ttttgtctac cattaacgta cacaccccta aatttaatcc ccagttgtct 6863

acaacacata tgtttgatca tactgtgaga taaatgaata aaccaagtga tatagcgcga 6923

tttaaaaatg tctttaaaac taaaggtaac catgtagcta gttagtctct agggtcctag 6983

aggtctacga gtgtgcatgc atggatttgg tgcgtttttt ctttttcatc ttcattttgt 7043

tttttgaaac agggaaccat aaacgaatat atatctaatt cttgtttgat atatagtttg 7103

gtcgaggctt catgtcaaga tttgctcatt cgtagttagt tgatctctag agaaattcaa 7163

aacacatggt gccactaaaa acacaaaatg caaatactta gctagagaac ttaatgatat 7223

gttttgtctt gatttttgca g gga acg caa cta acg gaa gag aac gag cga 7274

ctt ggc atg caa gtacgtggat atatatatag tcttgattat tttgaatttt 7326

gtatgtttgt tatcagttta atgttaaatt tggaatattt ttttttggtt acgtgtag 7384

ata tgt aac aat gtg cat gca cac ggt ggt gct gaa tcg gag aac gct 7432

gct gtg tac gag gaa gga cag tcg tcg gag tct att act aac gcc gga 7480

aac tct acc gga gcg cct gtt gac tcc gag agc tcc gac act tcc ctt 7528

agg ctc ggg taaccaatta atcacccttt ctttttacat tcttttgagt 7577

tgatagcttt acatgatttt ttttttgttt ttaaattgag acaaagaata taaaaatata 7637

tactttaaac gaccatatac atgtatgttg tccatattta ttggcattgc aagatatgga 7697

tgacacagaa tcgtaaataa atttggttta tttgttgtat atttgtaatt ctaacaaata 7757

acatgttatt ttgtatattt tacatgatat catgctattt gttaaaatta cctgtcataa 7817

aaaacaccac ttatttatgg tttgtttttt gaaaattctg gcacaaatct aatcatctac 7877

aaaatcactc taaaaaataa gttattgatt taatgtatgg tgttgtgcag c tta ccg 7934

tat ggt ggt tagagatgga acaattcaaa gaagttgatg gagtgaggag 7983

agtaatgtaa atctttttaa ctcggtagta acaagagaca atgtctaagt agtgaattct 8043

caaatgtttg tgtaagtttc tgcctatgga agaggctttc atttttatga ttttcactat 8103
gtatgatctc tcttcactgc atttctggtt agtaacgctt gtcaccgata aactttctcg 8163
ttatggaaag ttagaatata aaatattgta gaattttgat tatcgatata ctcttggtta 8223
gggatcaaaa atatatttta gtgatgcctc aaataactaa taatcatcat aggactcagt 8283
ctcattagaa attttagtgt aacaaaagtg tgattcgaat tcatcttgtt gttttgttga 8343
atttgttgca aagttttaca agtttctca gaccagagat ttcaagtgtc ttcattggtg 8403
atgattctgg tgaattgatg atcttacttg tgtgtgtgac acttgcaatg tgtcttgttt 8463
gagaaacaca tttgtagttt agttggagct gtgcggttgg atgagactga gttctatgat 8523
aataaattag aagcaaagtt tcctttccat attcatacga ttcattatgt tagcgacaag 8583
aaatatgaat atggtttgaa actttgcttc taattcatca ttcgttacac aacgtccaaa 8643
atctaaaaaa gtttcagatt ttctcaaaat tcagtaaatg ggcctctata taatgaggtt 8703
tgacgtttta gagatacttt gcacaagaaa gaaggttctg gtcggatcgt tacacaccga 8763
cctttgaccg gttcatcaaa acgtgtccgg ttttatgttt aaaccgtcac aaaggggaaa 8823
aaagttcttt aagattcaat tacttaatga ttaaattctt tgcttgcttc aacatcttta 8883
caactttttt attaaacaaa acgacatttt cacattcctt tatccactaa aattctttgt 8943
cagattaatc tatatataca tttttgcagc tattttagca aataaatctt agagttagat 9003
ttatttacaa ctcaatgcca ttaacattaa attttttttc caaaataatt aattttacat 9063
taaattttta atttaaattg tcaatcacat tatcaatcaa atttaatcca aacaaacttc 9123
acatcaatcc cttaaaatta gcataaactt cacattaatt atttagacat gaaaatattt 9183
atttaaagat ttaaatcttc taaattttttg cttttagagt tattatatca ttacctaaaa 9243
ggcaaaaaat attaattttt taaatattat tatttatata tttaaacttc ataaaacatt 9303
attaatattt gaatttataa aaagtttaat ctatcaaaac taattaacat tattatataa 9363
tatataaaat taaaaaaatc tcaaaaaaaa tttcgtcata ttttgtgatt cgaaatttta 9423
agaatgaaca tatatattaa ccaattggcg aaaaatgtgt gggttcaacg tcccgcaacg 9483
actaaaatat tttgacaatg attcataaac atattataaa taagatcaat attaataaaa 9543
taaatagttt ttttttgtgg acgggtttgg aaggacgggt ttggcaggac gttacttagt 9603
aacaatagta aactataaaa taatttacaa aattttatat atattaattt taaaaaatga 9663
attgtctccg cggtataccg cgggttaaaa tctagtttaa tttaataaaa tcatacattt 9723
ctgacatgtt ctgtaacgtt tgatgattca taagaatcat cggaagagaa tccttccgac 9783
ttctcctacc acgacgctgg cactataaca ttcgtgtaaa caccattcgt acatataaat 9843
caaatattga aaattttgta acaatttttt ttttaatagt ctttaaaata tttttaccaa 9903

aatactatta aggtagagag agagaaagaa gagcgaagtt tgctcccacc catgatcgct 9963

cttctctaag aaagaaagaa aaaaaaaaga gattcaattg ttgcacgttc tttcctcaat 10023

tctgattttg atttacaacc aaaaaagacc tttaagtttc cttgtgggtc tctctaaaaa 10083

ccgaaacttt tctctttctt tttcttttct tattaagatc tagactctac agagagagat 10143

tgttggttca cacacatatc aaagttacaa tctttctggg aatttgaatg aatacgatca 10203

aaaagagttg aaaaaacgaa aactttacga cgatgctgca acgagctgcg agtaatgcgt 10263

attcatggtg gtgggcgagt catattcgaa ccaaacaatc taaatggctc gaacaaaacc 10323

ttcaaggtct ctccttttga atccatgtct ctttatatag gtgtttgttt ctaaaaagtt 10383

gagatctttg aggtttgatc tattttttgt tttggatatg aatttgtaga tattgaagag 10443

aaggttcaat atgtgttgaa gctattacaa gaagatggag attcgtttgc gaagcgtgca 10503

gagatgtatt ataagaagag accagaactc attagcttcg ttgaagaatc ttacagagct 10563

tatagagctt tagctgaacg ttatgatcat atttctacag agcttcaaaa tgct 10617

<210> 8
<211> 32
<212> DNA
<213> Artificial Sequence

<220>
<223> Description of Artificial Sequence: primer LR32

<400> 8
actcgattct caacccgaaa gtatagatcc ca 32

<210> 9
<211> 16
<212> DNA
<213> Artificial Sequence

<220>
<223> Description of Artificial Sequence: primerAPL16

<400> 9
tatgggatca cattaa 16

<210> 10
<211> 26
<212> DNA
<213> Artificial Sequence

<220>
<223> Description of Artificial Sequence: En-1 specific primer En205R

<400> 10
agaagcacga cggctgtaga atagga 26

<210> 11

<211> 26
<212> DNA
<213> Artificial Sequence

<220>
<223> Description of Artificial Sequence: En-1 specific primer En91R

<400> 11
tgcagcaaaa cccacacttt tacttc 26

<210> 12
<211> 26
<212> DNA
<213> Artificial Sequence

<220>
<223> Description of Artificial Sequence: linker specific primer LR26

<400> 12
actcgattct caacccgaaa gtatag 26

<210> 13
<211> 35
<212> DNA
<213> Artificial Sequence

<220>
<223> Description of Artificial Sequence: SVP specific primeroU9 containing a XbaI restriction site at the 5' end

<400> 13
gcgtctagaa tggcgagaga aaagattcag atcag 35

<210> 14
<211> 38
<212> DNA
<213> Artificial Sequence

<220>
<223> Description of Artificial Sequence: SVP specific primer oU10 containing a XbaI restriction site at the 5' end

<400> 14
gcgtctagat tatctcacag tatgatcaaa catatgtg 38

<210> 15
<211> 30
<212> DNA
<213> Artificial Sequence

<220>
<223> Description of Artificial Sequence: primer S313

<400> 15
cagctagtca aggatccgaa ttcgagcggg 30

<210> 16
<211> 48
<212> DNA
<213> Artificial Sequence

<220>
<223> Description of Artificial Sequence: primer GC361

<400> 16
aagcagtggt aacaacgcag agtacttttt ttttttttt tttttttt 48

<210> 17
<211> 24
<212> DNA
<213> Artificial Sequence

<220>
<223> Description of Artificial Sequence: primer GC362

<400> 17
agctagtcaa ggatccgaat tcga 24

<210> 18
<211> 26
<212> DNA
<213> Artificial Sequence

<220>
<223> Description of Artificial Sequence: primer GC360

<400> 18
gcagtggtaa caacgcagag tacttt 26

<210> 19
<211> 36
<212> DNA
<213> Artificial Sequence

<220>
<223> Description of Artificial Sequence: PCR primer oH13

<400> 19
gcgtctagaa tgaaggaagt cctagagagg cataac 36

<210> 20
<211> 39
<212> DNA
<213> Artificial Sequence

<220>
<223> Description of Artificial Sequence: PCR primer oU26

<400> 20
gcgtctagac taaccaccat acggtaagcc gagcctaag 39

<210> 21
<211> 210
<212> PRT
<213> Arabidopsis thaliana

<400> 21
Met Ala Arg Glu Lys Ile Gln Ile Arg Lys Ile Asp Asn Ala Thr Ala
1 5 10 15

Arg Gln Val Thr Phe Ser Lys Arg Arg Arg Gly Leu Phe Lys Lys Ala
20 25 30

Glu Glu Leu Ser Val Leu Cys Asp Ala Asp Val Ala Leu Ile Ile Phe
35 40 45

Ser Ser Thr Gly Lys Leu Phe Glu Phe Cys Ser Ser Ser Met Lys Glu
50 55 60

Val Leu Glu Arg His Asn Leu Gln Ser Lys Asn Leu Glu Lys Leu Asp
65 70 75 80

Gln Pro Ser Leu Glu Leu Gln Leu Val Glu Asn Ser Asp His Ala Arg
85 90 95

Met Ser Lys Glu Ile Ala Asp Lys Ser His Arg Leu Arg Gln Met Arg
100 105 110

Gly Glu Glu Leu Gln Gly Leu Asp Ile Glu Glu Leu Gln Gln Leu Glu
115 120 125

Lys Ala Leu Glu Thr Gly Leu Thr Arg Val Ile Glu Thr Lys Ser Asp
130 135 140

Lys Ile Met Ser Glu Ile Ser Glu Leu Gln Lys Lys Gly Met Gln Leu
145 150 155 160

Met Asp Glu Asn Lys Arg Leu Arg Gln Gln Val Cys Val Leu Pro Ser
165 170 175

Leu Leu Ile Thr Asn Pro Phe Leu Leu Ser Thr Ile Asn Val His Thr
180 185 190

Pro Lys Phe Asn Pro Gln Leu Ser Thr Thr His Met Phe Asp His Thr
195 200 205

Val Arg
210

<210> 22
<211> 240
<212> PRT
<213> Arabidopsis thaliana

<400> 22
Met Ala Arg Glu Lys Ile Gln Ile Arg Lys Ile Asp Asn Ala Thr Ala
1 5 10 15

Arg Gln Val Thr Phe Ser Lys Arg Arg Arg Gly Leu Phe Lys Lys Ala
20 25 30

Glu Glu Leu Ser Val Leu Cys Asp Ala Asp Val Ala Leu Ile Ile Phe
35 40 45

Ser Ser Thr Gly Lys Leu Phe Glu Phe Cys Ser Ser Ser Met Lys Glu
50 55 60

Val Leu Glu Arg His Asn Leu Gln Ser Lys Asn Leu Glu Lys Leu Asp
65 70 75 80

Gln Pro Ser Leu Glu Leu Gln Leu Val Glu Asn Ser Asp His Ala Arg
85 90 95

Met Ser Lys Glu Ile Ala Asp Lys Ser His Arg Leu Arg Gln Met Arg
100 105 110

Gly Glu Glu Leu Gln Gly Leu Asp Ile Glu Glu Leu Gln Gln Leu Glu
115 120 125

Lys Ala Leu Glu Thr Gly Leu Thr Arg Val Ile Glu Thr Lys Ser Asp
130 135 140

Lys Ile Met Ser Glu Ile Ser Glu Leu Gln Lys Lys Gly Met Gln Leu
145 150 155 160

Met Asp Glu Asn Lys Arg Leu Arg Gln Gln Gly Thr Gln Leu Thr Glu
165 170 175

Glu Asn Glu Arg Leu Gly Met Gln Ile Cys Asn Asn Val His Ala His
180 185 190

Gly Gly Ala Glu Ser Glu Asn Ala Ala Val Tyr Glu Glu Gly Gln Ser
195 200 205

Ser Glu Ser Ile Thr Asn Ala Gly Asn Ser Thr Gly Ala Pro Val Asp
210 215 220

Ser Glu Ser Ser Asp Thr Ser Leu Arg Leu Gly Leu Pro Tyr Gly Gly
225 230 235 240